(19) Europäisches Patentamt
European Patent Office
Office européen des brevets

(11) EP 3 438 261 A1

(12) EUROPEAN PATENT APPLICATION
published in accordance with Art. 153(4) EPC

(43) Date of publication:
06.02.2019 Bulletin 2019/06

(21) Application number: 17775134.4

(22) Date of filing: 28.03.2017

(51) Int Cl.:
*C12N 15/09* (2006.01) *A61K 39/395* (2006.01)
*A61K 48/00* (2006.01) *A61P 37/06* (2006.01)
*C07K 16/18* (2006.01) *C12P 21/08* (2006.01)

(86) International application number:
PCT/JP2017/012736

(87) International publication number:
WO 2017/170597 (05.10.2017 Gazette 2017/40)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 29.03.2016 JP 2016066829

(71) Applicant: **Kyowa Hakko Kirin Co., Ltd.**
**Tokyo 100-8185 (JP)**

(72) Inventors:
- **KATO, Yuko**
  **Tokyo 100-8185 (JP)**
- **NAKANO, Ryosuke**
  **Tokyo 100-8185 (JP)**

(74) Representative: **Hoffmann Eitle**
**Patent- und Rechtsanwälte PartmbB**
**Arabellastraße 30**
**81925 München (DE)**

# (54) AUTOIMMUNE DISEASE THERAPEUTIC AGENT CONTAINING, AS ACTIVE INGREDIENT, ANTIBODY THAT BINDS TO HAPTOGLOBIN IN BLOOD TO FORM POLYVALENT IMMUNE COMPLEX

(57) The purpose of the present application is to provide an antibody that exhibits high binding activity to Fcγ receptors and can be used as a substitute for IVIG, DNA coding said antibody, a vector containing said DNA, a transformant obtained through the introduction of said vector, a method of producing an antibody using said transformant, and an autoimmune disease therapeutic agent containing said antibody as an active ingredient. The present application relates to a monoclonal antibody that specifically recognizes a beta chain of human haptoglobin having the amino acid sequence represented by SEQ ID NO. 70, and binds with said human haptoglobin to form a polyvalent immune complex.

EP 3 438 261 A1

SEQUENCE LISTING

<110> Kyowa Hakko Kirin Co., Ltd.

<120> Therapeutic agent for treatment of autoimmune diseases containing antibody binding to haptoglobin and forming immune complexes as an effective component

<130> W522557

<150> JP2016-066829
<151> 2016-3-29

<160> 98

<170> PatentIn version 3.3

<210> 1
<211> 32
<212> DNA
<213> Artificial

<220>
<223> hCD16a-F

<400> 1
taaatagaat tcggcatcat gtggcagctg ct 32

<210> 2
<211> 34
<212> DNA
<213> Artificial

<220>
<223> hCD16a-R

<400> 2
aataaaggat cctggggtca tttgtcttga gggt 34

<210> 3
<211> 765
<212> DNA
<213> Homo sapiens

<400> 3
atgtggcagc tgctcctccc aactgctctg ctacttctag tttcagctgg catgcggact 60

gaagatctcc caaaggctgt ggtgttcctg gagcctcaat ggtacagggt gctcgagaag 120

gacagtgtga ctctgaagtg ccagggagcc tactcccctg aggacaattc cacacagtgg 180

tttcacaatg agagcctcat ctcaagccag gcctcgagct acttcattga cgctgccaca 240

gtcgacgaca gtggagagta caggtgccag acaaacctct ccaccctcag tgacccggtg 300

cagctagaag tccatatcgg ctggctgttg ctccaggccc ctcggtgggt gttcaaggag 360

gaagacccta ttcacctgag gtgtcacagc tggaagaaca ctgctctgca taaggtcaca 420

tatttacaga atggcaaagg caggaagtat tttcatcata attctgactt ctacattcca 480

```
aaagccacac tcaaagacag cggctcctac ttctgcaggg ggcttgttgg gagtaaaaat    540

gtgtcttcag agactgtgaa catcaccatc actcaaggtt tggcagtgtc aaccatctca    600

tcattctttc cacctgggta ccaagtctct ttctgcttgg tgatggtact cctttttgca    660

gtggacacag gactatattt ctctgtgaag acaaacattc gaagctcaac aagagactgg    720

aaggaccata aatttaaatg gagaaaggac cctcaagaca aatga                    765


<210>  4
<211>  254
<212>  PRT
<213>  Homo sapiens

<400>  4

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95

Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Phe Tyr Ile Pro
145                 150                 155                 160

Lys Ala Thr Leu Lys Asp Ser Gly Ser Tyr Phe Cys Arg Gly Leu Val
                165                 170                 175

Gly Ser Lys Asn Val Ser Ser Glu Thr Val Asn Ile Thr Ile Thr Gln
```

```
            180                 185                 190

Gly Leu Ala Val Ser Thr Ile Ser Ser Phe Phe Pro Pro Gly Tyr Gln
        195                 200                 205

Val Ser Phe Cys Leu Val Met Val Leu Leu Phe Ala Val Asp Thr Gly
    210                 215                 220

Leu Tyr Phe Ser Val Lys Thr Asn Ile Arg Ser Ser Thr Arg Asp Trp
225                 230                 235                 240

Lys Asp His Lys Phe Lys Trp Arg Lys Asp Pro Gln Asp Lys
                245                 250


<210>  5
<211>  51
<212>  DNA
<213>  Artificial

<220>
<223>  FcgR3-1

<400>  5
tgttggatcc tgtcaatgat gatgatgatg atgaccttga gtgatggtga t            51


<210>  6
<211>  199
<212>  PRT
<213>  Artificial

<220>
<223>  CD16a-His-tag_AA

<400>  6

Met Trp Gln Leu Leu Leu Pro Thr Ala Leu Leu Leu Leu Val Ser Ala
1               5                   10                  15

Gly Met Arg Thr Glu Asp Leu Pro Lys Ala Val Val Phe Leu Glu Pro
            20                  25                  30

Gln Trp Tyr Arg Val Leu Glu Lys Asp Ser Val Thr Leu Lys Cys Gln
        35                  40                  45

Gly Ala Tyr Ser Pro Glu Asp Asn Ser Thr Gln Trp Phe His Asn Glu
    50                  55                  60

Ser Leu Ile Ser Ser Gln Ala Ser Ser Tyr Phe Ile Asp Ala Ala Thr
65                  70                  75                  80

Val Asp Asp Ser Gly Glu Tyr Arg Cys Gln Thr Asn Leu Ser Thr Leu
                85                  90                  95
```

```
Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125

His Ser Trp Lys Asn Thr Ala Leu His Lys Val Thr Tyr Leu Gln Asn
    130                 135                 140

Gly Lys Gly Arg Lys Tyr Phe His His Asn Ser Asp Phe Tyr Ile Pro
145                 150                 155                 160

Lys Ala Thr Leu Lys Asp Ser Gly Ser Tyr Phe Cys Arg Gly Leu Val
                165                 170                 175

Gly Ser Lys Asn Val Ser Ser Glu Thr Val Asn Ile Thr Ile Thr Gln
            180                 185                 190

Gly His His His His His His
        195
```

<210> 7
<211> 28
<212> DNA
<213> Artificial

<220>
<223> hhiu1

<400> 7
gcctgagttc cacgacaccg tcaccggt 28

<210> 8
<211> 27
<212> DNA
<213> Artificial

<220>
<223> hhiu3

<400> 8
caggggaag accgatgggc ccttggt 27

<210> 9
<211> 26
<212> DNA
<213> Artificial

<220>
<223> hk2

<400> 9

gttgaagctc tttgtgacgg gcgagc 26

<210> 10
<211> 30
<212> DNA
<213> Artificial

<220>
<223> hk5

<400> 10
aggcacacaa cagaggcagt tccagatttc 30

<210> 11
<211> 364
<212> DNA
<213> Artificial

<220>
<223> #4-VH_DNA

<400> 11
gaggtgcacc tattggagtc tgggggaggc ctggtacagc ctggggggctc cctgagactc 60

tcctgtcaag cctctgggtt cacctttaat agcaatgcca tgagctgggt ccgccgggct 120

ccagggaagg ggctggagtg gatctcatca attggtgcaa atactaatta catatacacc 180

gcagactccg tgaagggccg gttcaccatc tccagggaca attccaagaa cacgctgtat 240

ctgcaaatga acagcctgag agccgaggac acggccgcat attactgcgc aaagaatcgt 300

gatcggtact cgtacgacgc ttggagcgtc tggggccaag ggaccacggt cactgtttcc 360

tcag 364

<210> 12
<211> 324
<212> DNA
<213> Artificial

<220>
<223> #4-VL_DNA

<400> 12
gacatccaga tgacccagtc tccttccacc ctgtctgcat ctgtaggaga cagagtcacc 60

atcacttgcc gggccagtca gagtattgat atctggttgg cctggtatca gcagaaacca 120

gggaaagccc ctaaactcct gatttatgag gcatctagtt tagaaagtga ggtcccatca 180

aggttcagcg gcagtggatc tgggacagaa ttcactctca ccatcagcag cctgcagcct 240

gatgattttg caacttatta ctgccaagcg tataatagta attatccata cgcttttggc 300

caggggacca acctggagat caaa 324

<210> 13
<211> 364

<212> DNA
<213> Artificial

<220>
<223> #6-VH_DNA

<400> 13
gaggtgcagc tgttggagtc tgggggaggc ttggtacagc ctggggggtc cctgaagctc 60

tcctgtgtag cctctgaatt cacctttagc aactatgcca tgagttgggt ccgccaggct 120

ccagggaagg ggctggagtg ggtctcaacc tttagtagtg gtggtagccg tacattctac 180

gcagactccg tgaggggccg gttcaccatt tccagagaca attccaacaa tatgttgttt 240

ctgcaaatag acagcctgag agccgaggac acggccgtat attactgtgc gaaggcccag 300

tttgttggtc attgggccca atataactac tggggccagg gagccctggt caccgtctcc 360

tcag 364

<210> 14
<211> 321
<212> DNA
<213> Artificial

<220>
<223> #6-VL_DNA

<400> 14
gacatccaga tgactcagtc tccatcctcc ctgtctgcat ctgtgggaga cagagtcgcc 60

atcacttgcc gggcgagtca gggcattaga aattacttag cctggtatca gcagaaacca 120

gggaaagttc ctaagctcct gatctatgat gcatttattt tgcaatcagg ggtcccatct 180

cgcttcagcg gcagtggatc tgggacagat ttcactctca ccattagcag cctgcagcct 240

gaagatgttg caacttatta ctgtcaaaag tctgacagtc cccctctcac tttcggcgga 300

gggaccaagg tggagatcaa a 321

<210> 15
<211> 364
<212> DNA
<213> Artificial

<220>
<223> #27-VH_DNA

<400> 15
gaacagcagc tggtggagtc tgggggaggc ttggtacagc ctggcaggtc cctgacactc 60

tcctgtgtag cctctggatt cacgtttggt gattatgcca tgggctgggt ccgccaagct 120

ccaggaaagg ggctggagtg ggtctctagt attagttcgg atgctcttta caaaggttat 180

gtagactctg tgaagggccg attcaccgtc tccagagaca acgccaagaa ctccctgtat 240

ctgcaaatgg acaatctgag aactgaagat acggccttct attactgtac aaaagacttg 300

agaagcggca actttggcca gtacggcgac tggggccagg gaactctagt caccgtctct 360

tcag 364

<210> 16
<211> 321
<212> DNA
<213> Artificial

<220>
<223> #27-VL_DNA

<400> 16
gccatccaga tgacccagtc tccatcctcc ctctctgcat ctgtaggaga cagagtcacc 60

atcacttgcc gggcaagtca gggcatagga agtgatttaa cctggtatca gcaggagcca 120

gggaaagccc ctaaactcct gatctatgct gcgtccactt tacaaagtgg ggtcccatca 180

aggttcagcg gcggtggatc tggcacagat ttcactctca ccatcagcag cctgcagcct 240

gaagatgctg caacttatta ctgtctccaa tctcacggtt acccgttaac ttttggccag 300

gggaccaagc tggagatcaa a 321

<210> 17
<211> 378
<212> DNA
<213> Artificial

<220>
<223> #105-VH_DNA

<400> 17
caggtgcacc tacagcagtg gggcgcagga ctgttgaagc cttcggagac cctgtccctc 60

acctgcaatg tctatggttc gtccttaagt cagtacttct ggaattggat ccgccagccc 120

ccagggaagg ggctggagtg gatggggcgc atcacttctg gtggaagcac caactacaac 180

ccgtccctcg agagtcgagt caccataatc gtagacacgt tcaagaatca gttctccctg 240

aagctggtct ctgtgaccgc cgcagacacg gctgtatatt actgtgcgag agggccgttt 300

gttgagggcg cagcgccaac tgtacttgga actggtttca gcatttgggg ccaaggaca 360

atggtcaccg tctcttca 378

<210> 18
<211> 327
<212> DNA
<213> Artificial

<220>
<223> #105-VL_DNA

<400> 18
gaaattgtgt taacgcagtc tccaggcacc ctgtctttgt ctccagggga aagagccacc 60

ctctcctgca gggccagtca gagtgtgaac aacaactact tagcctggta ccagcagaaa 120

cctggccagg ctcccaggct cctcatctat gatgcatcca agagggccac tggcatccca 180

gacaggttca gtggcagtgg gtctgggaca gacttcactc tcaccatcag cagactggag 240

cctgaagatt ttgcagtgta ttactgtcaa caatacggtc actcacgtcc gtacactttt 300

ggccaggggg ccaagctgga gatcaaa 327

<210> 19
<211> 345
<212> DNA
<213> Artificial

<220>
<223> #96-6-VH_DNA

<400> 19
gaggtacaac tgctggagtc tgggggaggc ttggttcagc ctgggggtc cctgagactc 60

tcctgtgaag cctctggaag cagcgttagt tcccatttca tgacctgggt ccgccagagt 120

ccagggaagg ggctagagtg ggtctcaagc atctctccta atcctgcgta cacgtactac 180

gccaactccg tgaagggccg cttcaccatc tcccgagaca attcaaagaa tatgctgtat 240

ctacaaatga acgacctgag agccgaggac acggccagat attactgtgc gaggcgttct 300

gattttgata cctggggcca agggacaatg ctcaccgtct cttca 345

<210> 20
<211> 321
<212> DNA
<213> Artificial

<220>
<223> #96-6-VL_DNA

<400> 20
gacatccaga tgacccagtc tccatctgcc atgtctgcat ctgtaggaga cagagtcacc 60

atcacttgtc gggcgagtag gtacattggc aaagatttag cctggtttca gcagaaacca 120

ggggaagtcc ctaagcgcct gatctacgac gcatccagtc tgcagagtgg ggtcccatca 180

aggttcagcg gcagtggatc tgggacagaa ttcactctca cagtcagcaa cctgcaacct 240

gaagatttgg gaacttatta ctgccaacac tattatactt tccctctgac tttcggccct 300

gggaccaaag tggatatcaa a 321

<210> 21
<211> 121
<212> PRT
<213> Artificial

<220>
<223> #4-VH_AA

<400> 21

```
Glu Val His Leu Leu Glu Ser Gly Gly Gly Leu Val Gln Pro Gly Gly
1               5                   10                  15

Ser Leu Arg Leu Ser Cys Gln Ala Ser Gly Phe Thr Phe Asn Ser Asn
            20                  25                  30

Ala Met Ser Trp Val Arg Arg Ala Pro Gly Lys Gly Leu Glu Trp Ile
        35                  40                  45

Ser Ser Ile Gly Ala Asn Thr Asn Tyr Ile Tyr Thr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Ala Tyr Tyr Cys
                85                  90                  95

Ala Lys Asn Arg Asp Arg Tyr Ser Tyr Asp Ala Trp Ser Val Trp Gly
            100                 105                 110

Gln Gly Thr Thr Val Thr Val Ser Ser
        115                 120


<210>  22
<211>  108
<212>  PRT
<213>  Artificial

<220>
<223>  #4-VL_AA

<400>  22

Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Asp Ile Trp
            20                  25                  30

Leu Ala Trp Tyr Gln Gln Lys Pro Gly Lys Ala Pro Lys Leu Leu Ile
        35                  40                  45

Tyr Glu Ala Ser Ser Leu Glu Ser Glu Val Pro Ser Arg Phe Ser Gly
    50                  55                  60

Ser Gly Ser Gly Thr Glu Phe Thr Leu Thr Ile Ser Ser Leu Gln Pro
65                  70                  75                  80
```

```
Asp Asp Phe Ala Thr Tyr Tyr Cys Gln Ala Tyr Asn Ser Asn Tyr Pro
                85                  90                  95

Tyr Ala Phe Gly Gln Gly Thr Asn Leu Glu Ile Lys
            100                 105


<210>  23
<211>  121
<212>  PRT
<213>  Artificial

<220>
<223>  #6-VH_AA

<400>  23

Glu Val Gln Leu Leu Glu Ser Gly Gly Gly Leu Val Gln Pro Gly Gly
1               5                   10                  15

Ser Leu Lys Leu Ser Cys Val Ala Ser Glu Phe Thr Phe Ser Asn Tyr
            20                  25                  30

Ala Met Ser Trp Val Arg Gln Ala Pro Gly Lys Gly Leu Glu Trp Val
        35                  40                  45

Ser Thr Phe Ser Ser Gly Gly Ser Arg Thr Phe Tyr Ala Asp Ser Val
    50                  55                  60

Arg Gly Arg Phe Thr Ile Ser Arg Asp Asn Ser Asn Asn Met Leu Phe
65                  70                  75                  80

Leu Gln Ile Asp Ser Leu Arg Ala Glu Asp Thr Ala Val Tyr Tyr Cys
                85                  90                  95

Ala Lys Ala Gln Phe Val Gly His Trp Ala Gln Tyr Asn Tyr Trp Gly
            100                 105                 110

Gln Gly Ala Leu Val Thr Val Ser Ser
        115                 120


<210>  24
<211>  107
<212>  PRT
<213>  Artificial

<220>
<223>  #6-VL_AA

<400>  24

Asp Ile Gln Met Thr Gln Ser Pro Ser Ser Leu Ser Ala Ser Val Gly
1               5                   10                  15
```

```
Asp Arg Val Ala Ile Thr Cys Arg Ala Ser Gln Gly Ile Arg Asn Tyr
            20                  25                  30

Leu Ala Trp Tyr Gln Gln Lys Pro Gly Lys Val Pro Lys Leu Leu Ile
        35                  40                  45

Tyr Asp Ala Phe Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Lys Ser Asp Ser Pro Pro Leu
                85                  90                  95

Thr Phe Gly Gly Gly Thr Lys Val Glu Ile Lys
            100                 105


<210>  25
<211>  121
<212>  PRT
<213>  Artificial

<220>
<223>  #27-VH_AA

<400>  25

Glu Gln Gln Leu Val Glu Ser Gly Gly Gly Leu Val Gln Pro Gly Arg
1               5                   10                  15

Ser Leu Thr Leu Ser Cys Val Ala Ser Gly Phe Thr Phe Gly Asp Tyr
            20                  25                  30

Ala Met Gly Trp Val Arg Gln Ala Pro Gly Lys Gly Leu Glu Trp Val
        35                  40                  45

Ser Ser Ile Ser Ser Asp Ala Leu Tyr Lys Gly Tyr Val Asp Ser Val
    50                  55                  60

Lys Gly Arg Phe Thr Val Ser Arg Asp Asn Ala Lys Asn Ser Leu Tyr
65                  70                  75                  80

Leu Gln Met Asp Asn Leu Arg Thr Glu Asp Thr Ala Phe Tyr Tyr Cys
                85                  90                  95

Thr Lys Asp Leu Arg Ser Gly Asn Phe Gly Gln Tyr Gly Asp Trp Gly
            100                 105                 110
```

Gln Gly Thr Leu Val Thr Val Ser Ser
115 120

<210> 26
<211> 107
<212> PRT
<213> Artificial

<220>
<223> #27-VL_AA

<400> 26

Ala Ile Gln Met Thr Gln Ser Pro Ser Ser Leu Ser Ala Ser Val Gly
1 5 10 15

Asp Arg Val Thr Ile Thr Cys Arg Ala Ser Gln Gly Ile Gly Ser Asp
20 25 30

Leu Thr Trp Tyr Gln Gln Glu Pro Gly Lys Ala Pro Lys Leu Leu Ile
35 40 45

Tyr Ala Ala Ser Thr Leu Gln Ser Gly Val Pro Ser Arg Phe Ser Gly
50 55 60

Gly Gly Ser Gly Thr Asp Phe Thr Leu Thr Ile Ser Ser Leu Gln Pro
65 70 75 80

Glu Asp Ala Ala Thr Tyr Tyr Cys Leu Gln Ser His Gly Tyr Pro Leu
85 90 95

Thr Phe Gly Gln Gly Thr Lys Leu Glu Ile Lys
100 105

<210> 27
<211> 126
<212> PRT
<213> Artificial

<220>
<223> #105-VH_AA

<400> 27

Gln Val His Leu Gln Gln Trp Gly Ala Gly Leu Leu Lys Pro Ser Glu
1 5 10 15

Thr Leu Ser Leu Thr Cys Asn Val Tyr Gly Ser Ser Leu Ser Gln Tyr
20 25 30

Phe Trp Asn Trp Ile Arg Gln Pro Pro Gly Lys Gly Leu Glu Trp Met
35 40 45

```
Gly Arg Ile Thr Ser Gly Gly Ser Thr Asn Tyr Asn Pro Ser Leu Glu
    50                  55                  60

Ser Arg Val Thr Ile Ile Val Asp Thr Phe Lys Asn Gln Phe Ser Leu
65                  70                  75                  80

Lys Leu Val Ser Val Thr Ala Ala Asp Thr Ala Val Tyr Tyr Cys Ala
                85                  90                  95

Arg Gly Pro Phe Val Glu Gly Ala Ala Pro Thr Val Leu Gly Thr Gly
            100                 105                 110

Phe Ser Ile Trp Gly Gln Gly Thr Met Val Thr Val Ser Ser
        115                 120                 125


<210>  28
<211>  109
<212>  PRT
<213>  Artificial

<220>
<223>  #105-VL_AA

<400>  28

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Asn Asn Asn
            20                  25                  30

Tyr Leu Ala Trp Tyr Gln Gln Lys Pro Gly Gln Ala Pro Arg Leu Leu
        35                  40                  45

Ile Tyr Asp Ala Ser Lys Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly His Ser Arg
                85                  90                  95

Pro Tyr Thr Phe Gly Gln Gly Ala Lys Leu Glu Ile Lys
            100                 105


<210>  29
<211>  115
<212>  PRT
<213>  Artificial
```

<220>
<223> #96-6-VH_AA

<400> 29

Glu Val Gln Leu Leu Glu Ser Gly Gly Gly Leu Val Gln Pro Gly Gly
1 5 10 15

Ser Leu Arg Leu Ser Cys Glu Ala Ser Gly Ser Ser Val Ser Ser His
20 25 30

Phe Met Thr Trp Val Arg Gln Ser Pro Gly Lys Gly Leu Glu Trp Val
35 40 45

Ser Ser Ile Ser Pro Asn Pro Ala Tyr Thr Tyr Tyr Ala Asn Ser Val
50 55 60

Lys Gly Arg Phe Thr Ile Ser Arg Asp Asn Ser Lys Asn Met Leu Tyr
65 70 75 80

Leu Gln Met Asn Asp Leu Arg Ala Glu Asp Thr Ala Arg Tyr Tyr Cys
85 90 95

Ala Arg Arg Ser Asp Phe Asp Thr Trp Gly Gln Gly Thr Met Leu Thr
100 105 110

Val Ser Ser
115

<210> 30
<211> 107
<212> PRT
<213> Artificial

<220>
<223> #96-6-VL_AA

<400> 30

Asp Ile Gln Met Thr Gln Ser Pro Ser Ala Met Ser Ala Ser Val Gly
1 5 10 15

Asp Arg Val Thr Ile Thr Cys Arg Ala Ser Arg Tyr Ile Gly Lys Asp
20 25 30

Leu Ala Trp Phe Gln Gln Lys Pro Gly Glu Val Pro Lys Arg Leu Ile
35 40 45

Tyr Asp Ala Ser Ser Leu Gln Ser Gly Val Pro Ser Arg Phe Ser Gly
50 55 60

Ser Gly Ser Gly Thr Glu Phe Thr Leu Thr Val Ser Asn Leu Gln Pro
65 70 75 80

Glu Asp Leu Gly Thr Tyr Tyr Cys Gln His Tyr Tyr Thr Phe Pro Leu
85 90 95

Thr Phe Gly Pro Gly Thr Lys Val Asp Ile Lys
100 105

<210> 31
<211> 5
<212> PRT
<213> Artificial

<220>
<223> #27-VH CDR1_AA

<400> 31

Asp Tyr Ala Met Gly
1 5

<210> 32
<211> 17
<212> PRT
<213> Artificial

<220>
<223> #27-VH CDR2_AA

<400> 32

Ser Ile Ser Ser Asp Ala Leu Tyr Lys Gly Tyr Val Asp Ser Val Lys
1 5 10 15

Gly

<210> 33
<211> 12
<212> PRT
<213> Artificial

<220>
<223> #27-VH CDR3_AA

<400> 33

Asp Leu Arg Ser Gly Asn Phe Gly Gln Tyr Gly Asp
1 5 10

<210> 34
<211> 11
<212> PRT
<213> Artificial

<220>
<223> #27-VL CDR1_AA

<400> 34

Arg Ala Ser Gln Gly Ile Gly Ser Asp Leu Thr
1 5 10

<210> 35
<211> 7
<212> PRT
<213> Artificial

<220>
<223> #27-VL CDR2_AA

<400> 35

Ala Ala Ser Thr Leu Gln Ser
1 5

<210> 36
<211> 9
<212> PRT
<213> Artificial

<220>
<223> #27-VL CDR3_AA

<400> 36

Leu Gln Ser His Gly Tyr Pro Leu Thr
1 5

<210> 37
<211> 5
<212> PRT
<213> Artificial

<220>
<223> #105-VH CDR1_AA

<400> 37

Gln Tyr Phe Trp Asn
1 5

<210> 38
<211> 16
<212> PRT
<213> Artificial

<220>
<223> #105-VH CDR2_AA

<400> 38

Arg Ile Thr Ser Gly Gly Ser Thr Asn Tyr Asn Pro Ser Leu Glu Ser

1 5 10 15

<210> 39
<211> 18
<212> PRT
<213> Artificial

<220>
<223> #105-VH CDR3_AA

<400> 39

Gly Pro Phe Val Glu Gly Ala Ala Pro Thr Val Leu Gly Thr Gly Phe
1 5 10 15

Ser Ile

<210> 40
<211> 12
<212> PRT
<213> Artificial

<220>
<223> #105-VL CDR1_AA

<400> 40

Arg Ala Ser Gln Ser Val Asn Asn Asn Tyr Leu Ala
1 5 10

<210> 41
<211> 7
<212> PRT
<213> Artificial

<220>
<223> #105-VL CDR2_AA

<400> 41

Asp Ala Ser Lys Arg Ala Thr
1 5

<210> 42
<211> 10
<212> PRT
<213> Artificial

<220>
<223> #105-VL CDR3_AA

<400> 42

Gln Gln Tyr Gly His Ser Arg Pro Tyr Thr
1 5 10

<210> 43
<211> 102
<212> DNA
<213> Artificial

<220>
<223> #4-VH-F

<400> 43
ctcactatag cggccgcgac ccctcaccat gagagtgctt attttattgt ggctgttcac 60

agcctttcct ggtattctta gtgaggtgca cctattggag tc 102

<210> 44
<211> 50
<212> DNA
<213> Artificial

<220>
<223> #4-VH-R

<400> 44
gggggaagac cgatgggccc ttggtggaag ctgaggaaac agtgaccgtg 50

<210> 45
<211> 113
<212> DNA
<213> Artificial

<220>
<223> #4-VL-F

<400> 45
actcactata gaattcgcct cctcaaaatg cattttcaag tgcagatttt cagcttcctg 60

cttatttcgg cctcagtcat aatgtccaga ggagacatcc agatgaccca gtc 113

<210> 46
<211> 35
<212> DNA
<213> Artificial

<220>
<223> #4-VL-R

<400> 46
gtgcagccac cgtacgtttg atctccaggt tggtc 35

<210> 47
<211> 102
<212> DNA
<213> Artificial

<220>
<223> #6-VH-F

<400> 47
ctcactatag cggccgcgac ccctcaccat gagagtgctt attttattgt ggctgttcac 60

agcctttcct ggtattctta gtgaggtgca gctgttggag tc 102

<210> 48
<211> 50
<212> DNA
<213> Artificial

<220>
<223> #6-VH-R

<400> 48
gggggaagac cgatgggccc ttggtggaag ctgaggagac ggtgaccagg 50

<210> 49
<211> 113
<212> DNA
<213> Artificial

<220>
<223> #6-VL-F

<400> 49
actcactata gaattcgcct cctcaaaatg cattttcaag tgcagatttt cagcttcctg 60

cttatttcgg cctcagtcat aatgtccaga ggagacatcc agatgactca gtc 113

<210> 50
<211> 35
<212> DNA
<213> Artificial

<220>
<223> #6-VL-R

<400> 50
gtgcagccac cgtacgtttg atctccacct tggtc 35

<210> 51
<211> 102
<212> DNA
<213> Artificial

<220>
<223> #27-VH-F

<400> 51
ctcactatag cggccgcgac ccctcaccat gagagtgctt attttattgt ggctgttcac 60

agcctttcct ggtattctta gtgaacagca gctggtggag tc 102

<210> 52
<211> 50
<212> DNA
<213> Artificial

<220>
<223> #27-VH-R

<400> 52
gggggaagac cgatgggccc ttggtggaag ctgaagagac ggtgactaga 50

<210> 53
<211> 113
<212> DNA
<213> Artificial

<220>
<223> #27-VL-F

<400> 53
actcactata gaattcgcct cctcaaaatg cattttcaag tgcagatttt cagcttcctg 60

cttatttcgg cctcagtcat aatgtccaga ggagccatcc agatgaccca gtc 113

<210> 54
<211> 35
<212> DNA
<213> Artificial

<220>
<223> #27-VL-R

<400> 54
gtgcagccac cgtacgtttg atctccagct tggtc 35

<210> 55
<211> 102
<212> DNA
<213> Artificial

<220>
<223> #105-VH-F

<400> 55
ctcactatag cggccgcgac ccctcaccat gagagtgctt attttattgt ggctgttcac 60

agcctttcct ggtattctta gtcaggtgca cctacagcag tg 102

<210> 56
<211> 50
<212> DNA
<213> Artificial

<220>
<223> #105-VH-R

<400> 56
gggggaagac cgatgggccc ttggtggaag ctgaagagac ggtgaccatt 50

<210> 57
<211> 113
<212> DNA
<213> Artificial

<220>
<223> #105-VL-F

<400>  57
actcactata gaattcgcct cctcaaaatg cattttcaag tgcagatttt cagcttcctg     60

cttatttcgg cctcagtcat aatgtccaga ggagaaattg tgttaacgca gtc           113

<210>  58
<211>  35
<212>  DNA
<213>  Artificial

<220>
<223>  #105-VL-R

<400>  58
gtgcagccac cgtacgtttg atctccagct tggcc                                35

<210>  59
<211>  102
<212>  DNA
<213>  Artificial

<220>
<223>  #96-6-VH-F

<400>  59
ctcactatag cggccgcgac ccctcaccat gagagtgctt attttattgt ggctgttcac     60

agcctttcct ggtattctta gtgaggtaca actgctggag tc                       102

<210>  60
<211>  50
<212>  DNA
<213>  Artificial

<220>
<223>  #96-6-VH-R

<400>  60
gggggaagac cgatgggccc ttggtggaag ctgaagagac ggtgagcatt                50

<210>  61
<211>  113
<212>  DNA
<213>  Artificial

<220>
<223>  #96-6-VL-F

<400>  61
actcactata gaattcgcct cctcaaaatg cattttcaag tgcagatttt cagcttcctg     60

cttatttcgg cctcagtcat aatgtccaga ggagacatcc agatgaccca gtc           113

<210>  62
<211>  35
<212>  DNA
<213>  Artificial

<220>
<223> #96-6-VL-R

<400> 62
gtgcagccac cgtacgtttg atatccactt tggtc 35

<210> 63
<211> 999
<212> DNA
<213> Artificial

<220>
<223> hCg1-Xen

<400> 63
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccggatgtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

gccacgtacc gtgtggtcag cgtcctcacc gtcctgcacc aggactggct gaatggcaag 600

gagtacaagt gcaaggtctc caacaaagcc ctcccagccc ccgaggagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tgaggatcc 999

<210> 64
<211> 220
<212> PRT
<213> Artificial

<220>
<223> CD32a_AA

<400> 64

Met Ala Met Glu Thr Gln Met Ser Gln Asn Val Cys Pro Arg Asn Leu

1 5 10 15

Trp Leu Leu Gln Pro Leu Thr Val Leu Leu Leu Leu Ala Ser Ala Asp
20 25 30

Ser Gln Ala Ala Ala Pro Pro Lys Ala Val Leu Lys Leu Glu Pro Pro
35 40 45

Trp Ile Asn Val Leu Gln Glu Asp Ser Val Thr Leu Thr Cys Gln Gly
50 55 60

Ala Arg Ser Pro Glu Ser Asp Ser Ile Gln Trp Phe His Asn Gly Asn
65 70 75 80

Leu Ile Pro Thr His Thr Gln Pro Ser Tyr Arg Phe Lys Ala Asn Asn
85 90 95

Asn Asp Ser Gly Glu Tyr Thr Cys Gln Thr Gly Gln Thr Ser Leu Ser
100 105 110

Asp Pro Val His Leu Thr Val Leu Ser Glu Trp Leu Val Leu Gln Thr
115 120 125

Pro His Leu Glu Phe Gln Glu Gly Glu Thr Ile Met Leu Arg Cys His
130 135 140

Ser Trp Lys Asp Lys Pro Leu Val Lys Val Thr Phe Phe Gln Asn Gly
145 150 155 160

Lys Ser Gln Lys Phe Ser Arg Leu Asp Pro Thr Phe Ser Ile Pro Gln
165 170 175

Ala Asn His Ser His Ser Gly Asp Tyr His Cys Thr Gly Asn Ile Gly
180 185 190

Tyr Thr Leu Phe Ser Ser Lys Pro Val Thr Ile Thr Val Gln Val Pro
195 200 205

Ser Met Gly Ser Ser Ser His His His His His His
210 215 220

<210> 65
<211> 406
<212> PRT
<213> Homo sapiens

<400> 65

Met Ser Ala Leu Gly Ala Val Ile Ala Leu Leu Leu Trp Gly Gln Leu

1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

Val Tyr Thr Leu Asn Asp Lys Lys Gln Trp Ile Asn Lys Ala Val Gly
65 70 75 80

Asp Lys Leu Pro Glu Cys Glu Ala Asp Asp Gly Cys Pro Lys Pro Pro
85 90 95

Glu Ile Ala His Gly Tyr Val Glu His Ser Val Arg Tyr Gln Cys Lys
100 105 110

Asn Tyr Tyr Lys Leu Arg Thr Glu Gly Asp Gly Val Tyr Thr Leu Asn
115 120 125

Asn Glu Lys Gln Trp Ile Asn Lys Ala Val Gly Asp Lys Leu Pro Glu
130 135 140

Cys Glu Ala Val Cys Gly Lys Pro Lys Asn Pro Ala Asn Pro Val Gln
145 150 155 160

Arg Ile Leu Gly Gly His Leu Asp Ala Lys Gly Ser Phe Pro Trp Gln
165 170 175

Ala Lys Met Val Ser His His Asn Leu Thr Thr Gly Ala Thr Leu Ile
180 185 190

Asn Glu Gln Trp Leu Leu Thr Thr Ala Lys Asn Leu Phe Leu Asn His
195 200 205

Ser Glu Asn Ala Thr Ala Lys Asp Ile Ala Pro Thr Leu Thr Leu Tyr
210 215 220

Val Gly Lys Lys Gln Leu Val Glu Ile Glu Lys Val Val Leu His Pro
225 230 235 240

Asn Tyr Ser Gln Val Asp Ile Gly Leu Ile Lys Leu Lys Gln Lys Val
245 250 255

Ser Val Asn Glu Arg Val Met Pro Ile Cys Leu Pro Ser Lys Asp Tyr
260 265 270

Ala Glu Val Gly Arg Val Gly Tyr Val Ser Gly Trp Gly Arg Asn Ala
275 280 285

Asn Phe Lys Phe Thr Asp His Leu Lys Tyr Val Met Leu Pro Val Ala
290 295 300

Asp Gln Asp Gln Cys Ile Arg His Tyr Glu Gly Ser Thr Val Pro Glu
305 310 315 320

Lys Lys Thr Pro Lys Ser Pro Val Gly Val Gln Pro Ile Leu Asn Glu
325 330 335

His Thr Phe Cys Ala Gly Met Ser Lys Tyr Gln Glu Asp Thr Cys Tyr
340 345 350

Gly Asp Ala Gly Ser Ala Phe Ala Val His Asp Leu Glu Glu Asp Thr
355 360 365

Trp Tyr Ala Thr Gly Ile Leu Ser Phe Asp Lys Ser Cys Ala Val Ala
370 375 380

Glu Tyr Gly Val Tyr Val Lys Val Thr Ser Ile Gln Asp Trp Val Gln
385 390 395 400

Lys Thr Ile Ala Glu Asn
405

<210> 66
<211> 347
<212> PRT
<213> Homo sapiens

<400> 66

Met Ser Ala Leu Gly Ala Val Ile Ala Leu Leu Leu Trp Gly Gln Leu
1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

```
Val Tyr Thr Leu Asn Asn Glu Lys Gln Trp Ile Asn Lys Ala Val Gly
65                  70                  75                  80

Asp Lys Leu Pro Glu Cys Glu Ala Val Cys Gly Lys Pro Lys Asn Pro
                85                  90                  95

Ala Asn Pro Val Gln Arg Ile Leu Gly Gly His Leu Asp Ala Lys Gly
            100                 105                 110

Ser Phe Pro Trp Gln Ala Lys Met Val Ser His His Asn Leu Thr Thr
        115                 120                 125

Gly Ala Thr Leu Ile Asn Glu Gln Trp Leu Leu Thr Thr Ala Lys Asn
    130                 135                 140

Leu Phe Leu Asn His Ser Glu Asn Ala Thr Ala Lys Asp Ile Ala Pro
145                 150                 155                 160

Thr Leu Thr Leu Tyr Val Gly Lys Lys Gln Leu Val Glu Ile Glu Lys
                165                 170                 175

Val Val Leu His Pro Asn Tyr Ser Gln Val Asp Ile Gly Leu Ile Lys
            180                 185                 190

Leu Lys Gln Lys Val Ser Val Asn Glu Arg Val Met Pro Ile Cys Leu
        195                 200                 205

Pro Ser Lys Asp Tyr Ala Glu Val Gly Arg Val Gly Tyr Val Ser Gly
    210                 215                 220

Trp Gly Arg Asn Ala Asn Phe Lys Phe Thr Asp His Leu Lys Tyr Val
225                 230                 235                 240

Met Leu Pro Val Ala Asp Gln Asp Gln Cys Ile Arg His Tyr Glu Gly
                245                 250                 255

Ser Thr Val Pro Glu Lys Lys Thr Pro Lys Ser Pro Val Gly Val Gln
            260                 265                 270

Pro Ile Leu Asn Glu His Thr Phe Cys Ala Gly Met Ser Lys Tyr Gln
        275                 280                 285

Glu Asp Thr Cys Tyr Gly Asp Ala Gly Ser Ala Phe Ala Val His Asp
    290                 295                 300

Leu Glu Glu Asp Thr Trp Tyr Ala Thr Gly Ile Leu Ser Phe Asp Lys
305                 310                 315                 320
```

Ser Cys Ala Val Ala Glu Tyr Gly Val Tyr Val Lys Val Thr Ser Ile
325 330 335

Gln Asp Trp Val Gln Lys Thr Ile Ala Glu Asn
340 345

<210> 67
<211> 1221
<212> DNA
<213> Homo sapiens

<400> 67
atgagtgccc tgggagctgt cattgccctc ctgctctggg gacagctttt tgcagtggac 60

tcaggcaatg atgtcacgga tatcgcagat gacggctgcc cgaagccccc cgagattgca 120

catggctatg tggagcactc ggttcgctac cagtgtaaga actactacaa actgcgcaca 180

gaaggagatg gagtatacac cttaaatgat aagaagcagt ggataaataa ggctgttgga 240

gataaacttc ctgaatgtga agcagatgac ggctgcccga agccccccga gattgcacat 300

ggctatgtgg agcactcggt tcgctaccag tgtaagaact actacaaact gcgcacagaa 360

ggagatggag tgtacacctt aaacaatgag aagcagtgga taaataaggc tgttggagat 420

aaacttcctg aatgtgaagc agtatgtggg aagcccaaga atccggcaaa cccagtgcag 480

cggatcctgg gtggacacct ggatgccaaa ggcagctttc cctggcaggc taagatggtt 540

tcccaccata atctcaccac aggtgccacg ctgatcaatg aacaatggct gctgaccacg 600

gctaaaaatc tcttcctgaa ccattcagaa aatgcaacag cgaaagacat tgcccctact 660

ttaacactct atgtggggaa aaagcagctt gtagagattg agaaggttgt tctacaccct 720

aactactccc aggtagatat tgggctcatc aaactcaaac agaaggtgtc tgttaatgag 780

agagtgatgc ccatctgcct accttcaaag gattatgcag aagtagggcg tgtgggttat 840

gtttctggct gggggcgaaa tgccaatttt aaatttactg accatctgaa gtatgtcatg 900

ctgcctgtgg ctgaccaaga ccaatgcata aggcattatg aaggcagcac agtccccgaa 960

aagaagacac cgaagagccc tgtaggggtg cagcccatac tgaatgaaca caccttctgt 1020

gctggcatgt ctaagtacca agaagacacc tgctatggcg atgcgggcag tgcctttgcc 1080

gttcacgacc tggaggagga cacctggtat gcgactggga tcttaagctt tgataagagc 1140

tgtgctgtgg ctgagtatgg tgtgtatgtg aaggtgactt ccatccagga ctgggttcag 1200

aagaccatag ctgagaacta a 1221

<210> 68
<211> 1044
<212> DNA
<213> Homo sapiens

<400> 68
atgagtgccc tgggagctgt cattgccctc ctgctctggg gacagctttt tgcagtggac 60

tcaggcaatg atgtcacgga tatcgcagat gacggctgcc cgaagccccc cgagattgca 120

catggctatg tggagcactc ggttcgctac cagtgtaaga actactacaa actgcgcaca 180

gaaggagatg gagtgtacac cttaaacaat gagaagcagt ggataaataa ggctgttgga 240

gataaacttc ctgaatgtga agcagtatgt gggaagccca agaatccggc aaacccagtg 300

cagcggatcc tgggtggaca cctggatgcc aaaggcagct ttccctggca ggctaagatg 360

gtttcccacc ataatctcac cacaggtgcc acgctgatca atgaacaatg gctgctgacc 420

acggctaaaa atctcttcct gaaccattca gaaaatgcaa cagcgaaaga cattgcccct 480

actttaacac tctatgtggg gaaaaagcag cttgtagaga ttgagaaggt tgttctacac 540

cctaactact cccaggtaga tattgggctc atcaaactca aacagaaggt gtctgttaat 600

gagagagtga tgcccatctg cctaccttca aaggattatg cagaagtagg gcgtgtgggt 660

tatgtttctg gctgggggcg aaatgccaat tttaaattta ctgaccatct gaagtatgtc 720

atgctgcctg tggctgacca agaccaatgc ataaggcatt atgaaggcag cacagtcccc 780

gaaaagaaga caccgaagag ccctgtaggg gtgcagccca tactgaatga acacaccttc 840

tgtgctggca tgtctaagta ccaagaagac acctgctatg gcgatgcggg cagtgccttt 900

gccgttcacg acctggagga ggacacctgg tatgcgactg ggatcttaag ctttgataag 960

agctgtgctg tggctgagta tggtgtgtat gtgaaggtga cttccatcca ggactgggtt 1020

cagaagacca tagctgagaa ctaa 1044

<210> 69
<211> 142
<212> PRT
<213> Homo sapiens

<400> 69

Val Asp Ser Gly Asn Asp Val Thr Asp Ile Ala Asp Asp Gly Cys Pro
1 5 10 15

Lys Pro Pro Glu Ile Ala His Gly Tyr Val Glu His Ser Val Arg Tyr
20 25 30

Gln Cys Lys Asn Tyr Tyr Lys Leu Arg Thr Glu Gly Asp Gly Val Tyr
35 40 45

Thr Leu Asn Asp Lys Lys Gln Trp Ile Asn Lys Ala Val Gly Asp Lys
50 55 60

Leu Pro Glu Cys Glu Ala Asp Asp Gly Cys Pro Lys Pro Pro Glu Ile
65 70 75 80

```
Ala His Gly Tyr Val Glu His Ser Val Arg Tyr Gln Cys Lys Asn Tyr
                85                  90                  95

Tyr Lys Leu Arg Thr Glu Gly Asp Gly Val Tyr Thr Leu Asn Asn Glu
            100                 105                 110

Lys Gln Trp Ile Asn Lys Ala Val Gly Asp Lys Leu Pro Glu Cys Glu
        115                 120                 125

Ala Val Cys Gly Lys Pro Lys Asn Pro Ala Asn Pro Val Gln
    130                 135                 140


<210>  70
<211>  245
<212>  PRT
<213>  Homo sapiens

<400>  70

Ile Leu Gly Gly His Leu Asp Ala Lys Gly Ser Phe Pro Trp Gln Ala
1               5                   10                  15

Lys Met Val Ser His His Asn Leu Thr Thr Gly Ala Thr Leu Ile Asn
            20                  25                  30

Glu Gln Trp Leu Leu Thr Thr Ala Lys Asn Leu Phe Leu Asn His Ser
        35                  40                  45

Glu Asn Ala Thr Ala Lys Asp Ile Ala Pro Thr Leu Thr Leu Tyr Val
    50                  55                  60

Gly Lys Lys Gln Leu Val Glu Ile Glu Lys Val Val Leu His Pro Asn
65                  70                  75                  80

Tyr Ser Gln Val Asp Ile Gly Leu Ile Lys Leu Lys Gln Lys Val Ser
                85                  90                  95

Val Asn Glu Arg Val Met Pro Ile Cys Leu Pro Ser Lys Asp Tyr Ala
            100                 105                 110

Glu Val Gly Arg Val Gly Tyr Val Ser Gly Trp Gly Arg Asn Ala Asn
        115                 120                 125

Phe Lys Phe Thr Asp His Leu Lys Tyr Val Met Leu Pro Val Ala Asp
    130                 135                 140

Gln Asp Gln Cys Ile Arg His Tyr Glu Gly Ser Thr Val Pro Glu Lys
145                 150                 155                 160
```

```
Lys Thr Pro Lys Ser Pro Val Gly Val Gln Pro Ile Leu Asn Glu His
                165                 170                 175

Thr Phe Cys Ala Gly Met Ser Lys Tyr Gln Glu Asp Thr Cys Tyr Gly
            180                 185                 190

Asp Ala Gly Ser Ala Phe Ala Val His Asp Leu Glu Glu Asp Thr Trp
        195                 200                 205

Tyr Ala Thr Gly Ile Leu Ser Phe Asp Lys Ser Cys Ala Val Ala Glu
    210                 215                 220

Tyr Gly Val Tyr Val Lys Val Thr Ser Ile Gln Asp Trp Val Gln Lys
225                 230                 235                 240

Thr Ile Ala Glu Asn
                245
```

<210> 71
<211> 83
<212> PRT
<213> Homo sapiens

<400> 71

```
Val Asp Ser Gly Asn Asp Val Thr Asp Ile Ala Asp Asp Gly Cys Pro
1               5                   10                  15

Lys Pro Pro Glu Ile Ala His Gly Tyr Val Glu His Ser Val Arg Tyr
            20                  25                  30

Gln Cys Lys Asn Tyr Tyr Lys Leu Arg Thr Glu Gly Asp Gly Val Tyr
        35                  40                  45

Thr Leu Asn Asn Glu Lys Gln Trp Ile Asn Lys Ala Val Gly Asp Lys
    50                  55                  60

Leu Pro Glu Cys Glu Ala Val Cys Gly Lys Pro Lys Asn Pro Ala Asn
65                  70                  75                  80

Pro Val Gln
```

<210> 72
<211> 999
<212> DNA
<213> Artificial

<220>

<223> hCg1-Xen-236-239-332

<400> 72
gctagcacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctggcggga 360

ccggatgtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccgaggagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa tgaggatcc 999

<210> 73
<211> 401
<212> PRT
<213> Artificial

<220>
<223> bHp_AA

<400> 73

Met Ser Ala Leu Gln Ala Val Val Thr Leu Leu Leu Cys Gly Gln Leu
1 5 10 15

Leu Ala Val Glu Thr Gly Ser Glu Ala Thr Ala Asp Ser Cys Pro Lys
20 25 30

Ala Pro Glu Ile Ala Asn Ser His Val Glu Tyr Ser Val Arg Tyr Gln
35 40 45

Cys Asp Lys Tyr Tyr Lys Leu His Ala Gly Asn Gly Val Tyr Thr Phe
50 55 60

Asn Asn Lys Gln Trp Ile Asn Lys Asp Ile Gly Gln Gln Leu Pro Glu
65 70 75 80

Cys Glu Glu Asp Asp Ser Cys Pro Glu Pro Pro Lys Ile Glu Asn Gly
85 90 95

Tyr Val Glu Tyr Leu Val Arg Tyr Gln Cys Lys Pro Tyr Tyr Thr Leu
100 105 110

Arg Thr Cys Gly Asp Gly Val Tyr Thr Phe Asn Ser Lys Lys Gln Trp
115 120 125

Ile Asn Lys Asn Ile Gly Gln Lys Leu Pro Glu Cys Glu Ala Val Cys
130 135 140

Gly Lys Pro Lys His Pro Val Asp Gln Val Gln Arg Ile Ile Gly Gly
145 150 155 160

Ser Leu Asp Ala Lys Gly Ser Phe Pro Trp Gln Ala Lys Met Val Ser
165 170 175

Gln His Asn Leu Ile Ser Gly Ala Thr Leu Ile Asn Glu Arg Trp Leu
180 185 190

Leu Thr Thr Ala Lys Asn Leu Tyr Leu Gly His Ser Ser Asp Lys Lys
195 200 205

Ala Lys Asp Ile Thr Pro Thr Leu Arg Leu Tyr Val Gly Lys Asn Gln
210 215 220

Leu Val Glu Val Glu Lys Val Val Leu His Pro Asp His Ser Lys Val
225 230 235 240

Asp Ile Gly Leu Ile Lys Leu Arg Gln Lys Val Pro Val Asn Asp Lys
245 250 255

Val Met Pro Ile Cys Leu Pro Ser Lys Asp Tyr Val Lys Val Gly Arg
260 265 270

Val Gly Tyr Val Ser Gly Trp Gly Arg Asn Glu Asn Phe Asn Phe Thr
275 280 285

Glu His Leu Lys Tyr Val Met Leu Pro Val Ala Asp Gln Asp Lys Cys
290 295 300

Val Lys His Tyr Glu Gly Val Asp Ala Pro Lys Asn Lys Thr Ala Lys
305 310 315 320

Ser Pro Val Gly Val Gln Pro Ile Leu Asn Glu Asn Thr Phe Cys Val
325 330 335

Gly Leu Ser Lys Tyr Gln Asp Asp Thr Cys Tyr Gly Asp Ala Gly Ser
340 345 350

Ala Phe Val Val His Asp Lys Glu Asp Asp Thr Trp Tyr Ala Ala Gly
355 360 365

Ile Leu Ser Phe Asp Lys Ser Cys Ala Val Ala Glu Tyr Gly Val Tyr
370 375 380

Val Lys Val Thr Ser Ile Leu Asp Trp Val Arg Lys Thr Ile Ala Asn
385 390 395 400

Asn

<210> 74
<211> 347
<212> PRT
<213> Artificial

<220>
<223> mHp_AA

<400> 74

Met Arg Ala Leu Gly Ala Val Val Thr Leu Leu Leu Trp Gly Gln Leu
1 5 10 15

Phe Ala Val Glu Leu Gly Asn Asp Ala Met Asp Phe Glu Asp Asp Ser
20 25 30

Cys Pro Lys Pro Pro Glu Ile Ala Asn Gly Tyr Val Glu His Leu Val
35 40 45

Arg Tyr Arg Cys Arg Gln Phe Tyr Arg Leu Arg Ala Glu Gly Asp Gly
50 55 60

Val Tyr Thr Leu Asn Asp Glu Lys Gln Trp Val Asn Thr Val Ala Gly
65 70 75 80

Glu Lys Leu Pro Glu Cys Glu Ala Val Cys Gly Lys Pro Lys His Pro
85 90 95

Val Asp Gln Val Gln Arg Ile Ile Gly Gly Ser Met Asp Ala Lys Gly
100 105 110

```
Ser Phe Pro Trp Gln Ala Lys Met Ile Ser Arg His Gly Leu Thr Thr
        115                 120                 125

Gly Ala Thr Leu Ile Ser Asp Gln Trp Leu Leu Thr Thr Ala Lys Asn
    130                 135                 140

Leu Phe Leu Asn His Ser Glu Thr Ala Ser Ala Lys Asp Ile Thr Pro
145                 150                 155                 160

Thr Leu Thr Leu Tyr Val Gly Lys Asn Gln Leu Val Glu Ile Glu Lys
                165                 170                 175

Val Val Leu His Pro Asn His Ser Val Val Asp Ile Gly Leu Ile Lys
            180                 185                 190

Leu Lys Gln Arg Val Leu Val Thr Glu Arg Val Met Pro Ile Cys Leu
        195                 200                 205

Pro Ser Lys Asp Tyr Ile Ala Pro Gly Arg Val Gly Tyr Val Ser Gly
    210                 215                 220

Trp Gly Arg Asn Ala Asn Phe Arg Phe Thr Asp Arg Leu Lys Tyr Val
225                 230                 235                 240

Met Leu Pro Val Ala Asp Gln Asp Lys Cys Val Val His Tyr Glu Asn
                245                 250                 255

Ser Thr Val Pro Glu Lys Lys Asn Leu Thr Ser Pro Val Gly Val Gln
            260                 265                 270

Pro Ile Leu Asn Glu His Thr Phe Cys Ala Gly Leu Thr Lys Tyr Gln
        275                 280                 285

Glu Asp Thr Cys Tyr Gly Asp Ala Gly Ser Ala Phe Ala Ile His Asp
    290                 295                 300

Met Glu Glu Asp Thr Trp Tyr Ala Ala Gly Ile Leu Ser Phe Asp Lys
305                 310                 315                 320

Ser Cys Ala Val Ala Glu Tyr Gly Val Tyr Val Arg Ala Thr Asp Leu
                325                 330                 335

Lys Asp Trp Val Gln Glu Thr Met Ala Lys Asn
            340                 345
```

<210> 75
<211> 355

<212> PRT
<213> Artificial

<220>
<223> chimeraA_AA

<400> 75

```
Met Ser Ala Leu Gly Ala Val Ile Ala Leu Leu Leu Trp Gly Gln Leu
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
    50                  55                  60

Val Tyr Thr Leu Asn Asn Glu Lys Gln Trp Ile Asn Lys Ala Val Gly
65                  70                  75                  80

Asp Lys Leu Pro Glu Cys Glu Ala Val Cys Gly Lys Pro Lys Asn Pro
                85                  90                  95

Ala Asn Pro Val Gln Arg Ile Leu Gly Gly His Leu Asp Ala Lys Gly
            100                 105                 110

Ser Phe Pro Trp Gln Ala Lys Met Val Ser His His Asn Leu Thr Thr
        115                 120                 125

Gly Ala Thr Leu Ile Asn Glu Gln Trp Leu Leu Thr Thr Ala Lys Asn
    130                 135                 140

Leu Phe Leu Asn His Ser Glu Asn Ala Thr Ala Lys Asp Ile Ala Pro
145                 150                 155                 160

Thr Leu Thr Leu Tyr Val Gly Lys Lys Gln Leu Val Glu Ile Glu Lys
                165                 170                 175

Val Val Leu His Pro Asn Tyr Ser Gln Val Asp Ile Gly Leu Ile Lys
            180                 185                 190

Leu Lys Gln Lys Val Ser Val Asn Glu Arg Val Met Pro Ile Cys Leu
        195                 200                 205

Pro Ser Lys Asp Tyr Ala Glu Val Gly Arg Val Gly Tyr Val Ser Gly
    210                 215                 220
```

Trp Gly Arg Asn Ala Asn Phe Lys Phe Thr Asp His Leu Lys Tyr Val
225 230 235 240

Met Leu Pro Val Ala Asp Gln Asp Gln Cys Ile Arg His Tyr Glu Gly
245 250 255

Val Asp Ala Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 76
<211> 355
<212> PRT
<213> Artificial

<220>
<223> chimeraB_AA

<400> 76

Met Ser Ala Leu Gly Ala Val Ile Ala Leu Leu Leu Trp Gly Gln Leu
1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

Val Tyr Thr Leu Asn Asn Glu Lys Gln Trp Ile Asn Lys Ala Val Gly
65 70 75 80

Asp Lys Leu Pro Glu Cys Glu Ala Val Cys Gly Lys Pro Lys Asn Pro
85 90 95

Ala Asn Pro Val Gln Arg Ile Leu Gly Gly His Leu Asp Ala Lys Gly
100 105 110

Ser Phe Pro Trp Gln Ala Lys Met Val Ser His His Asn Leu Thr Thr
115 120 125

Gly Ala Thr Leu Ile Asn Glu Arg Trp Leu Leu Thr Thr Ala Lys Asn
130 135 140

Leu Phe Leu Asn His Ser Glu Asn Ala Thr Ala Lys Asp Ile Ala Pro
145 150 155 160

Thr Leu Thr Leu Tyr Val Gly Lys Lys Gln Leu Val Glu Ile Glu Lys
165 170 175

Val Val Leu His Pro Asn Tyr Ser Gln Val Asp Ile Gly Leu Ile Lys
180 185 190

Leu Lys Gln Lys Val Ser Val Asn Glu Arg Val Met Pro Ile Cys Leu
195 200 205

Pro Ser Lys Asp Tyr Ala Glu Val Gly Arg Val Gly Tyr Val Ser Gly
210 215 220

Trp Gly Arg Asn Ala Asn Phe Lys Phe Thr Asp His Leu Lys Tyr Val
225 230 235 240

Met Leu Pro Val Ala Asp Gln Asp Gln Cys Ile Arg His Tyr Glu Gly
245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
                325                 330                 335

Gln Asp Trp Val Arg Lys Thr Ile Ala Glu Asn Val Asp His His His
            340                 345                 350

His His His
        355

<210>  77
<211>  355
<212>  PRT
<213>  Artificial

<220>
<223>  chimeraC_AA

<400>  77

Met Ser Ala Leu Gly Ala Val Ile Ala Leu Leu Leu Trp Gly Gln Leu
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
    50                  55                  60

Val Tyr Thr Leu Asn Asn Glu Lys Gln Trp Ile Asn Lys Ala Val Gly
65                  70                  75                  80

Asp Lys Leu Pro Glu Cys Glu Ala Val Cys Gly Lys Pro Lys Asn Pro
                85                  90                  95

Ala Asn Pro Val Gln Arg Ile Leu Gly Gly His Leu Asp Ala Lys Gly
            100                 105                 110

Ser Phe Pro Trp Gln Ala Lys Met Val Ser His His Asn Leu Ile Thr
        115                 120                 125

Gly Ala Thr Leu Ile Asn Glu Gln Trp Leu Leu Thr Thr Ala Lys Asn
    130                 135                 140

Leu Phe Leu Asn His Ser Glu Asn Ala Thr Ala Lys Asp Ile Ala Pro
145                 150                 155                 160

Thr Leu Thr Leu Tyr Val Gly Lys Lys Gln Leu Val Glu Ile Glu Lys
165 170 175

Val Val Leu His Pro Asn Tyr Ser Gln Val Asp Ile Gly Leu Ile Lys
180 185 190

Leu Lys Gln Lys Val Ser Val Asn Glu Arg Val Met Pro Ile Cys Leu
195 200 205

Pro Ser Lys Asp Tyr Ala Glu Val Gly Arg Val Gly Tyr Val Ser Gly
210 215 220

Trp Gly Arg Asn Ala Asn Phe Lys Phe Thr Glu His Leu Lys Tyr Val
225 230 235 240

Met Leu Pro Val Ala Asp Gln Asp Gln Cys Ile Arg His Tyr Glu Gly
245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 78
<211> 355
<212> PRT
<213> Artificial

<220>
<223> chimeraD_AA

<400> 78

Met Ser Ala Leu Gly Ala Val Ile Ala Leu Leu Leu Trp Gly Gln Leu

```
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
    50                  55                  60

Val Tyr Thr Leu Asn Asn Glu Lys Gln Trp Ile Asn Lys Ala Val Gly
65                  70                  75                  80

Asp Lys Leu Pro Glu Cys Glu Ala Val Cys Gly Lys Pro Lys Asn Pro
                85                  90                  95

Ala Asn Pro Val Gln Arg Ile Leu Gly Gly His Leu Asp Ala Lys Gly
            100                 105                 110

Ser Phe Pro Trp Gln Ala Lys Met Val Ser His His Asn Leu Thr Thr
        115                 120                 125

Gly Ala Thr Leu Ile Asn Glu Gln Trp Leu Leu Thr Thr Ala Lys Asn
    130                 135                 140

Leu Phe Leu Asn His Ser Glu Asn Ala Thr Ala Lys Asp Ile Ala Pro
145                 150                 155                 160

Thr Leu Thr Leu Tyr Val Gly Lys Lys Gln Leu Val Glu Ile Glu Lys
                165                 170                 175

Val Val Leu His Pro Asn Tyr Ser Gln Val Asp Ile Gly Leu Ile Lys
            180                 185                 190

Leu Lys Gln Lys Val Ser Val Asn Glu Lys Val Met Pro Ile Cys Leu
        195                 200                 205

Pro Ser Lys Asp Tyr Ala Glu Val Gly Arg Val Gly Tyr Val Ser Gly
    210                 215                 220

Trp Gly Arg Asn Ala Asn Phe Lys Phe Thr Glu His Leu Lys Tyr Val
225                 230                 235                 240

Met Leu Pro Val Ala Asp Gln Asp Gln Cys Ile Arg His Tyr Glu Gly
                245                 250                 255
```

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 79
<211> 355
<212> PRT
<213> Artificial

<220>
<223> chimeraE_AA

<400> 79

Met Ser Ala Leu Gly Ala Val Ile Ala Leu Leu Leu Trp Gly Gln Leu
1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

Val Tyr Thr Leu Asn Asn Glu Lys Gln Trp Ile Asn Lys Ala Val Gly
65 70 75 80

Asp Lys Leu Pro Glu Cys Glu Ala Val Cys Gly Lys Pro Lys Asn Pro
85 90 95

Ala Asn Pro Val Gln Arg Ile Leu Gly Gly His Leu Asp Ala Lys Gly

100 105 110

Ser Phe Pro Trp Gln Ala Lys Met Val Ser His His Asn Leu Thr Thr
115 120 125

Gly Ala Thr Leu Ile Asn Glu Arg Trp Leu Leu Thr Thr Ala Lys Asn
130 135 140

Leu Phe Leu Asn His Ser Glu Asn Ala Thr Ala Lys Asp Ile Ala Pro
145 150 155 160

Thr Leu Thr Leu Tyr Val Gly Lys Lys Gln Leu Val Glu Ile Glu Lys
165 170 175

Val Val Leu His Pro Asn Tyr Ser Gln Val Asp Ile Gly Leu Ile Lys
180 185 190

Leu Arg Gln Lys Val Ser Val Asn Glu Arg Val Met Pro Ile Cys Leu
195 200 205

Pro Ser Lys Asp Tyr Ala Glu Val Gly Arg Val Gly Tyr Val Ser Gly
210 215 220

Trp Gly Arg Asn Ala Asn Phe Lys Phe Thr Asp His Leu Lys Tyr Val
225 230 235 240

Met Leu Pro Val Ala Asp Gln Asp Gln Cys Ile Arg His Tyr Glu Gly
245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

```
His His His
        355


<210>  80
<211>  355
<212>  PRT
<213>  Artificial

<220>
<223>  chimeraF_AA

<400>  80

Met Ser Ala Leu Gly Ala Val Ile Ala Leu Leu Leu Trp Gly Gln Leu
1               5                   10                  15


Phe Ala Val Asp Ser Gly Asn Asp Val Thr Asp Ile Ala Asp Asp Gly
            20                  25                  30


Cys Pro Lys Pro Pro Glu Ile Ala His Gly Tyr Val Glu His Ser Val
        35                  40                  45


Arg Tyr Gln Cys Lys Asn Tyr Tyr Lys Leu Arg Thr Glu Gly Asp Gly
    50                  55                  60


Val Tyr Thr Leu Asn Asn Glu Lys Gln Trp Ile Asn Lys Ala Val Gly
65                  70                  75                  80


Asp Lys Leu Pro Glu Cys Glu Ala Val Cys Gly Lys Pro Lys Asn Pro
                85                  90                  95


Ala Asn Pro Val Gln Arg Ile Leu Gly Gly His Leu Asp Ala Lys Gly
            100                 105                 110


Ser Phe Pro Trp Gln Ala Lys Met Val Ser His His Asn Leu Thr Thr
        115                 120                 125


Gly Ala Thr Leu Ile Asn Glu Gln Trp Leu Leu Thr Thr Ala Lys Asn
    130                 135                 140


Leu Tyr Leu Asn His Ser Ser Asn Ala Thr Ala Lys Asp Ile Ala Pro
145                 150                 155                 160


Thr Leu Thr Leu Tyr Val Gly Lys Lys Gln Leu Val Glu Ile Glu Lys
                165                 170                 175


Val Val Leu His Pro Asn Tyr Ser Gln Val Asp Ile Gly Leu Ile Lys
            180                 185                 190


Leu Lys Gln Lys Val Ser Val Asn Glu Arg Val Met Pro Ile Cys Leu
```

```
        195                 200                 205

Pro Ser Lys Asp Tyr Ala Glu Val Gly Arg Val Gly Tyr Val Ser Gly
    210                 215                 220

Trp Gly Arg Asn Ala Asn Phe Lys Phe Thr Asp His Leu Lys Tyr Val
225                 230                 235                 240

Met Leu Pro Val Ala Asp Gln Asp Gln Cys Ile Arg His Tyr Glu Gly
                245                 250                 255

Ser Thr Val Pro Glu Lys Lys Thr Pro Lys Ser Pro Val Gly Val Gln
            260                 265                 270

Pro Ile Leu Asn Glu His Thr Phe Cys Ala Gly Met Ser Lys Tyr Gln
        275                 280                 285

Glu Asp Thr Cys Tyr Gly Asp Ala Gly Ser Ala Phe Ala Val His Asp
    290                 295                 300

Leu Glu Glu Asp Thr Trp Tyr Ala Thr Gly Ile Leu Ser Phe Asp Lys
305                 310                 315                 320

Ser Cys Ala Val Ala Glu Tyr Gly Val Tyr Val Lys Val Thr Ser Ile
                325                 330                 335

Gln Asp Trp Val Gln Lys Thr Ile Ala Glu Asn Val Asp His His His
            340                 345                 350

His His His
        355


<210>  81
<211>  1105
<212>  DNA
<213>  Artificial

<220>
<223>  chimeraA_DNA

<400>  81
ctagcctcga gaattcccac catgtccgca ctcggggctg tgattgccct tctcctttgg      60

ggacagttgt ttgcagtaga tagcggtaac gacgttacag atatcgcgga tgacggttgc     120

cctaaacccc ctgagattgc acacggttat gtagaacatt cagtgaggta tcaatgtaaa     180

aactactaca aactgcgaac cgaaggggat ggagtttata cattgaacaa tgaaaagcag     240

tggattaaca aggctgtagg tgataagttg cctgagtgtg aagccgtgtg cgggaagcct     300

aagaacccgg cgaaccccgt acagagaatc ctcggtggac atctcgacgc taagggctca     360
```

tttccctggc aggctaagat ggtcagccat cacaatctta ccacgggggc gaccctgatt 420

aatgagcaat ggctgctgac aactgccaaa aaccttttcc ttaatcacag cgagaatgct 480

actgcaaagg acatcgcgcc gacactgacg ctctacgtcg gtaagaaaca gcttgtagag 540

atcgaaaagg tggtcctgca ccccaattac tctcaagtgg atataggttt gataaagctc 600

aagcagaaag tctcagttaa tgagcgcgta atgcccatct gtctcccgtc caaggattat 660

gctgaagttg ggagggttgg ctatgtgtcc ggttggggtc ggaatgccaa ctttaagttt 720

actgaccact tgaagtacgt catgctcccg gtcgcagacc aggaccaatg catcagacac 780

tacgagggag tcgacgcacc tgaaaaaaag acgcctaaaa gccctgtggg agtacagccc 840

atcttgaacg agcatacatt ctgtgctgga atgagcaaat accaggagga tacctgttat 900

ggtgatgcag gtagtgcctt tgccgtacat gacctcgaag aagatacttg gtacgctact 960

ggcatactgt cttttgataa gtcctgtgcg gtcgctgagt atggcgtcta tgtcaaggta 1020

acatctattc aagactgggt ccagaagaca atagcggaaa atgtggacca tcatcatcac 1080

caccactgag tcgacccggg cggcc 1105

<210> 82
<211> 1105
<212> DNA
<213> Artificial

<220>
<223> chimeraB_DNA

<400> 82
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

tggattaaca aggctgtagg tgataagttg cctgagtgtg aagccgtgtg cgggaagcct 300

aagaacccgg cgaaccccgt acagagaata ctcggagggc accttgatgc aaagggatca 360

ttcccgtggc aggccaaaat ggtgtctcac cacaacctta caaccggtgc aaccctgatc 420

aatgaacggt ggctcttgac gactgcgaag aacttgtttt tgaatcatag cgagaacgcc 480

acagcaaagg acatagctcc taccctcacg ctttacgtgg gcaaaaagca gcttgtagag 540

atagaaaagg tcgtattgca ccctaattac tcacaagtag acatcggact tattaagctt 600

aagcaaaagg tgtccgtaaa tgaacgagtc atgcccatct gtctgccgtc taaagattac 660

gccgaggttg gcagggtggg atacgtgtcc gggtgggggc gaaacgccaa cttcaagttc 720

accgatcatc tcaagtatgt catgcttcct gtagccgatc aggatcagtg catacgacac 780

tacgagggga gcacggtccc agagaaaaaa acacctaaat ctcccgtcgg ggtccaacca 840

atactcaacg aacatacatt ttgcgccggt atgtccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccgaaaaact attgctgaga acgtggacca tcatcatcac 1080

caccactgag tcgacccggg cggcc 1105

<210> 83
<211> 1105
<212> DNA
<213> Artificial

<220>
<223> chimeraC_DNA

<400> 83
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

tggattaaca aggctgtagg tgataagttg cctgagtgtg aagccgtgtg cgggaagcct 300

aagaacccgg cgaaccccgt acagagaata ctcggagggc accttgatgc aaagggatca 360

ttcccgtggc aggccaaaat ggtgtctcac cacaacctta ttaccggtgc aaccctgatc 420

aatgaacaat ggctcttgac gactgcgaag aacttgtttt tgaatcatag cgagaacgcc 480

acagcaaagg acatagctcc taccctcacg ctttacgtgg gcaaaaagca gcttgtagag 540

atagaaaagg tcgtattgca ccctaattac tcacaagtag acatcggact tattaagctt 600

aagcaaaagg tgtccgtaaa tgaacgagtc atgcccatct gtctgccgtc taaagattac 660

gccgaggttg gcagggtggg atacgtgtcc gggtgggggc gaaacgccaa cttcaagttc 720

accgagcatc tcaagtatgt catgcttcct gtagccgatc aggatcagtg catacgacac 780

tacgagggga gcacggtccc agagaaaaaa acacctaaat ctcccgtcgg ggtccaacca 840

atactcaacg aacatacatt ttgcgccggt atgtccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccagaaaact attgctgaga acgtggacca tcatcatcac 1080

caccactgag tcgacccggg cggcc 1105

<210> 84
<211> 1105

<212> DNA
<213> Artificial

<220>
<223> chimeraD_DNA

<400> 84
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

tggattaaca aggctgtagg tgataagttg cctgagtgtg aagccgtgtg cgggaagcct 300

aagaacccgg cgaaccccgt acagagaata ctcggagggc accttgatgc aaagggatca 360

ttcccgtggc aggccaaaat ggtgtctcac cacaacctta caaccggtgc aaccctgatc 420

aatgaacaat ggctcttgac gactgcgaag aacttgtttt tgaatcatag cgagaacgcc 480

acagcaaagg acatagctcc taccctcacg ctttacgtgg gcaaaaagca gcttgtagag 540

atagaaaagg tcgtattgca ccctaattac tcacaagtag acatcggact tattaagctt 600

aagcaaaagg tgtccgtaaa tgaaaaggtc atgcccatct gtctgccgtc taaagattac 660

gccgaggttg gcagggtggg atacgtgtcc gggtgggggc gaaacgccaa cttcaagttc 720

accgaacatc tcaagtatgt catgcttcct gtagccgatc aggatcagtg catacgacac 780

tacgagggga gcacggtccc agagaaaaaa acacctaaat ctcccgtcgg ggtccaacca 840

atactcaacg aacatacatt ttgcgccggt atgtccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccagaaaact attgctgaga acgtggacca tcatcatcac 1080

caccactgag tcgacccggg cggcc 1105

<210> 85
<211> 1105
<212> DNA
<213> Artificial

<220>
<223> chimeraE_DNA

<400> 85
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

```
tggattaaca aggctgtagg tgataagttg cctgagtgtg aagccgtgtg cgggaagcct     300

aagaacccgg cgaaccccgt acagagaata ctcggagggc accttgatgc aaagggatca     360

ttcccgtggc aggccaaaat ggtgtctcac cacaacctta caaccggtgc aaccctgatc     420

aatgaaaggt ggctcttgac gactgcgaag aacttgtttt tgaatcatag cgagaacgcc     480

acagcaaagg acatagctcc taccctcacg ctttacgtgg gcaaaaagca gcttgtagag     540

atagaaaagg tcgtattgca ccctaattac tcacaagtag acatcggact tattaagctt     600

aggcaaaagg tgtccgtaaa tgaacgagtc atgcccatct gtctgccgtc taaagattac     660

gccgaggttg gcagggtggg atacgtgtcc gggtggggc gaaacgccaa cttcaagttc     720

accgatcatc tcaagtatgt catgcttcct gtagccgatc aggatcagtg catacgacac     780

tacgagggga gcacggtccc agagaaaaa acacctaaat ctcccgtcgg ggtccaacca     840

atactcaacg aacatacatt ttgcgccggt atgtccaagt atcaagaaga cacctgctac     900

ggtgatgccg gtagtgcttt tgctgtgcat gatcttgagg aggacacctg gtatgcgaca     960

gggattctca gttttgacaa gtcttgtgca gtggctgaat atggggtgta cgtcaaagtc    1020

acgtccatcc aggactgggt ccagaaaact attgctgaga acgtggacca tcatcatcac    1080

caccactgag tcgacccggg cggcc                                          1105
```

<210> 86
<211> 1105
<212> DNA
<213> Artificial

<220>
<223> chimeraF_DNA

<400> 86

```
ctagcctcga gaattcccac catgtccgca ctcggggctg tgattgccct tctcctttgg      60

ggacagttgt ttgcagtaga tagcggtaac gacgttacag atatcgcgga tgacggttgc     120

cctaaacccc ctgagattgc acacggttat gtagaacatt cagtgaggta tcaatgtaaa     180

aactactaca aactgcgaac cgaaggggat ggagtttata cattgaacaa tgaaaagcag     240

tggattaaca aggctgtagg tgataagttg cctgagtgtg aagccgtgtg cgggaagcct     300

aagaacccgg cgaaccccgt acagagaata ctcggagggc accttgatgc aaagggatca     360

ttcccgtggc aggccaaaat ggtgtctcac cacaacctta caaccggtgc aaccctgatc     420

aatgaacaat ggctcttgac gactgcgaag aacttgtact tgaatcatag ctccaacgcc     480

acagcaaagg acatagctcc taccctcacg ctttacgtgg gcaaaaagca gcttgtagag     540

atagaaaagg tcgtattgca ccctaattac tcacaagtag acatcggact tattaagctt     600

aagcaaaagg tgtccgtaaa tgaacgagtc atgcccatct gtctgccgtc taaagattac     660

gccgaggttg gcagggtggg atacgtgtcc gggtggggc gaaacgccaa cttcaagttc     720
```

accgatcatc tcaagtatgt catgcttcct gtagccgatc aggatcagtg catacgacac 780

tacgagggga gcacggtccc agagaaaaaa acacctaaat ctcccgtcgg ggtccaacca 840

atactcaacg aacatacatt ttgcgccggt atgtccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccagaaaact attgctgaga acgtggacca tcatcatcac 1080

caccactgag tcgacccggg cggcc 1105

<210> 87
<211> 355
<212> PRT
<213> Artificial

<220>
<223> rhHp_AA

<400> 87

Met Ser Ala Leu Gly Ala Val Ile Ala Leu Leu Leu Trp Gly Gln Leu
1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

Val Tyr Thr Leu Asn Asn Glu Lys Gln Trp Ile Asn Lys Ala Val Gly
65 70 75 80

Asp Lys Leu Pro Glu Cys Glu Ala Val Cys Gly Lys Pro Lys Asn Pro
85 90 95

Ala Asn Pro Val Gln Arg Ile Leu Gly Gly His Leu Asp Ala Lys Gly
100 105 110

Ser Phe Pro Trp Gln Ala Lys Met Val Ser His His Asn Leu Thr Thr
115 120 125

Gly Ala Thr Leu Ile Asn Glu Gln Trp Leu Leu Thr Thr Ala Lys Asn
130 135 140

Leu Phe Leu Asn His Ser Glu Asn Ala Thr Ala Lys Asp Ile Ala Pro

145 150 155 160

Thr Leu Thr Leu Tyr Val Gly Lys Lys Gln Leu Val Glu Ile Glu Lys
165 170 175

Val Val Leu His Pro Asn Tyr Ser Gln Val Asp Ile Gly Leu Ile Lys
180 185 190

Leu Lys Gln Lys Val Ser Val Asn Glu Arg Val Met Pro Ile Cys Leu
195 200 205

Pro Ser Lys Asp Tyr Ala Glu Val Gly Arg Val Gly Tyr Val Ser Gly
210 215 220

Trp Gly Arg Asn Ala Asn Phe Lys Phe Thr Asp His Leu Lys Tyr Val
225 230 235 240

Met Leu Pro Val Ala Asp Gln Asp Gln Cys Ile Arg His Tyr Glu Gly
245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 88
<211> 1105
<212> DNA
<213> Artificial

<220>
<223> rhHp_DNA

<400> 88
```
ctagcctcga gaattcccac catgtccgca ctcggggctg tgattgccct tctcctttgg      60
ggacagttgt ttgcagtaga tagcggtaac gacgttacag atatcgcgga tgacggttgc     120
cctaaacccc ctgagattgc acacggttat gtagaacatt cagtgaggta tcaatgtaaa     180
aactactaca aactgcgaac cgaaggggat ggagtttata cattgaacaa tgaaaagcag     240
tggattaaca aggctgtagg tgataagttg cctgagtgtg aagccgtgtg cgggaagcct     300
aagaacccgg cgaacccccgt acagagaata ctcggagggc accttgatgc aaagggatca     360
ttcccgtggc aggccaaaat ggtgtctcac cacaacctta caaccggtgc aaccctgatc     420
aatgaacaat ggctcttgac gactgcgaag aacttgtttt tgaatcatag cgagaacgcc     480
acagcaaagg acatagctcc taccctcacg ctttacgtgg gcaaaaagca gcttgtagag     540
atagaaaagg tcgtattgca ccctaattac tcacaagtag acatcggact tattaagctt     600
aagcaaaagg tgtccgtaaa tgaacgagtc atgcccatct gtctgccgtc taaagattac     660
gccgaggttg gcagggtggg atacgtgtcc gggtgggggc gaaacgccaa cttcaagttc     720
accgatcatc tcaagtatgt catgcttcct gtagccgatc aggatcagtg catacgacac     780
tacgagggga gcacggtccc agagaaaaaa acacctaaat ctcccgtcgg ggtccaacca     840
atactcaacg aacatacatt ttgcgccggt atgtccaagt atcaagaaga cacctgctac     900
ggtgatgccg gtagtgcttt tgctgtgcat gatcttgagg aggacacctg gtatgcgaca     960
gggattctca gttttgacaa gtcttgtgca gtggctgaat atggggtgta cgtcaaagtc    1020
acgtccatcc aggactgggt ccagaaaact attgctgaga acgtggacca tcatcatcac    1080
caccactgag tcgacccggg cggcc                                          1105
```

<210> 89
<211> 355
<212> PRT
<213> Artificial

<220>
<223> chimeraG_AA

<400> 89

```
Met Ser Ala Leu Gly Ala Val Ile Ala Leu Leu Leu Trp Gly Gln Leu
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
```

```
        50                  55                  60

Val Tyr Thr Leu Asn Asn Glu Lys Gln Trp Ile Asn Lys Ala Val Gly
65                  70                  75                  80

Asp Lys Leu Pro Glu Cys Glu Ala Val Cys Gly Lys Pro Lys Asn Pro
                85                  90                  95

Ala Asn Pro Val Gln Arg Ile Ile Gly Gly Ser Leu Asp Ala Lys Gly
            100                 105                 110

Ser Phe Pro Trp Gln Ala Lys Met Val Ser His His Asn Leu Thr Thr
        115                 120                 125

Gly Ala Thr Leu Ile Asn Glu Gln Trp Leu Leu Thr Thr Ala Lys Asn
    130                 135                 140

Leu Phe Leu Asn His Ser Glu Asn Ala Thr Ala Lys Asp Ile Ala Pro
145                 150                 155                 160

Thr Leu Thr Leu Tyr Val Gly Lys Lys Gln Leu Val Glu Ile Glu Lys
                165                 170                 175

Val Val Leu His Pro Asn Tyr Ser Gln Val Asp Ile Gly Leu Ile Lys
            180                 185                 190

Leu Lys Gln Lys Val Ser Val Asn Glu Arg Val Met Pro Ile Cys Leu
        195                 200                 205

Pro Ser Lys Asp Tyr Ala Glu Val Gly Arg Val Gly Tyr Val Ser Gly
    210                 215                 220

Trp Gly Arg Asn Ala Asn Phe Lys Phe Thr Asp His Leu Lys Tyr Val
225                 230                 235                 240

Met Leu Pro Val Ala Asp Gln Asp Gln Cys Ile Arg His Tyr Glu Gly
                245                 250                 255

Ser Thr Val Pro Glu Lys Lys Thr Pro Lys Ser Pro Val Gly Val Gln
            260                 265                 270

Pro Ile Leu Asn Glu His Thr Phe Cys Ala Gly Leu Ser Lys Tyr Gln
        275                 280                 285

Glu Asp Thr Cys Tyr Gly Asp Ala Gly Ser Ala Phe Ala Val His Asp
    290                 295                 300
```

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 90
<211> 355
<212> PRT
<213> Artificial

<220>
<223> chimeraH_AA

<400> 90

Met Ser Ala Leu Gly Ala Val Ile Ala Leu Leu Leu Trp Gly Gln Leu
1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

Val Tyr Thr Leu Asn Asn Glu Lys Gln Trp Ile Asn Lys Ala Val Gly
65 70 75 80

Asp Lys Leu Pro Glu Cys Glu Ala Val Cys Gly Lys Pro Lys Asn Pro
85 90 95

Ala Asn Pro Val Gln Arg Ile Leu Gly Gly His Leu Asp Ala Lys Gly
100 105 110

Ser Phe Pro Trp Gln Ala Lys Met Val Ser Arg His Asn Leu Thr Thr
115 120 125

Gly Ala Thr Leu Ile Asn Glu Gln Trp Leu Leu Thr Thr Ala Lys Asn
130 135 140

Leu Phe Leu Asn His Ser Glu Asn Ala Ser Ala Lys Asp Ile Ala Pro

145 150 155 160

Thr Leu Thr Leu Tyr Val Gly Lys Lys Gln Leu Val Glu Ile Glu Lys
165 170 175

Val Val Leu His Pro Asn His Ser Val Val Asp Ile Gly Leu Ile Lys
180 185 190

Leu Lys Gln Lys Val Ser Val Asn Glu Arg Val Met Pro Ile Cys Leu
195 200 205

Pro Ser Lys Asp Tyr Ala Glu Val Gly Arg Val Gly Tyr Val Ser Gly
210 215 220

Trp Gly Arg Asn Ala Asn Phe Lys Phe Thr Asp His Leu Lys Tyr Val
225 230 235 240

Met Leu Pro Val Ala Asp Gln Asp Gln Cys Ile Arg His Tyr Glu Gly
245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 91
<211> 355
<212> PRT
<213> Artificial

<220>
<223> chimeraI_AA

<400> 91

```
Met Ser Ala Leu Gly Ala Val Ile Ala Leu Leu Leu Trp Gly Gln Leu
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
    50                  55                  60

Val Tyr Thr Leu Asn Asn Glu Lys Gln Trp Ile Asn Lys Ala Val Gly
65                  70                  75                  80

Asp Lys Leu Pro Glu Cys Glu Ala Val Cys Gly Lys Pro Lys Asn Pro
                85                  90                  95

Ala Asn Pro Val Gln Arg Ile Leu Gly Gly His Leu Asp Ala Lys Gly
            100                 105                 110

Ser Phe Pro Trp Gln Ala Lys Met Val Ser His His Asn Leu Thr Thr
        115                 120                 125

Gly Ala Thr Leu Ile Asn Glu Gln Trp Leu Leu Thr Thr Ala Lys Asn
    130                 135                 140

Leu Phe Leu Asn His Ser Glu Asn Ala Thr Ala Lys Asp Ile Ala Pro
145                 150                 155                 160

Thr Leu Thr Leu Tyr Val Gly Lys Asn Gln Leu Val Glu Ile Glu Lys
                165                 170                 175

Val Val Leu His Pro Asn Tyr Ser Gln Val Asp Ile Gly Leu Ile Lys
            180                 185                 190

Leu Lys Gln Lys Val Ser Val Asn Glu Arg Val Met Pro Ile Cys Leu
        195                 200                 205

Pro Ser Lys Asp Tyr Ala Glu Val Gly Arg Val Gly Tyr Val Ser Gly
    210                 215                 220

Trp Gly Arg Asn Ala Asn Phe Arg Phe Thr Asp His Leu Lys Tyr Val
225                 230                 235                 240

Met Leu Pro Val Ala Asp Gln Asp Gln Cys Ile Arg His Tyr Glu Gly
```

245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Met Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His
340 345 350

His His His
355

<210> 92
<211> 355
<212> PRT
<213> Artificial

<220>
<223> chimeraJ_AA

<400> 92

Met Ser Ala Leu Gly Ala Val Ile Ala Leu Leu Leu Trp Gly Gln Leu
1 5 10 15

Phe Ala Val Asp Ser Gly Asn Asp Val Thr Asp Ile Ala Asp Asp Gly
20 25 30

Cys Pro Lys Pro Pro Glu Ile Ala His Gly Tyr Val Glu His Ser Val
35 40 45

Arg Tyr Gln Cys Lys Asn Tyr Tyr Lys Leu Arg Thr Glu Gly Asp Gly
50 55 60

Val Tyr Thr Leu Asn Asn Glu Lys Gln Trp Ile Asn Lys Ala Val Gly
65 70 75 80

Asp Lys Leu Pro Glu Cys Glu Ala Val Cys Gly Lys Pro Lys Asn Pro
85 90 95

Ala Asn Pro Val Gln Arg Ile Leu Gly Gly His Leu Asp Ala Lys Gly
100 105 110

Ser Phe Pro Trp Gln Ala Lys Met Val Ser His His Asn Leu Thr Thr
115 120 125

Gly Ala Thr Leu Ile Asn Glu Gln Trp Leu Leu Thr Thr Ala Lys Asn
130 135 140

Leu Phe Leu Asn His Ser Glu Asn Ala Thr Ala Lys Asp Ile Ala Pro
145 150 155 160

Thr Leu Thr Leu Tyr Val Gly Lys Lys Gln Leu Val Glu Ile Glu Lys
165 170 175

Val Val Leu His Pro Asn Tyr Ser Gln Val Asp Ile Gly Leu Ile Lys
180 185 190

Leu Lys Gln Lys Val Ser Val Asn Glu Arg Val Met Pro Ile Cys Leu
195 200 205

Pro Ser Lys Asp Tyr Ala Ala Val Gly Arg Val Gly Tyr Val Ser Gly
210 215 220

Trp Gly Arg Asn Ala Asn Phe Lys Phe Thr Asp His Leu Lys Tyr Val
225 230 235 240

Met Leu Pro Val Ala Asp Gln Asp Lys Cys Ile Arg His Tyr Glu Gly
245 250 255

Ser Thr Val Pro Glu Lys Lys Thr Pro Lys Ser Pro Val Gly Val Gln
260 265 270

Pro Ile Leu Asn Glu His Thr Phe Cys Ala Gly Leu Ser Lys Tyr Gln
275 280 285

Glu Asp Thr Cys Tyr Gly Asp Ala Gly Ser Ala Phe Ala Val His Asp
290 295 300

Leu Glu Glu Asp Thr Trp Tyr Ala Thr Gly Ile Leu Ser Phe Asp Lys
305 310 315 320

Ser Cys Ala Val Ala Glu Tyr Gly Val Tyr Val Lys Val Thr Ser Ile
325 330 335

Gln Asp Trp Val Gln Lys Thr Ile Ala Glu Asn Val Asp His His His

```
            340                 345                 350

His His His
        355


<210>  93
<211>  355
<212>  PRT
<213>  Artificial

<220>
<223>  chimeraK_AA

<400>  93

Met Ser Ala Leu Gly Ala Val Ile Ala Leu Leu Leu Trp Gly Gln Leu
1               5                   10                  15


Phe Ala Val Asp Ser Gly Asn Asp Val Thr Asp Ile Ala Asp Asp Gly
            20                  25                  30


Cys Pro Lys Pro Pro Glu Ile Ala His Gly Tyr Val Glu His Ser Val
        35                  40                  45


Arg Tyr Gln Cys Lys Asn Tyr Tyr Lys Leu Arg Thr Glu Gly Asp Gly
    50                  55                  60


Val Tyr Thr Leu Asn Asn Glu Lys Gln Trp Ile Asn Lys Ala Val Gly
65                  70                  75                  80


Asp Lys Leu Pro Glu Cys Glu Ala Val Cys Gly Lys Pro Lys Asn Pro
                85                  90                  95


Ala Asn Pro Val Gln Arg Ile Leu Gly Gly His Leu Asp Ala Lys Gly
            100                 105                 110


Ser Phe Pro Trp Gln Ala Lys Met Val Ser His His Asn Leu Thr Thr
        115                 120                 125


Gly Ala Thr Leu Ile Asn Glu Gln Trp Leu Leu Thr Thr Ala Lys Asn
    130                 135                 140


Leu Phe Leu Asn His Ser Glu Asn Ala Thr Ala Lys Asp Ile Ala Pro
145                 150                 155                 160


Thr Leu Thr Leu Tyr Val Gly Lys Lys Gln Leu Val Glu Ile Glu Lys
                165                 170                 175


Val Val Leu His Pro Asn Tyr Ser Gln Val Asp Ile Gly Leu Ile Lys
            180                 185                 190
```

Leu Lys Gln Lys Val Ser Val Asn Glu Arg Val Met Pro Ile Cys Leu
        195                 200                 205

Pro Ser Lys Asp Tyr Ala Glu Val Gly Arg Val Gly Tyr Val Ser Gly
    210                 215                 220

Trp Gly Arg Asn Ala Asn Phe Lys Phe Thr Asp His Leu Lys Tyr Val
225                 230                 235                 240

Met Leu Pro Val Ala Asp Gln Asp Lys Cys Val Val His Tyr Glu Gly
                245                 250                 255

Ser Thr Val Pro Glu Lys Lys Thr Leu Lys Ser Pro Val Gly Val Gln
            260                 265                 270

Pro Ile Leu Asn Glu His Thr Phe Cys Ala Gly Met Ser Lys Tyr Gln
        275                 280                 285

Glu Asp Thr Cys Tyr Gly Asp Ala Gly Ser Ala Phe Ala Val His Asp
    290                 295                 300

Leu Glu Glu Asp Thr Trp Tyr Ala Thr Gly Ile Leu Ser Phe Asp Lys
305                 310                 315                 320

Ser Cys Ala Val Ala Glu Tyr Gly Val Tyr Val Lys Val Thr Ser Ile
                325                 330                 335

Gln Asp Trp Val Gln Lys Thr Ile Ala Glu Asn Val Asp His His His
            340                 345                 350

His His His
        355

<210> 94
<211> 1105
<212> DNA
<213> Artificial

<220>
<223> chimeraG_DNA

<400> 94
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

```
tggattaaca aggctgtagg tgataagttg cctgagtgtg aagccgtgtg cgggaagcct    300

aagaacccgg cgaaccccgt acagagaata attggagggt ctcttgatgc aaagggatca    360

ttcccgtggc aggccaaaat ggtgtctcac cacaacctta caaccggtgc aaccctgatc    420

aatgaacaat ggctcttgac gactgcgaag aacttgtttt tgaatcatag cgagaacgcc    480

acagcaaagg acatagctcc taccctcacg ctttacgtgg gcaaaaagca gcttgtagag    540

atagaaaagg tcgtattgca ccctaattac tcacaagtag acatcggact tattaagctt    600

aagcaaaagg tgtccgtaaa tgaacgagtc atgcccatct gtctgccgtc taaagattac    660

gccgaggttg gcagggtggg atacgtgtcc gggtggggc gaaacgccaa cttcaagttc    720

accgatcatc tcaagtatgt catgcttcct gtagccgatc aggatcagtg catacgacac    780

tacgagggga gcacggtccc agagaaaaaa acacctaaat ctcccgtcgg ggtccaacca    840

atactcaacg aacatacatt ttgcgccggt ctttccaagt atcaagaaga cacctgctac    900

ggtgatgccg gtagtgcttt tgctgtgcat gatcttgagg aggacacctg gtatgcgaca    960

gggattctca gttttgacaa gtcttgtgca gtggctgaat atggggtgta cgtcaaagtc   1020

acgtccatcc aggactgggt ccagaaaact attgctgaga acgtggacca tcatcatcac   1080

caccactgag tcgacccggg cggcc                                         1105
```

<210> 95
<211> 1105
<212> DNA
<213> Artificial

<220>
<223> chimeraH_DNA

<400> 95

```
ctagcctcga gaattcccac catgtccgca ctcggggctg tgattgccct tctcctttgg     60

ggacagttgt ttgcagtaga tagcggtaac gacgttacag atatcgcgga tgacggttgc    120

cctaaacccc ctgagattgc acacggttat gtagaacatt cagtgaggta tcaatgtaaa    180

aactactaca aactgcgaac cgaaggggat ggagtttata cattgaacaa tgaaaagcag    240

tggattaaca aggctgtagg tgataagttg cctgagtgtg aagccgtgtg cgggaagcct    300

aagaacccgg cgaaccccgt acagagaata ctcggagggc accttgatgc aaagggatca    360

ttcccgtggc aggccaaaat ggtgtctcga cacaacctta caaccggtgc aaccctgatc    420

aatgaacaat ggctcttgac gactgcgaag aacttgtttt tgaatcatag cgagaacgcc    480

agtgcaaagg acatagctcc taccctcacg ctttacgtgg gcaaaaagca gcttgtagag    540

atagaaaagg tcgtattgca ccctaatcat tcagtagtag acatcggact tattaagctt    600

aagcaaaagg tgtccgtaaa tgaacgagtc atgcccatct gtctgccgtc taaagattac    660

gccgaggttg gcagggtggg atacgtgtcc gggtggggc gaaacgccaa cttcaagttc    720
```

accgatcatc tcaagtatgt catgcttcct gtagccgatc aggatcagtg catacgacac 780

tacgagggga gcacggtccc agagaaaaaa acacctaaat ctcccgtcgg ggtccaacca 840

atactcaacg aacatacatt ttgcgccggt atgtccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccagaaaact attgctgaga acgtggacca tcatcatcac 1080

caccactgag tcgacccggg cggcc 1105

<210> 96
<211> 1105
<212> DNA
<213> Artificial

<220>
<223> chimeraI_DNA

<400> 96
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

tggattaaca aggctgtagg tgataagttg cctgagtgtg aagccgtgtg cgggaagcct 300

aagaacccgg cgaaccccgt acagagaata ctcggagggc accttgatgc aaagggatca 360

ttcccgtggc aggccaaaat ggtgtctcac cacaacctta caaccggtgc aaccctgatc 420

aatgaacaat ggctcttgac gactgcgaag aacttgtttt tgaatcatag cgagaacgcc 480

acagcaaagg acatagctcc taccctcacg ctttacgtgg gcaaaaacca gcttgtagag 540

atagaaaagg tcgtattgca ccctaattac tcacaagtag acatcggact tattaagctt 600

aagcaaaagg tgtccgtaaa tgaacgagtc atgcccatct gtctgccgtc taaagattac 660

gccgaggttg gcagggtggg atacgtgtcc gggtgggggc gaaacgccaa cttccgattc 720

accgatcatc tcaagtatgt catgcttcct gtagccgatc aggatcagtg catacgacac 780

tacgagggga gcacggtccc agagaaaaaa acacctaaat ctcccgtcgg ggtccaacca 840

atactcaacg aacatacatt ttgcgccggt atgtccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccagaaaact attgctgaga acgtggacca tcatcatcac 1080

caccactgag tcgacccggg cggcc 1105

<210> 97
<211> 1105
<212> DNA
<213> Artificial

<220>
<223> chimeraJ_DNA

<400> 97
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

aactactaca aactgcgaac cgaaggggat ggagtttata cattgaacaa tgaaaagcag 240

tggattaaca aggctgtagg tgataagttg cctgagtgtg aagccgtgtg cgggaagcct 300

aagaacccgg cgaaccccgt acagagaata ctcggagggc accttgatgc aaagggatca 360

ttcccgtggc aggccaaaat ggtgtctcac cacaacctta caaccggtgc aaccctgatc 420

aatgaacaat ggctcttgac gactgcgaag aacttgtttt tgaatcatag cgagaacgcc 480

acagcaaagg acatagctcc taccctcacg ctttacgtgg gcaaaaagca gcttgtagag 540

atagaaaagg tcgtattgca ccctaattac tcacaagtag acatcggact tattaagctt 600

aagcaaaagg tgtccgtaaa tgaacgagtc atgcccatct gtctgccgtc taaagattac 660

gccgccgttg gcagggtggg atacgtgtcc gggtgggggc gaaacgccaa cttcaagttc 720

accgatcatc tcaagtatgt catgcttcct gtagccgatc aggataagtg catacgacac 780

tacgagggga gcacggtccc agagaaaaaa acacctaaat ctcccgtcgg ggtccaacca 840

atactcaacg aacatacatt ttgcgccggt ctttccaagt atcaagaaga cacctgctac 900

ggtgatgccg gtagtgcttt tgctgtgcat gatcttgagg aggacacctg gtatgcgaca 960

gggattctca gttttgacaa gtcttgtgca gtggctgaat atggggtgta cgtcaaagtc 1020

acgtccatcc aggactgggt ccagaaaact attgctgaga acgtggacca tcatcatcac 1080

caccactgag tcgacccggg cggcc 1105

<210> 98
<211> 1105
<212> DNA
<213> Artificial

<220>
<223> chimeraK_DNA

<400> 98
ctagcctcga gaattcccac catgtccgca ctcggggctg tgattgccct tctcctttgg 60

ggacagttgt ttgcagtaga tagcggtaac gacgttacag atatcgcgga tgacggttgc 120

cctaaacccc ctgagattgc acacggttat gtagaacatt cagtgaggta tcaatgtaaa 180

```
aactactaca aactgcgaac cgaaggggat ggagtttata cattgaacaa tgaaaagcag    240
tggattaaca aggctgtagg tgataagttg cctgagtgtg aagccgtgtg cgggaagcct    300
aagaacccgg cgaaccccgt acagagaata ctcggagggc accttgatgc aaagggatca    360
ttcccgtggc aggccaaaat ggtgtctcac cacaacctta caaccggtgc aaccctgatc    420
aatgaacaat ggctcttgac gactgcgaag aacttgtttt tgaatcatag cgagaacgcc    480
acagcaaagg acatagctcc taccctcacg ctttacgtgg gcaaaaagca gcttgtagag    540
atagaaaagg tcgtattgca ccctaattac tcacaagtag acatcggact tattaagctt    600
aagcaaaagg tgtccgtaaa tgaacgagtc atgcccatct gtctgccgtc taaagattac    660
gccgaggttg gcagggtggg atacgtgtcc gggtgggggc gaaacgccaa cttcaagttc    720
accgatcatc tcaagtatgt catgcttcct gtagccgatc aggataagtg cgttgtccac    780
tacgagggga gcacggtccc agagaaaaaa acactgaaat ctcccgtcgg ggtccaacca    840
atactcaacg aacatacatt ttgcgccggt atgtccaagt atcaagaaga cacctgctac    900
ggtgatgccg gtagtgcttt tgctgtgcat gatcttgagg aggacacctg gtatgcgaca    960
gggattctca gttttgacaa gtcttgtgca gtggctgaat atggggtgta cgtcaaagtc   1020
acgtccatcc aggactgggt ccagaaaact attgctgaga acgtggacca tcatcatcac   1080
caccactgag tcgacccggg cggcc                                         1105
```

## Description

### Technical Field

The present invention relates to an antibody, which forms a polyvalent immune complex by binding to human haptoglobin in blood and exhibits high binding activity to an Fcγ receptor, DNA encoding the antibody, a vector containing the DNA, a transformant obtained by the introduction of the vector, a method for manufacturing an antibody by using the transformant, and a therapeutic agent for treatment of autoimmune diseases containing the antibody as an active component.

### Background Art

Intravenous immunoglobulin (IVIG) is a blood product obtained by purifying IgG derived from the plasma of a healthy person in high purity. For hypogammaglobulinemia or agammaglobulinemia, IVIG is used for the purpose of providing protection against infection, and for autoimmune diseases, IVIG is used for the purpose of suppressing the immune response.

Specific examples of IVIG include Venoglobulin IH from Mitsubishi Tanabe Pharma Corporation., Venilon I from TEIJIN PHARMA LIMITED., Polyglobin N from Japanese Red Cross Society, Gammagard from Baxter, Globenin I from NIHON PHARMACEUTICAL CO., LTD., Privigen from CSL Behring, and the like.

Various theories have been proposed regarding the efficacy mechanism of IVIG. For example, the inhibition of an Fcγ receptor (FcγR) on the surface of a macrophage, the inhibition of phagocytosis by increasing the expression of FcγR II b in a macrophage, the increase of the expression of FcγR II b in a macrophage by sialylated antibody, the inhibition of the growth and the induction of apoptosis of B cells, the inhibition of the production of autoantibodies from B cells, the neutralization of an autoantibody by an anti-idiotypic antibody, the inhibition of complement consumption by an autoantibody, the suppression of the function of cytokines or T cells, and the like have been proposed (Non-Patent Documents 1 and 2).

The efficacy of IVIG based on the inhibition of FcγR is brought about, for example, in treating immune thrombocytopenic purpura [ITP, referred to as idiopathic (immune) thrombocytopenic purpura as well]. In a case where platelets are opsonized by autoantibodies against the platelets and are ingested by macrophages through various FcγRs in the reticuloendothelial system, the onset of ITP occurs (Non-Patent Document 1).

Accordingly, IVIG is considered to inhibit the ingestion of platelets by inhibiting FcγR (Non-Patent Document 3). Actually, it has been confirmed that the FcγR III a antibody (trade name: GMA161) from Macrogenics, Inc exhibits efficacy for ITP, although the development thereof was stopped at Phase I (Clinical Trials. gov NCT00244257) (Non-Patent Document 4).

There is also a hypothesis stating that the inhibition of FcγR by IVIG is further enhanced by components such as various immune complexes (IC) formed of antibodies contained in IVIG and serum proteins or dimers or aggregates of antibodies contained in IVIG (Non-Patent Document 5).

Particularly, Lemieux et al revealed that IVIG contains a component, which reacts with self-serum protein, at a proportion up to about 3%, and a soluble immune complex is formed in a case where the component is mixed with the serum (Non-Patent Documents 6 and 7). Furthermore, it was revealed that the specific activity of the soluble immune complex to a mouse ITP model is higher than that of IVIG (Non-Patent Document 8).

These findings show that the binding of the components contained in IVIG to serum proteins brings about an immunosuppressive effect of suppressing the FcγR-mediated phagocytic function performed on a self-tissue. Actually, a concept of purifying autoantibodies and using them for treating autoimmune diseases has been proposed (Patent Document 1)

In addition, it is known that according to the basic prescription for autoimmune diseases, IVIG is administered for five consecutive days at a daily dose of 0.4 g/kg which is an extremely high dose. The high dose administration raises problems such as a side effect like thrombosis and prolonged administration due to infiltration (Non-Patent Document 9). Accordingly, it is considered that there is a great need for reducing the dose by substituting IVIG with a drug formed of active components having high specific activity.

### Related Art

#### Patent Document

Patent Document 1: US Patent Application Publication No. 2004/0101909

Non-Patent Document

Non-Patent Document 1: Trends in Immunology, 2008. 29(12): p. 608-615
Non-Patent Document 2: Science. 2006, 313(5787): p. 670-673
Non-Patent Document 3: Yasuo Ikeda, The Latest Separate Volume for Medicine, Thrombocytopenia·Thrombocytosis, Saishin Igaku
Non-Patent Document 4: ASH Annual Meeting 2006 Abstract 1074
Non-Patent Document 5: J Clin Invest. 2005, 115(1): p. 25-27
Non-Patent Document 6: Int Immunol. 2004, 16(7): p. 929-936
Non-Patent Document 7: Curr Pharm Des. 2006, 12(2): p. 173-179
Non-Patent Document 8: Br J Haematol. 2004, 127(1): p. 90-96
Non-Patent Document 9: Blood Frontier 2007, 17(1): p. 17-19

Disclosure of Invention

Technical Problem

Identifying the active components contained in IVIG and using them as substitutes for IVIG have been expected to become means that can further simplify the treatment of autoimmune diseases in which IVIG has to be administered at a high dose. Accordingly, an object of the present invention is to provide an antibody which can become a substitute for IVIG and exhibits high binding activity to an Fcγ receptor, DNA encoding the antibody, a vector containing the DNA, a transformant obtained by the introduction of the vector, a method for manufacturing an antibody by using the transformant, and a therapeutic agent for treatment of autoimmune diseases containing the antibody as an active component.

Problems to Be Solved by the Invention

For the purpose of identifying active components from IVIG, the inventors of the present invention tried to separate and identify an IgG component, which is contained in a mixed solution of IVIG and serum and exhibits high binding activity to CD16a, by using a column in which FcγR III a (CD16a) is immobilized.
As a result, as an IgG component from which the high binding activity is detected with excellent reproducibility, an IgG component recognizing haptoglobin (Hp; hereinafter, described as Hp in some cases) which is a serum protein was identified. This component is considered as one of the active components in IVIG.
As a result of making an attempt to obtain an anti-Hp antibody, the inventors succeeded to obtain an anti-Hp antibody, which binds more strongly to CD16a compared to IVIG by forming an immune complex formed of the antibody and Hp, and accomplished the present invention.
The present invention relates to (1) to (22) described below.

(1) A monoclonal antibody which specifically recognizes a β chain of human haptoglobin consisting of an amino acid sequence represented by SEQ ID NO: 70 and forms a polyvalent immune complex by binding to the human haptoglobin.
(2) A monoclonal antibody which specifically recognizes a β chain of the human haptoglobin represented by SEQ ID NO: 70 in competition with one antibody selected from the following (a) to (d) and binds to the same epitope as an epitope included in the human haptoglobin to which the selected antibody binds:

(a) antibody comprising a heavy chain (hereinafter, described as H chain) of an antibody in which complementary determining regions (hereinafter, described as CDRs) 1 to 3 comprise amino acid sequences represented by SEQ ID NOs: 31, 32, and 33, respectively, and a light chain (hereinafter, described as L chain) of an antibody in which CDRs 1 to 3 comprise amino acid sequences represented by SEQ ID NOs: 34, 35, and 36, respectively;
(b) antibody comprising an H chain of an antibody in which CDRs 1 to 3 comprise amino acid sequences represented by SEQ ID NOs: 37, 38, and 39, respectively, and an L chain of an antibody in which CDRs 1 to 3 comprise amino acid sequences represented by SEQ ID NOs: 40,41, and 42, respectively;
(c) antibody comprising an H chain of an antibody comprising an amino acid sequence represented by SEQ ID NO: 25 and an L chain of an antibody comprising an amino acid sequence represented by SEQ ID NO: 26; and
(d) antibody comprising an H chain of an antibody comprising an amino acid sequence represented by SEQ ID NO: 27 and an L chain of an antibody comprising an amino acid sequence represented by SEQ ID NO: 28.

(3) A monoclonal antibody selected from the following (a) to (d),

(a) antibody comprising an H chain of an antibody in which CDRs 1 to 3 comprise amino acid sequences represented by SEQ ID NOs: 31, 32, and 33, respectively, and an L chain of an antibody in which CDRs 1 to 3 comprise amino acid sequences represented by SEQ ID NOs: 34, 35, and 36, respectively;
(b) antibody comprising an H chain of an antibody in which CDRs 1 to 3 comprise amino acid sequences represented by SEQ ID NOs: 37, 38, and 39, respectively, and an L chain of an antibody in which CDRs 1 to 3 comprise amino acid sequences represented by SEQ ID NOs: 40, 41, and 42, respectively;
(c) antibody comprising an H chain of an antibody comprising an amino acid sequence represented by SEQ ID NO: 25 and an L chain of an antibody comprising an amino acid sequence represented by SEQ ID NO: 26; and
(d) antibody comprising an H chain of an antibody comprising an amino acid sequence represented by SEQ ID NO: 27 and an L chain of an antibody comprising an amino acid sequence represented by SEQ ID NO: 28.

(4) The monoclonal antibody according to any one of (1) to (3) which binds to at least Phe at position 44 and Glu at position 49 in the amino acid sequence of the β chain of the human haptoglobin represented by SEQ ID NO: 70.
(5) The monoclonal antibody according to any one of (1) to (3) which binds to at least Ser at position 155, Thr at position 156, and Val at position 157 in the amino acid sequence of the β chain of the human haptoglobin represented by SEQ ID NO: 70.
(6) The monoclonal antibody according to any one of (1) to (5) which is a recombinant antibody.
(7) The monoclonal antibody according to (6) that is a recombinant antibody selected from a human chimeric antibody, a humanized antibody, and a human antibody.
(8) The monoclonal antibody according to any one of (1) to (7),
wherein the polyvalent immune complex formed of the monoclonal antibody binds to at least one human Fcγ receptor selected from the group consisting of a human Fcγ receptor III, a human Fcγ receptor II, and a human Fcγ receptor I.
(9) The monoclonal antibody according to any one of (1) to (8),
wherein the polyvalent immune complex formed of the monoclonal antibody comprises at least two antibody molecules and one human haptoglobin molecule.
(10) The monoclonal antibody according to any one of (1) to (9),
wherein fucose is not bound to N-acetylglucosamine at a reducing terminal of an N-glycoside-linked sugar chain linked to a Fc domain of the antibody.
(11) DNA encoding the monoclonal antibody according to any one of (1) to (10).
(12) A recombinant vector comprising:
the DNA according to (11).
(13) A transformant obtained by introducing the recombinant vector according to (12) into a host cell.
(14) A method for producing the monoclonal antibody according to any one of (1) to (10), comprising:

culturing the transformant according to (13) in a medium such that the monoclonal antibody according to any one of (1) to (10) is generated and accumulated in a culture; and
collecting the antibody from the culture.

(15) A method for treating autoimmune diseases, comprising:
administering the monoclonal antibody according to any one of (1) to (10).
(16) A therapeutic agent for treatment of autoimmune diseases, comprising:

the monoclonal antibody according to any one of (1) to (10); and
a pharmacologically accepted carrier.

(17) A therapeutic agent for treatment of autoimmune diseases, comprising:
a monoclonal antibody binding to human haptoglobin.
(18) The therapeutic agent for treatment of autoimmune diseases according to (17),
wherein the monoclonal antibody is a recombinant antibody.
(19) The therapeutic agent for treatment of autoimmune diseases according to (18),
wherein the monoclonal antibody is a recombinant antibody selected from a human chimeric antibody, a humanized antibody, and a human antibody.
(20) The therapeutic agent for treatment of autoimmune diseases according to any one of (17) to (19),
wherein the monoclonal antibody is an antibody forming a polyvalent immune complex by binding to human haptoglobin.
(21) The therapeutic agent for treatment of autoimmune diseases according to any one of (17) to (20),

wherein the polyvalent immune complex formed of the monoclonal antibody binds to at least one human Fcγ receptor selected from the group consisting of a human Fcγ receptor III, a human Fcγ receptor II, and a human Fcγ receptor I.
(22) The therapeutic agent for treatment of autoimmune diseases according to (20) or (21),
wherein the polyvalent immune complex formed of the monoclonal antibody comprises at least two antibody molecules and one human haptoglobin molecule.

Effects of the Invention

The antibody of the present invention forms a polyvalent immune complex by binding to human haptoglobin. In this way, the antibody binds to CD16a in the Fc domain of the antibody and more potently inhibits the antibody-dependent cellular cytotoxicity (ADCC) reaction compared to IVIG. Therefore, according to the present invention, there is provided a therapeutic agent for treatment of autoimmune diseases containing, as an active component, an antibody binding to the human haptoglobin.

Brief Descriptions of Drawings

Fig. 1A to Fig. 1F show the binding activity of an anti-Hp antibody, an immune complex formed of the anti-Hp antibody and the human Hp, and IVIG to CD16a. The abscissa shows antibody concentration (μg/mL), and the ordinate shows absorbance (415 to 490 nm). Fig. 1A shows the binding activity of an antibody #4, Fig. 1B shows the binding activity of an antibody #6, Fig. 1C shows the binding activity of an antibody #27, Fig. 1D shows the binding activity of an antibody #105, Fig. 1E shows the binding activity of an antibody #96-6, and Fig. 1F shows the binding activity of IVIG. ■ represents the anti-Hp antibody (Fig. 1A to Fig. 1E) or IVIG (Fig. 1F), and □ represents the immune complex formed of the anti-Hp antibody and the human Hp (Fig. 1A to Fig. 1F).
Fig. 2 shows the binding activity of the anti-Hp antibody to the human Hp. The abscissa shows antibody concentration (μg/mL), and the ordinate shows absorbance (405 nm). ■ represents the antibody #6, □ represents the antibody #27, ▲ represents the antibody #105, Δ represents the antibody 96-6, and ○ represents an anti-DNP antibody.
Fig. 3A to Fig. 3E show the binding activity of the anti-Hp antibody and the immune complex formed of the anti-Hp antibody and the human Hp to CD32a. The abscissa shows antibody concentration (μg/mL), and the ordinate shows absorbance (415 to 490 nm). Fig. 3A shows the binding activity of the antibody #4, Fig. 3B shows the binding activity of the antibody #6, Fig. 3C shows the binding activity of the antibody #27, Fig. 3D shows the binding activity of the antibody #105, and Fig. 3E shows the binding activity of the antibody #96-6. ■ represents the anti-Hp antibody, and □ represents the immune complex formed of the anti-Hp antibody and the human Hp.
Fig. 4 shows the inhibitory activity of the anti-Hp antibody (a fucosylated type and a defucosylated type), an immune complex formed of the defucosylated anti-Hp antibody and the human Hp, and IVIG to binding between an anti-DNP antibody and CD16a. The abscissa shows antibody concentration (μg/mL), and the ordinate shows fluorescence intensity. ● represents IVIG, ■ represents the antibody #27, □ represents a defucosylated antibody #27, ◊ represents an immune complex formed of the defucosylated antibody #27, Δ represents an immune complex formed of a defucosylated antibody #105, and *represents a sample free of an antibody.
Fig. 5 shows the inhibitory activity of the anti-Hp antibody (a fucosylated type and a defucosylated type), an immune complex formed of the defucosylated anti-Hp antibody and the human Hp, and IVIG to the ADCC reaction using PBMC. The abscissa shows antibody concentration (μg/mL), and the ordinate shows ADCC (%). ● represents IVIG, ■ represents the antibody #105, □ represents a defucosylated antibody #105, Δ represents the immune complex formed of the defucosylated antibody #27, ◊ represents the immune complex formed of the defucosylated antibody #105, *represents a sample free of an antibody, and - represents a sample containing only the human Hp.
Fig. 6A shows the inhibitory activity of the antibody #27, the antibody #105, the anti-DNP antibody, various modified forms of these antibodies, and IVIG to the ADCC reaction using the peripheral blood from a healthy human donor and rituximab. The ordinate shows the count proportional to the number of B cells. 1 represents a result obtained from a sample in which the ADCC reaction is not performed, 2 represents a result obtained from a sample in which the ADCC reaction is performed by using rituximab, and 3 to 14 represent results obtained from samples in which the ADCC reaction is performed using rituximab in the presence of various antibodies and the like. Specifically, regarding the various antibodies and the like, 3 represents IVIG (3,333 μg/mL), 4 represents the antibody #105 (250 μg/mL), 5 represents the antibody #27 (250 μg/mL), 6 represents the defucosylated antibody #105 (250 μg/mL), 7 represents the defucosylated antibody #27 (250 μg/mL), 8 represents a defucosylated anti-DNP antibody (250 μg/mL), 9 represents the defucosylated antibody #105 (50 μg/mL) in which Fc amino acids are modified (S239D and I332E), 10 represents the defucosylated antibody #27 (50 μg/mL) in which Fc amino acids are modified (S239D and I332E), 11 represents a defucosylated anti-DNP antibody (50 μg/mL) in which Fc amino acids are modified (S239D and I332E), 12 represents the defucosylated antibody #105 (10 μg/mL) in which Fc amino acids are modified (S239D and I332E), 13 represents the defucosylated antibody #27 (10 μg/mL) in which Fc amino acids are modified (S239D and I332E), and 14 represents the defucosylated anti-DNP antibody (10 μg/mL) in which Fc amino acids are modified (S239D and I332E). Fig. 6B shows the inhibitory activity of various modification forms of the antibody #4 and the antibody #27 and IVIG to the ADCC reaction using the peripheral blood from a healthy human donor and rituximab. The ordinate shows the proportion of B cells in lymphocytes. 1 represents a result obtained from a sample in which the ADCC reaction is not performed, 2 represents a result obtained from a sample in which the ADCC reaction is performed using rituximab, and 3 to 8 represent results obtained from samples in which the ADCC reaction is performed using rituximab in the presence of various antibodies and the like. Specifically, regarding the various antibodies and the like, 3 represents IVIG (2,000 μg/mL), 4 represents IVIG (8,000 μg/mL), 5 represents the defucosylated antibody #4 (50 μg/mL) in which Fc amino acids are modified (S239D, S298A, and I332E), 6 represents the defucosylated antibody #27 (50 μg/mL) in which Fc amino acids are modified (S239D, S298A, and I332E), 7 represents the defucosylated antibody #4 (10 μg/mL) in which Fc amino acids are modified (S239D, S298A, and I332E), and 8 represents the defucosylated antibody #27 (10 μg/mL) in which Fc amino acids are modified (S239D, S298A, and I332E).

Fig. 7 shows the action in a mouse immune thrombocytopenia (ITP) model. The ordinate shows the number of platelets ($\times 10^3/\mu L$). 1 shows a result obtained from a group to which an anti-platelet antibody is not administered, 2 shows a result obtained from a control group, 3 and 4 show results obtained from groups to which IVIG is administered at a dose of 10 and 40 mg/head, respectively, and 5 to 8 show results obtained from the groups to which the defucosylated #105 and #105 in which Fc amino acids are modified (G236A, S239D, and I332E) are administered at a dose of 0.08, 0.4, 0, 2, and 10 mg/head, respectively. Embodiments for Carrying Out the Invention

The human haptoglobin is a glycoprotein in which two or more subunits (hereinafter, described as α/β-subunits) formed of one α chain and one β chain are bonded to each other. The human haptoglobin synthesis process is divided into three stages.

At the first stage, an α chain and a β chain encoded to one open reading frame are linked to each other, and as a result, a single-chain polypeptide is expressed.

At the second stage, a disulfide bond is formed between α chains of the polypeptide, and as a result, a haptoglobin precursor constituted with two or more polypeptide chains is formed. In addition, a disulfide bond is also formed between the α chain and the β chain in the polypeptide molecule.

At the third stage, due to specific proteolysis, the polypeptide is split into the α chain and the β chain and becomes an α/β-subunit in which a disulfide bond is formed between the α chain and the β chain. Through the process described above, the human haptoglobin forms a structure in which two or more α/β-subunits described above are bonded to each other [Redox Rep., 6, 379 (2001)].

The basic structure of the human haptoglobin in the present invention is constituted with four α/β-subunits bonded to each other. The gene of the human haptoglobin linking and encoding the α chain and the β chain includes two kinds of gene polymorphisms which are each represented by GenBank accession Nos. NM_005143. 3 (SEQ ID NO: 67) and NM 001126102. 1 (SEQ ID NO: 68). These genes are different from each other in terms of the portion encoding the α chain, but the portion encoding the β chain is the same for the genes.

Specifically, in SEQ ID NO: 67, the regions of exon 3 and exon 4 of the α chain overlap with each other. Because a cysteine residue is encoded to the overlapping regions, the haptoglobin comprising the α chain generated by the translation of SEQ ID NO: 67 can form a bigger polymer in which three or more α/β-subunits are bonded to each other through a disulfide bond [J. Biochem. Mol. Biol., 40, 1028 (2007)].

The amino acid sequence of the α chain is represented by an amino acid sequence (SEQ ID NO: 69) consisting of the $19^{th}$ to $160^{th}$ amino acids in the GenBank accession No. NP_005134. 1 (SEQ ID NO: 65) or an amino acid sequence (SEQ ID NO: 71) consisting of the $19^{th}$ to $101^{st}$ amino acids in the GenBank accession No. NP_001119574. 1 (SEQ ID NO: 66). The amino acid sequence of the β chain is represented by an amino acid sequence (SEQ ID NO: 70) consisting of the $162^{nd}$ to $406^{th}$ amino acids in the GenBank accession No NP_005134. 1 (SEQ ID NO: 65) or an amino acid sequence (SEQ ID NO: 70) consisting of the $103^{rd}$ to $347^{th}$ amino acids in the GenBank accession No NP_001119574. 1 (SEQ ID NO: 66).

Examples of the human haptoglobin in the present invention comprise a polypeptide which comprises an amino acid sequence represented by SEQ ID NO: 69 or SEQ ID NO: 71 as an α chain and an amino acid sequence represented by SEQ ID NO: 70 as a β chain and has functions as human haptoglobin; a polypeptide which comprises amino acid sequences which is obtained by the deletion, substitution, insertion, or addition of one or more amino acids in the either or both of the α chain and the β chain in the amino acid sequence represented by SEQ ID NO: 69 or SEQ ID NO: 71 as an α chain and the amino acid sequence represented by SEQ ID NO: 70 as a β chain and functions as human haptoglobin; a polypeptide which comprises amino acid sequences in which either or both of the α chain and the β chain share identity equal to or higher than 60%, preferably equal to or higher than 80%, even more preferably equal to or higher than 90%, and most preferably equal to or higher than 95% with the amino acid sequence represented by SEQ ID NO: 69 or SEQ ID NO: 71 as an $\alpha$ chain and the amino acid sequence represented by SEQ ID NO: 70 as a $\beta$ chain and has functions as human haptoglobin; a polypeptide which comprises amino acid sequences in which either or both of the $\alpha$ chain and the $\beta$ chain comprise a partial sequence of the amino acid sequence represented by SEQ ID NO: 69 or SEQ ID NO: 71 as an $\alpha$ chain and the amino acid sequence represented by SEQ ID NO: 70 as a $\beta$ chain and has functions as human haptoglobin; and the like. Furthermore, examples of the human haptoglobin in the present invention comprise human haptoglobin marketed by Japan Blood Products Organization, and the like.

For example, the human haptoglobin has a function of forming an immune complex by specifically binding to hemoglobin which is freed in the blood due to hemolysis and preventing the free hemoglobin from being released into the urine, such that the leakage of iron from the body or the oxidative vascular disorders and renal tubular disorders resulting from the free hemoglobin are prevented.

The polypeptide, which have amino acid sequences obtained by the deletion, substitution, insertion, or addition of one or more amino acids in the amino acid sequence represented by SEQ ID NO: 69 or SEQ ID NO: 71 as the $\alpha$ chain and the amino acid sequence represented by SEQ ID NO: 70 as the $\beta$ chain, can be obtained by a method in which site-specific mutation is introduced into DNA encoding, for example, the polypeptide comprising the amino acid sequence represented by SEQ ID NO: 69, 70, or 71, that is, a gene encoding the human haptoglobin by using a site-specific mutagenesis method [Molecular Cloning, A Laboratory Manual, Second Edition, Cold Spring Harbor Laboratory Press (1989), Current Protocols in Molecular Biology, John Wiley & Sons (1987-1997), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proc. Natl. Acad. Sci. USA, 82, 488 (1985)] and the like. The number of amino acids to be deleted, substituted, or added is not particularly limited, but is preferably one to tens of amino acids such as 1 to 20, and more preferably 1 to several amino acids such as 1 to 5.

Examples of the gene encoding the human haptoglobin include a base sequence represented by SEQ ID NO: 67 or SEQ ID NO: 68. A gene which has a base sequence obtained by the deletion, substitution, or addition of one or more bases in the base sequence represented by SEQ ID NO: 67 or SEQ ID NO: 68 and comprises DNA encoding a protein having the function of the human haptoglobin, a gene which has a base sequence sharing identity of at least equal to or higher than 60%, preferably sharing identity equal to or higher than 80%, and even more preferably sharing identity equal to or higher than 95% with the base sequence represented by SEQ ID NO: 67 or SEQ ID NO: 68 and comprises DNA encoding a polypeptide having the function of the human haptoglobin, a gene which comprises DNA hybridized with DNA having the base sequence represented by SEQ ID NO: 67 or SEQ ID NO: 68 under stringent conditions and comprises DNA encoding a polypeptide having the function of the human haptoglobin, and the like are also included in the gene encoding the haptoglobin of the present invention.

The DNA hybridized under stringent conditions means hybridizable DNA obtained by a colony hybridization method, a plaque hybridization method, a Southern blotting hybridization method, a DNA microarray method, or the like in which DNA having the base sequence represented by SEQ ID NO: 67 or SEQ ID NO: 68 is used as a probe.

Specifically, DNA derived from a hybridized colony or plaque, or a PCR product or oligo DNA having the sequence thereof is fixed to a filter or slide glass, and by using the filter or the slide glass, hybridization is performed at 65°C in the presence of sodium chloride at 0.7 to 1.0 mol/L [Molecular Cloning, A Laboratory Manual, Second Edition, Cold Spring Harbor Laboratory Press (1989), Current Protocols in Molecular Biology, John Wiley & Sons (1987-1997), DNA Cloning 1: Core Techniques, A Practical Approach, Second Edition, Oxford University, (1995)]. Then, the filter or the slide glass is washed with an SSC solution with 0.1 to 2 times concentration (the SSC solution with 1 time concentration is composed of 150 mmol/L sodium chloride and 15 mmol/L sodium citrate) under the condition of 65°C. DNA which can be identified in this way can be exemplified as the hybridizable DNA.

Examples of the hybridizable DNA include DNA which shares identity of at least equal to or higher than 60% with the base sequence represented by SEQ ID NO: 67 or SEQ ID NO: 68, preferably include DNA which shares identity equal to or higher than 80% with the same base sequence, and more preferably include DNA which shares identity equal to or higher than 95% with the same base sequence.

In the base sequence of a gene encoding proteins of eukaryotes, gene polymorphisms are frequently found. The gene used in the present invention having a base sequence in which small-scale mutation has occurred due to the polymorphisms is also included in the gene encoding the haptoglobin used in the present invention.

Unless otherwise specified, the numerical value of identity in the present invention may be a numerical value calculated using identity search programs known to those skilled in the art. Regarding base sequences, examples thereof include numerical values calculated using default parameters in BLAST [J. Mol. Biol., 215, 403 (1990)] and the like. Regarding amino acid sequences, examples thereof include numerical values calculated using default parameters in BLAST2 [Nucleic Acids Res., 25, 3389 (1997), Genome Res., 7, 649 (1997), http://www.ncbi.nlm.nih.gov/Education/BLASTinfo/information3.html] and the like.

Regarding the default parameters, in a case where G (Cost to open gap) is a base sequence, the default parameter is 5, and in a case where G is an amino acid sequence, the default parameter is 11. In a case where -E (Cost to extend gap) is a base sequence, the default parameter is 2, and in a case where -E is an amino acid sequence, the default parameter is 1, -q (Penalty for nucleotide mismatch) is -3, -r (reward for nucleotide match) is 1, and -e (expect value) is 10. In a case where -W (wordsize) is a base sequence, the default parameter is 11 residues, and in a case where -W is an amino acid sequence, the default parameter is 3 residues. In a case where -y [Dropoff (X) for blast extensions in bits] is blastn, the default parameter is 20. In a program other than blastn, the default parameter is 7, -X (X dropoff value for gapped alignment in bits) is 15. In a case where Z (final X dropoff value for gapped alignment in bits) is blastn, the default parameter is 50. In a program other than blastn, the default parameter is 25 (http://www.ncbi.nlm.nih.gov/blast/html/blastcgihelp.html).

The polypeptide consisting of the partial sequences of the amino acid sequence represented by SEQ ID NO: 69 or SEQ ID NO: 71 as the α chain and the amino acid sequence represented by SEQ ID NO: 70 as the β chain can be prepared, for example, by a method of deleting a portion of DNA encoding the amino acid sequence represented by SEQ ID NO: 69, 70, or 71 and culturing a transformant into which a recombinant vector comprising the DNA is introduced.

Furthermore, the polypeptide comprising amino acid sequences obtained by the deletion, substitution, or addition of one or more amino acids in the partial sequence of the amino acid sequence represented by SEQ ID NO: 69 or SEQ ID NO: 71 as the α chain and the amino acid sequence represented by SEQ ID NO: 70 as the β chain can also be obtained by the site-specific mutagenesis method described above.

In addition, the polypeptide consisting of partial sequences of the amino acid sequence represented by SEQ ID NO: 69 or SEQ ID NO: 71 as the α chain and the amino acid sequence represented by SEQ ID NO: 70 as the β chain, or the polypeptide having amino acid sequences obtained by the deletion, substitution, or addition of one or more amino acids in the partial sequence of the amino acid sequence represented by SEQ ID NO: 69 or SEQ ID NO: 70 as the α chain and the amino acid sequence represented by SEQ ID NO: 71 as the β chain can also be manufactured by a chemical synthesis method such as a fluorenylmethyloxycarbonyl (Fmoc) method or a t-butyloxycarbonyl (tBoc) method.

The monoclonal antibody of the present invention (hereinafter, also called the antibody of the present invention as well) is an antibody which specifically recognizes and binds to the amino acid sequence of the β chain in the human haptoglobin or to the 3-dimensional structure of the human haptoglobin, and forms a polyvalent immune complex in the blood.

The human haptoglobin in the present invention may have any 3-dimensional structure as long as the structure has the same effect as that of the structure that the human haptoglobin, which comprises the amino acid sequence represented by SEQ ID NO: 69 or SEQ ID NO: 71 as the α chain and the amino acid sequence represented by SEQ ID NO: 70 as the β chain, can have in the natural state. The 3-dimensional structure that the human haptoglobin can have in the natural state refers to the 3-dimensional structure of the natural human haptoglobin.

Examples of the polyvalent immune complex in the present invention specifically include an immune complex comprising at least two antibody molecules of the present invention and at least one human haptoglobin molecule. In a case where the immune complex comprises at least two antibody molecules and one human haptoglobin molecule, the immune complex exhibits high binding activity to CD16 and CD32 and exhibits excellent inhibitory activity to the ADCC reaction.

One human haptoglobin molecule described above comprises four α/β-subunits comprising one α chain having the amino acid sequence represented by SEQ ID NO: 69 or SEQ ID NO: 71 and one β chain having the amino acid sequence represented by SEQ ID NO: 70.

The molecular weight of the polyvalent immune complex formed of the monoclonal antibody of the present invention and the human haptoglobin bonded to each other is preferably equal to or greater than $5 \times 10^5$ and more preferably equal to or greater than $7 \times 10^5$. In a case where the molecular weight of the immune complex is equal to or greater than $5 \times 10^5$, the immune complex exhibits high binding activity to CD16 and CD32 and exhibits excellent inhibitory activity to the ADCC reaction.

Whether the antibody of the present invention forms the polyvalent immune complex can be checked, for example, by gel filtration chromatography using HPLC and the like.

Whether the immune complex formed of the antibody of the present invention comprises at least two antibody molecules of the present invention and at least one human haptoglobin molecule can be checked from the molecular weight of the immune complex. The molecular weight of the immune complex can be measured, for example, by separating and detecting the immune complex by gel filtration chromatography using HPLC and the like. The molecular weight of the antibody of the present invention is about 150 kDa, and the molecular weight of the human haptoglobin is about 200 to 220 kDa. Based on this information, the number of molecules of the antibody and the antigen comprised in the immune complex can be calculated.

Specifically, examples of the antibody of the present invention include an antibody binding to at least phenylalanine (Phe) at position 44 and glutamic acid (Glu) at position 49 in the amino acid sequence of the β chain of the human haptoglobin represented by SEQ ID NO: 70. Furthermore, specifically, examples of the antibody of the present invention also include an antibody binding to at least serine (Ser) at position 155, threonine (Thr) at position 156, and valine (Val) at position 157 in the amino acid sequence of the β chain of the human haptoglobin represented by SEQ ID NO: 70.

Specifically, examples of the antibody include the following monoclonal antibody (a) or (b).

(a) Antibody comprising an H chain of an antibody in which CDRs 1 to 3 have amino acid sequences represented by SEQ ID NOs: 31, 32, and 33, respectively, and an L chain of an antibody in which CDRs 1 to 3 have amino acid sequences represented by SEQ ID NOs: 34, 35, and 36, respectively

(b) Antibody comprising an H chain of an antibody in which CDRs 1 to 3 have amino acid sequences represented by SEQ ID NOs: 37, 38, and 39, respectively, and an L chain of an antibody in which CDRs 1 to 3 have amino acid sequences represented by SEQ ID NOs: 40, 41, and 42, respectively

More specifically, examples of the monoclonal antibody of the present invention include the following monoclonal antibody (c) or (d).

(c) Antibody comprising an H chain of an antibody comprising an amino acid sequence represented by SEQ ID NO: 25 and an L chain of an antibody comprising an amino acid sequence represented by SEQ ID NO: 26

(d) Antibody comprising an H chain of an antibody comprising an amino acid sequence represented by SEQ ID NO: 27 and an L chain of an antibody comprising an amino acid sequence represented by SEQ ID NO: 28

Examples of the monoclonal antibody of the present invention also include a monoclonal antibody which competes with the aforementioned monoclonal antibody, specifically recognizes the amino acid sequence of the human haptoglobin or the 3-dimensional structure thereof, and binds to the same epitope as the epitope comprised in the human haptoglobin to which the aforementioned monoclonal antibody binds.

In the present invention, the antibody competing with the monoclonal antibody refers to an antibody recognizing an epitope (also called antigenic determinant), which is totally or partially the same as the epitope the monoclonal antibody of the present invention recognizes, and binding to the epitope. The antibody binding to the same epitope as the epitope to which the monoclonal antibody of the present invention binds refers to an antibody recognizing and binding to the same sequence as the amino acid sequence of the human haptoglobin that the monoclonal antibody of the present invention recognizes.

Whether the monoclonal antibody of the present invention binds to the amino acid sequence of the human haptoglobin or the 3-dimensional structure thereof can be checked by methods for investigating the binding activity between a certain antigen and an antibody against the certain antigen, such as radioimmunoassay, enzyme-linked immunosorbent assay (ELISA), a Western blotting method, an immunohistochemical staining (IHC) method, and known immunological detection method.

Specifically, examples of the methods include a fluorescent antibody staining method [Cancer Immunol. Immunother., 36, 373 (1993)] using an FMAT8100HTS system (Applied Biosystems), a fluorescent staining method using flow cytometry, surface plasmon resonance using a Biacore system (GE Healthcare), isothermal titration calorimetry using ITC (DKSH Management Ltd.), and the like.

In a case where whether the monoclonal antibody of the present invention binds to the amino acid sequence of the human haptoglobin or to the 3-dimensional structure thereof is checked by surface plasmon resonance using a Biacore system (GE Healthcare) and the like, it is preferable to fix anti-IgG antibodies to a sensor chip CM5 by an amine coupling method, then allow the monoclonal antibodies to flow such that a sufficient amount of the monoclonal antibodies bind to the anti-IgG antibodies, allow the human haptoglobin of a plurality of known concentrations or a fusion thereof to flow, and measure the binding and dissociation.

Furthermore, whether the monoclonal antibody of the present invention binds to the amino acid sequence of the human haptoglobin or the 3-dimensional structure thereof can also be checked by combining other known immunological detection methods [Monoclonal Antibodies-Principles and Practice, Third edition, Academic Press (1996), Antibodies-A Laboratory Manual, Cold Spring Harbor Laboratory (1988), Monoclonal Antibody Experiment Manual, Kodansha Scientific Ltd. (1987)] and the like.

Examples of the monoclonal antibody of the present invention include an antibody produced from a hybridoma, or a recombinant antibody produced from a transformant obtained by transformation by using a recombinant vector comprising an antibody gene.

The monoclonal antibody is an antibody secreted from a monoclonal antibody-producing cell. The monoclonal antibody is characterized in that it recognizes only one epitope (also called antigen determinant) and the amino acid sequence (primary structure) constituting the monoclonal antibody is uniform.

Examples of the epitope include a single amino acid sequence which is recognized by the monoclonal antibody and to which the monoclonal antibody binds, a 3-dimensional structure formed of the amino acid sequence, an amino acid sequence modified by the post-translation modification, a 3-dimensional structure formed of the amino acid sequence, and the like.

Examples of the amino acid sequence modified by the post-translation modification include an amino acid sequence to which an O-linked sugar chain is added (the sugar chain is bonded to threonine and serine having a OH substituent), an amino acid sequence to which a N-linked sugar chain is added (bonded to glutamine and asparagine having a $NH_2$ substituent), an amino acid sequence to which a sulfuric acid group is added (the sulfuric acid molecule is bonded to threonine having a OH substituent), and the like.

The epitope of the human haptoglobin to which the monoclonal antibody of the present invention binds can be determined by investigating the binding activity of the monoclonal antibody of the present invention to a deletion variant obtained by deleting a portion of the domain of the human haptoglobin, a mutant obtained by substituting some of the amino acids constituting the human haptoglobin with other amino acids, a human haptoglobin mutant obtained by the substitution with a domain of the human haptoglobin with the domain derived from another protein, a partial peptide fragment of the human haptoglobin, and the like.

Furthermore, the epitope of the human haptoglobin to which the monoclonal antibody of the present invention binds can also be determined by adding the antibody of the present invention to the human haptoglobin digested by a protease and performing epitope mapping by using known mass spectrometry.

The epitope of the human haptoglobin to which the monoclonal antibody of the present invention binds is comprised in an amino acid sequence from positions 25 to 239 in the amino acid sequence represented by SEQ ID NO: 70 as the β chain of the haptoglobin.

The epitope of the haptoglobin to which the monoclonal antibody of the present invention binds comprises at least Phe at position 44 and Glu at position 49 in the amino acid sequence of the β chain of the human haptoglobin represented by SEQ ID NO: 70. Furthermore, the epitope of the haptoglobin to which the monoclonal antibody of the present invention binds comprises at least Ser at position 155, Thr at position 156, and Val at position 157 in the amino acid sequence of the β chain of the human haptoglobin represented by SEQ ID NO: 70.

The hybridoma can be prepared, for example, by preparing the human haptoglobin as an antigen, inducing an antibody-producing cell having antigen specificity from an animal immunized with the antigen, and fusing the antibody-producing cell with a myeloma cell. An anti-human haptoglobin monoclonal antibody can be obtained by culturing the hybridoma or by administering the hybridoma cell to an animal so as to induce ascites cancer in the animal and separating and purifying the culture solution or the ascitic fluid.

As the animal to be immunized with the antigen, any animal can be used as long as the hybridomas can be prepared. However, it is preferable to use a mouse, a rat, a hamster, a chicken, a rabbit, and the like. Furthermore, an antibody produced from a hybridoma, which is prepared by obtaining a cell capable of producing antibodies from the aforementioned animal, immunizing the cell in vitro, and then fusing the cell with a myeloma cell, and the like are also comprised in the antibody of the present invention.

In the present invention, the recombinant antibody comprises antibodies produced by gene recombination, such as a human chimeric antibody, a human CDR-grafted antibody, and a human antibody. As a treatment agent, the recombinant antibody having the characteristic of the monoclonal antibody, exhibiting low antigenicity, and having a prolonged half-life in blood is preferable. Examples of the recombinant antibody include an antibody obtained by modifying the monoclonal antibody of the present invention by using a gene recombination technique.

The human chimeric antibody refers to an antibody consisting of VH and VL of a non-human animal antibody and a heavy chain constant region (hereinafter, described as CH) and a light chain constant region (hereinafter, described as CL) of a human antibody. The human chimeric antibody of the present invention can be produced by obtaining cDNA encoding VH and VL from a hybridoma producing a monoclonal antibody, which specifically recognizes and binds to the amino acid sequence of the human haptoglobin or the 3-dimensional structure thereof, inserting the cDNA into a recombinant vector for animal cell having genes encoding CH and CL of the human antibody so as to establish a human chimeric antibody recombinant vector, and introducing the vector into an animal cell.

CH of the human chimeric antibody is not limited as long as it belongs to the human immunoglobulin (hereinafter, described as hIg). CH belonging to the hIgG class is preferably used, and CH of any of the subclasses such as hIgG1, hIgG2, hIgG3, and hIgG4 belonging to the IgG class can also be used. Furthermore, CL of the human chimeric antibody is not limited as long as it belongs to hIg, and CL belonging to the κ class or the λ class can be used.

Examples of the chimeric antibody of the present invention include a chimeric antibody which competes with the monoclonal antibody of the present invention, specifically recognizes the amino acid sequence of the human haptoglobin or the 3-dimensional structure thereof, and binds to the same epitope as the epitope present in the human haptoglobin to which the monoclonal antibody binds.

The human CDR-grafted antibody is also called humanized antibody in some cases, and refers to an antibody obtained by grafting the amino acid sequence of CDR of VH and VL of a non-human animal antibody to an appropriate position of VH and VL of a human antibody. The human CDR-grafted antibody of the present invention can be manufactured by establishing cDNA encoding the V region obtained by grafting the amino acid sequence of CDR of VH and VL of a non-human animal monoclonal antibody, which specifically recognizes and binds to the amino acid sequence of the human haptoglobin or the 3-dimensional structure thereof, to a framework region (hereinafter, described as FR) of VH and VL of any human antibody, inserting the cDNA to a recombinant vector for animal cells having genes encoding CH and CL of a human antibody so as to establish a human CDR-grafted antibody recombinant vector, and introducing the vector into an animal cell such that the antibody is expressed.

CH of the human CDR-grafted antibody is not limited as long as it belongs to hIg. CH belonging to the hIgG class is preferably used, and CH of any of the subclasses such as hIgG1, hIgG2, hIgG3, and hIgG4 belonging to the hIgG class can be used. Furthermore, CL of the human CDR-grafted antibody is not limited as long as it belongs to hIg, and CL belonging to the κ class or the λ class can be used.

Specifically, examples of the human CDR-grafted antibody of the present invention include a humanized antibody comprising VH of an antibody in which CDR1 to 3 have amino acid sequences represented by SEQ ID NOs: 31 to 33, respectively, and VL of an antibody in which CDR1 to 3 have amino acid sequences represented by SEQ ID NOs: 34 to 36, respectively, and a humanized antibody comprising VH of an antibody in which CDR1 to 3 have amino acid sequences represented by SEQ ID NOs: 37 to 39, respectively, and VL of an antibody in which CDR1 to 3 have amino acid sequences represented by SEQ ID NOs: 40 to 42, respectively.

Examples of the humanized antibody of the present invention include a humanized antibody which competes with the monoclonal antibody of the present invention, specifically recognizes the amino acid sequence of the human haptoglobin or the 3-dimensional structure thereof, and binds to the same epitope as the epitope present in the human haptoglobin to which the monoclonal antibody binds.

Although the human antibody originally refers to a naturally occurring antibody present in the human body, antibodies obtained from a human antibody phage library and a human antibody-producing transgenic animal, which are prepared with the latest advances in the techniques of genetic engineering, cell engineering, and development engineering, and the like are also included in the human antibody.

For example, by isolating lymphocytes from the human peripheral blood, immortalizing the cells by the infection with EB viruses and the like, and performing cloning, lymphocytes producing the naturally occurring antibody present in the human body can be cultured. From the culture supernatant, the antibody can be purified.

The human antibody phage library is a library obtained by inserting an antibody gene prepared from the human B cell into a phage gene such that antibody fragments such as Fab and scFv are expressed on the surface of the phage. From the library, a phage expressing an antibody fragment having desired antigen binding activity on the surface thereof can be collected using the binding activity to a substrate, to which an antigen is fixed, as an index. By a genetic engineering technique, the antibody fragment can also be converted into a human antibody molecule consisting of two complete H chains and two complete L chains.

The human antibody-producing transgenic animal means an animal having cells into which human antibody genes are incorporated. Specifically, for example, by introducing human antibody genes into mouse ES cells, grafting the ES cells to an early mouse embryo, and then causing development, the human antibody-producing transgenic animal can be prepared. The human antibody from the human antibody-producing transgenic animal can be prepared by obtaining a human antibody-producing hybridoma by using a hybridoma preparation method that is generally performed in non-human animals and culturing the hybridoma such that human antibodies are produced and accumulated in the culture supernatant.

Specific examples of the human antibody of the present invention include a human antibody in which VH of the antibody has an amino acid sequence represented by SEQ ID NO: 25 and VL of the antibody has an amino acid sequence represented by SEQ ID NO: 26, and a human antibody in which VH of the antibody has an amino acid sequence represented by SEQ ID NO: 27 and VL of the antibody has an amino acid sequence represented by SEQ ID NO: 28.

The polyvalent immune complex formed of the antibody of the present invention binds to the human Fcγ receptor through the Fc domain of the antibody of the present invention. Accordingly, the antibody of the present invention can inhibit the reaction that is induced in a case where an autoantibody binds to the Fcγ receptor.

All of human Fcγ receptors specifically binding to the Fc domain of the human IgG antibody are comprised in the human Fcγ receptor to which the polyvalent immune complex formed of the antibody of the present invention binds. Examples of the human Fcγ receptor include a human Fcγ receptor III (CD16), a human Fcγ receptor II (CD32), and a human Fcγ receptor I (CD64). These human Fcγ receptors are expressed on the surface of a cell such as a leukocyte or a macrophage.

The human Fcγ receptor III [J. Exp. Med., 170, 481 (1989)] means a type III Fcγ receptor among three kinds of Fcγ receptors which have activity of binding to the Fc domain of the human IgG antibody comprising the γ heavy chain and are expressed on the surface of a human cell. The human Fcγ receptor III has two isoforms encoded to different genes, and is classified into a human Fcγ receptor IIIa (comprising CD16a and allotypes V158 and F158) and a human Fcγ receptor IIIb (comprising CD16b, an allotype FcγRIIIb-NA1, and an allotype FcγRIIIb-NA2). In the present invention, all these isoforms and allotypes are comprised in the human Fcγ receptor III.

The human Fcγ receptor II [J. Exp. Med., 170, 1369 (1989)] means a type II Fcγ receptor among three kinds of Fcγ receptors which have activity of binding to the Fc domain of the human IgG antibody comprising the γ heavy chain and are expressed on the surface of a human cell. The human Fcγ receptor II has three isoforms encoded to different genes, and is classified into a human Fcγ receptor IIa (comprising allotypes H131 [type H] and R131 [type R]), a human Fcγ receptor IIb (comprising FcγRIIb-1 and FcγRIIb-2), and a human Fcγ receptor IIc. In the present invention, all of these isoforms and allotypes are included in the human Fcγ receptor III.

The human Fcγ receptor I [J. Biol Chem., 266, 13449 (1991)] means a type I Fcγ receptor among three kinds of Fcγ receptors which have activity of binding to the Fc domain of the human IgG antibody having the γ heavy chain and are expressed on the surface of a human cell. The human Fcγ receptor I has three isoforms encoded to different genes, and is classified into a human Fcγ receptor Ia, a human Fcγ receptor Ib, and a human Fcγ receptor Ic. In the present invention, all of these isoforms are included in the human Fcγ receptor I.

The human Fcγ receptor I is known to bind to the human IgG antibody, which is a monomer, with high affinity, and the human Fcγ receptors II and III are known to bind to an immune complex comprising the human IgG antibody with affinity higher than the affinity the receptors exhibit at the time of binding to a monomer [Trends in Immunology, 29, 608 (2008)]. Accordingly, the antibody of the present invention binds to the human Fcγ receptor I regardless whether or not an immune complex is formed.

Examples of the reaction, which can be inhibited by the antibody of the present invention and is included in a case where an autoantibody binds to the Fcγ receptor, include a reaction in which an autoantibody belonging to the IgG class binds to the Fcγ receptor on the surface of a cell such as a leukocyte or a macrophage and induces the damage or phagocytosis of a self-cell. Examples of the damage or phagocytosis of the self-cell include the antibody-dependent cellular cytotoxicity activity (ADCC activity) or the antibody-dependent cellular phagocytosis activity (ADCP activity).

In a case where the autoantibody molecule having bound to the self-tissue binds, for example, to a natural killer cell (hereinafter, described as NK cell) expressing the Fcγ receptor through the Fc portion, a cytotoxic molecule such as autoantibody perforin or granzyme is released, or the phagocytosis is stimulated. The ADCC activity is a cytotoxic reaction that occurs as a result of the mechanism described above [Clark M, Chemical Immunology, 65, 88 (1997); Gorter et al., Immunol Today, 20, 576 (1999)].

In addition, in a case where the autoantibody molecule having bound to the self-tissue is captured, for example, by a macrophage expressing the Fcγ receptor through the Fc portion, the cell undergoes phagocytosis and is destroyed. The ADCP activity is self-tissue damage that occurs as a result of the mechanism described above.

The antibody of the present invention includes an antibody obtained by modifying amino acids of the Fc domain of the antibody so as to improve the binding activity to the Fcγ receptor. Specifically, for example, in the case of the antibody obtained by combining the antibody of the present invention with the potelligent technique and/or a technique of modifying the Fc amino acids of the antibody, the Fc domain of the antibody forming an immune complex together with Hp more strongly binds to CD16a and more potently inhibits the ADCC activity compared to IVIG.

Examples of the antibody, which is obtained by modifying amino acids of the Fc domain of the antibody so as to improve the binding activity to the Fcγ receptor, include an antibody in which fucose is not bound to N-acetylglucosamine of the reducing terminal of an N-glycoside-linked sugar chain linked to the Fc domain of the antibody.

Examples of the antibody in which fucose is not bound to N-acetylglucosamine of the reducing terminal of an N-glycoside-linked sugar chain linked to the Fc domain of the antibody include an antibody prepared using, for example, a CHO cell in which an α1,6-fucose transferase gene is deleted (International Publication No. 2005/035586 and International Publication No. 02/31140).

In the related art, the technique of modifying the structure of the N-glycoside-linked sugar chain linked to the Fc domain of the existing antibody into the structure in which fucose is not bound to N-acetylglucosamine of the reducing terminal of the N-glycoside-linked sugar chain has been used for improving the ADCC activity of the existing antibody. However, because the antibody of the present invention has high affinity with the Fcγ receptor, by causing the antibody to bind to the NK cell or the like expressing the Fcγ receptor, the aforementioned technique can be used as a method for inhibiting the ADCC activity stimulated under pathological conditions.

Examples of the antibody, which is obtained by modifying amino acids of the Fc domain of the antibody so as to improve the binding activity to the Fcγ receptor, include antibody molecules prepared by the method described in US Patent No. 7,317,091.

Furthermore, the half-life of the antibody of the present invention in blood can be increased by modifying the surface charge of a polypeptide comprising the variable region of the antibody or by modifying the antigen binding activity at the internal pH of an early endosome.

Examples of the method for increasing the half-life in blood by modifying the surface charge of a polypeptide comprising the variable region of the antibody molecule or by modifying the antigen binding activity at the internal pH of an early endosome include the methods described in JP-A-2013-165716 and Japanese Patent No. 4961501.

A monoclonal antibody, which is obtained by the deletion, substitution, insertion, or addition of one or more amino acids in the amino acid sequence constituting the aforementioned antibody and has the same activity as that of the aforementioned antibody, is also included in the antibody of the present invention.

The number of amino acids to be deleted, substituted, inserted, or added is not particularly limited as long as it is equal to or greater than 1. The number of amino acids described above is approximately the number of amino acids that can be deleted, substituted, or added by known techniques such as the site-specific mutagenesis method described in Molecular Cloning, Second Edition, Current Protocols in Molecular Biology, Nucleic Acids Research, 10, 6487 (1982), Proc. Natl. Acad. Sci., USA, 79, 6409 (1982), Gene, 34, 315 (1985), Nucleic Acids Research, 13, 4431 (1985), Proc. Natl. Acad. Sci USA, 82, 488 (1985), and the like. For example, the number of amino acids is 1 to tens of amino acids, preferably 1 to 20, more preferably 1 to 10, and even more preferably 1 to 5.

The deletion, substitution, insertion, or addition of one or more amino acid residues in the amino acid sequence of the antibody of the present invention means the following. That is, it means that in the amino acid sequence of the antibody, the deletion, addition, substitution, or insertion of one or plural amino acid residues occurs. Furthermore, the deletion, addition, substitution, or insertion simultaneously occur in some cases. In addition, the amino acid residues deleted, added, substituted, or inserted include both the natural amino acid residues and the non-natural amino acid residues in some cases.

Examples of the natural amino acid residues include L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine, L-cysteine, and the like.

Preferred examples of amino acid residues that can be substituted with each other will be shown below. The amino acid residues that belong to the same group can be substituted with each other.

A group: leucine, isoleucine, norleucine, valine, norvaline, alanine, 2-aminobutanoic acid, methionine, O-methylserine, t-butylglycine, t-butylalanine, and cyclohexylalanine
B group: aspartic acid, glutamic acid, isoaspartic acid, isoglutamic acid, 2-aminoadipic acid, and 2-aminosuberic acid
C group: asparagine and glutamine
D group: lysine, arginine, ornithine, 2,4-diaminobutanoic acid, and 2,3-diaminopropionic acid
E group: proline, 3-hydroxyproline, and 4-hydroxyproline
F group: serine, threonine, and homoserine
G group: phenylalanine, and tyrosine

The transformant of the present invention includes any transformant as long as the transformant is obtained by introducing a recombinant vector comprising DNA encoding an antibody molecule, which specifically recognizes the β chain of the human haptoglobin represented by SEQ ID NO: 70, binds to the human haptoglobin, and forms a polyvalent immune complex, into a host cell and produces the antibody of the present invention. Specific examples of the host cell include the following (1) to (5).

(1) CHO cell derived from the Chinese hamster's ovarian tissue;
(2) Rat myeloma cell line YB2/3HL. P2. G11. 16Ag. 20;
(3) Mouse myeloma cell line NS0 cell;
(4) Mouse myeloma cell line SP2/0-Ag14 cell;
(5) BHK cell derived from Syrian hamster's kidney tissue

As the transformant of the present invention producing the antibody in which fucose is not bound to N-acetylglucosamine of the reducing terminal of a N-glycoside-linked sugar chain binding to the Fc domain of the antibody, it is possible to use a host cell, in which the reduction or deletion of glycosyltransferase is induced, prepared by the methods described in International Publication No. 2005/035586 and International Publication No. 02/31140.

The present invention relates to a therapeutic agent for treatment of autoimmune diseases, comprising the monoclonal antibody of the present invention and a pharmacologically accepted carrier.

Furthermore, the present invention relates to a therapeutic agent for treatment of autoimmune diseases, comprising a monoclonal antibody binding to human haptoglobin. As the human haptoglobin, human haptoglobin is preferable which has an amino acid sequence represented by SEQ ID NO: 69 or SEQ ID NO: 71 as an α chain and an amino acid sequence represented by SEQ ID NO: 70 as a β chain.

The autoimmune diseases are not limited, and examples thereof include autoimmune diseases relating to tissue damage caused by autoantibodies. Examples of the autoimmune diseases include autoimmune diseases for which an IVIG preparation is used as a treatment agent, such as immune thrombocytopenic purpura (also called idiopathic thrombocytopenic purpura or thrombocytopenia), Kawasaki disease, ANCA-associated vasculitis, Guillain-Barre syndrome, chronic demyelinating polyradiculoneuropathy, myasthenia gravis, multifocal motor neuropathy, rheumatoid arthritis, systemic lupus erythematosus, autoimmune hemolytic anemia, scleroderma, autoimmune urticaria, pemphigus, Goodpasture's syndrome, psoriatic arthritis, Sjogren's syndrome, and polymyositis/dermatomyositis, and autoimmune diseases in which the production of autoantibodies is involved with the pathological condition, such as IgG4-related diseases.

Furthermore, the therapeutic agent for treatment of autoimmune diseases of the present invention can be used for treating and/or preventing autoimmune diseases relating to tissue damage caused by autoantibodies.

The treatment agent of the present invention may contain only the antibody or derivatives thereof as an active component. However, generally, the treatment agent is mixed with one or more pharmacologically accepted carriers and provided as a pharmaceutical preparation produced by methods known in the technical field of pharmaceutics.

It is preferable to administer the treatment agent through a route that is the most effective for treatment. Examples of the route of administration include oral administration or parenteral administration such as intraoral administration, intra-airway administration, intrarectal administration, subcutaneous administration, intradermal administration, intravenous administration, or intraperitoneal administration. The treatment agent can be administered, for example, in the form of an aerosol, a capsule, a tablet, powder, granules, a syrup, an emulsion, a suppository, an injection, an ointment, or a tape.

The dose and the frequency of administration vary with the desired therapeutic effect, the treatment method, the treatment period, the age, the body weight, and the like. Generally, the daily dose for adult is 10 μg/kg to 100 mg/kg.

The treatment agent of the present invention may be used alone or used in combination with at least another treatment agent. As the method for using the treatment agent of the present invention in combination with another treatment agent, the treatment agent of the present invention and the treatment agent used in combination with the treatment agent of the present invention may be simultaneously administered or consecutively administered. Examples of another treatment agent described above include steroid, aspirin, cyclophosphamide, cyclosporine, and the like.

Hereinafter, a method for producing the antibody of the present invention and methods for treating diseases will be specifically described.

1. Method for producing antibody

(1) Preparation of antigen

Human haptoglobin or a cell expressing human haptoglobin that will become an antigen can be obtained by introducing a recombinant vector comprising cDNA, which encodes full length or partial length of the α chain and the β chain forming the human haptoglobin, into E. coli, yeast, an insect cell, or an animal cell. Furthermore, the antigen can also be obtained by purifying human haptoglobin from various human culture cells, human tissues, and the like expressing a large amount of human haptoglobin.

In addition, the culture cells, the tissues, and the like can be used as they are as the antigen. Furthermore, as the antigen, it is possible to use a synthetic peptide which is prepared by a chemical synthesis method such as the Fmoc method or the tBoc method and comprises a partial sequence of the human haptoglobin.

A known tag such as FLAG or His may be added to the C-terminal or the N-terminal of the synthetic peptide comprising the human haptoglobin or a partial sequence of the human haptoglobin.

The human haptoglobin used in the present invention can be produced by causing a host cell to express DNA encoding the human haptoglobin by using the method described in Molecular Cloning, A Laboratory Manual, Second Edition, Cold Spring Harbor Laboratory Press (1989) or Current Protocols in Molecular Biology, John Wiley & Sons (1987-1997), or the like, for example, by the following method.

First, full-length cDNA comprising a portion encoding the α chain and the β chain forming the human haptoglobin is inserted into the downstream of a promoter of an appropriate recombinant vector, thereby preparing a recombinant vector. Instead of the full-length cDNA, a DNA fragment having an appropriate length that is prepared based on full-length cDNA and comprises a portion encoding a polypeptide may be used. Then, by introducing the obtained recombinant vector into a host cell appropriate for the recombinant vector, a transformant producing a polypeptide can be obtained.

As the recombinant vector, any recombinant vector can be used as long as it can perform autonomous replication in the host cell to be used or can be incorporated into the chromosome and comprises an appropriate promoter at a position where DNA encoding a polypeptide can be transcribed.

As the host cell, any of microorganisms of genus Escherichia such as E. coli, yeast, an insect cell, an animal cell, and the like can be used as long as they can express a target gene.

In a case where prokaryote such as E. coli is used as the host cell, the recombinant vector is preferably a vector which can perform autonomous replication in the prokaryote and comprises a promoter, a ribosome binding sequence, DNA comprising a portion encoding the human haptoglobin, and a transcription termination sequence. Although the recombinant vector does not have to comprise the transcription termination sequence, it is preferable that the transcription termination sequence is disposed immediately under the structural gene. Furthermore, the recombinant vector may comprise a gene controlling the promoter.

As the recombinant vector, it is preferable to use a plasmid in which the distance between the Shine-Dalgarno sequence (also called SD sequence) as a ribosome binding sequence and the start codon is appropriately controlled (for example, 6 to 18 bases).

In the base sequence of the DNA encoding the human haptoglobin, base substitution can be carried out so as to prepare a codon optimal for the expression in the host. In this way, it is possible to improve the production rate of the target human haptoglobin.

As the recombinant vector, any of recombinant vectors can be used as long as it can function in the host cell to be used. Examples thereof include pBTrp2, pBTacl, pBTac2 (all manufactured by Roche Diagnostics), pKK233-2 (Pharmacia LLC.), pSE280 (Invitrogen), pGEMEX-1 (Promega Corporation), pQE-8 (QIAGEN), pKYP10 (JP-A-S58-110600), pKYP200 [Agricultural Biological Chemistry, 48, 669 (1984)], pLSA1 [Agric Biol. Chem., 53, 277 (1989)], pGEL1 [Proc. Natl. Acad. Sci. USA, 82, 4306 (1985)], pBluescript II SK (-) (Stratagene Corporation), pTrs30 [prepared from E. coli JM109/pTrS30 (FERM BP-5407)], pTrs32 [prepared from E. coli JM109/pTrS32 (FERM BP-5408)], pGHA2 [prepared from E. coli IGHA2 (FERM BP-400), JP-A-S60-221091], pGKA2 [prepared from E. coli IGKA2 (FERM BP6798), JP-A-S60-221091], pTerm2 (US Patent No. 4,686,191, US Patent No. 4,939,094, US Patent No. 5,160,735), pSupex, pUB110, pTP5, pC194, pEG400 [J. Bacteriol., 172, 2392 (1990)], pGEX (Pharmacia LLC.), pET system (Novagen), pME18SFL3, and the like.

As the promoter, any promoter may be used as long as it can function in the host cell to be used. Examples thereof include promoters derived from E. coli or phages, such as a trp promoter (Ptrp), an lac promoter, a PL promoter, a PR promoter, and a T7 promoter. Furthermore, it is also possible to use promoters obtained by artificially modify design, such as a tandem promoter in which two Ptrp promoters are connected to each other in series, a tac promoter, an IacT7 promoter, and an letl promoter.

Examples of the host cell include E. coli XL-1 Blue, E. coli XL2-Blue, E. coli DH1, E. coli MC1000, E. coli KY3276, E. coli W1485, E. coli JM109, E. coli HB101, E. coli No. 49, E. coli W3110, E. coli NY49, E. coli DH5$\alpha$, and the like.

As the method for introducing the recombinant vector into the host cell, any method can be used as long as DNA can be introduced into the host cell to be used. Examples thereof include a method using a calcium ion [Proc. Natl. Acad. Sci. USA, 69, 2110 (1972), Gene, 17, 107 (1982), Molecular & General Genetics, 168, 111 (1979)].

In a case where an animal cell is used as a host, as the recombinant vector, any recombinant vector can be used as long as it can function in the animal cell. Examples thereof include pcDNA I, pcDM8 (all manufactured by Funakoshi Co., Ltd.), pAGE107 [JP-A-H03-22979; Cytotechnology, 3, 133 (1990)], pAS3-3 (JP-A-H02-227075), pcDM8 [Nature, 329, 840 (1987)], pcDNA I/Amp, pcDNA3.1, pREP4 (all manufactured by Invitrogen), pAGE103 [J. Biochemistry, 101, 1307 (1987)], pAGE210, pME18SFL3, pKANTEX93 (International Publication No. 97/10354), N5KG1val (US Patent No. 6001358), INPEP4 (Biogen-IDEC Inc.), a transposon vector (International Publication No. 2010/143698), and the like.

As the promoter, any promoter can be used as long as it can function in the animal cell. Examples thereof include a promoter of an immediate early (IE) gene of cytomegalovirus (CMV), an early promoter of SV40, a promoter of retrovirus, a metallothionein promoter, a heat shock promoter, an SR$\alpha$ promoter, and a promoter or an enhancer of Moloney murine leukemia virus. Furthermore, an enhancer of IE gene of human CMV may be used in combination with the promoter.

Examples of the host cell include a human leukemia cell, a Namalwa cell, a COS cell derived from monkey kidney tissue, a CHO cell derived from Chinese hamster's ovarian tissue [Journal of Experimental Medicine, 108, 945 (1958); Proc. Natl. Acad. Sci. USA, 60, 1275 (1968); Genetics, 55, 513 (1968); Chromosoma, 41, 129 (1973); Methods in Cell Science, 18, 115 (1996); Radiation Research, 148, 260 (1997); Proc. Natl. Acad. Sci. USA, 77, 4216 (1980); Proc. Natl. Acad. Sci. USA, 60,1275 (1968); Cell, 6,121 (1975); Molecular Cellgenetics, Appendix I, II (pp.883-900)], CHO/DG44, CHO-K1 (ATCC NO: CCL-61), DUkXB11 (ATCC NO: CCL-9096), Pro-5 (ATCC NO: CCL-1781), CHO-S (Life Technologies, Cat #11619), Pro-3, a rat myeloma cell line YB2/3HL. P2. G11. 16Ag. 20 cell (also called YB2/0 cell), a mouse myeloma cell line NS0 cell, a mouse myeloma cell line SP2/0-Ag14 cell, a BHK or HBT5637 cell derived from Syrian hamster's kidney tissue (JP-AS63-000299), and the like.

As the method for introducing the recombinant vector into the host cell, any method can be used as long as DNA can be introduced into an animal cell. Examples thereof include an electroporation method [Cytotechnology, 3, 133 (1990)], a calcium phosphate method (JP-A-H02-227075), a lipofection method [Proc. Natl. Acad. Sci. USA, 84, 7413 (1987)], and the like.

The transformant derived from the microorganism, the animal cell, or the like containing the recombinant vector, into which DNA encoding the human haptoglobin obtained as above is incorporated, is cultured in a medium such that the human haptoglobin is generated and accumulated in the culture, and the human haptoglobin is collected from the culture. In this way, the human haptoglobin can be produced. The method for culturing the transformant in a medium can be performed according to a method generally used for culturing the host.

In a case where the human haptoglobin is expressed in a cell derived from eukaryote, it is possible to obtain human haptoglobin to which sugar or a sugar chain is added.

In a case where a microorganism transformed by a recombinant vector using an inductive promoter is cultured, if necessary, an inducer may be added to the medium. For example, in a case where a microorganism transformed by a recombinant vector using an lac promoter is cultured, isopropyl-$\beta$-D-thiogalactopyranoside or the like may be added to the medium, and in a case where a microorganism transformed by a recombinant vector using a trp promoter is cultured, indole acrylate or the like may be added to the medium.

Examples of the medium for culturing the transformant obtained using an animal cell as a host include the generally used RPMI 1640 medium [The Journal of the American Medical Association, 199, 519 (1967)], the Eagle's MEM [Science, 122, 501 (1952)], the Dulbecco's modified MEM [Virology, 8, 396 (1959)], the 199 medium [Proc. Soc. Exp. Biol. Med., 73, 1 (1950)], the Iscove's Modified Dulbecco's Medium (IMDM), media obtained by adding fetal bovine serum (FBS) to these media, and the like. Generally, the transformant is cultured for 1 to 7 days in the presence of 5% $CO_2$, for example, under the condition of pH of 6 to 8 and a temperature of 30°C to 40°C. During the culturing, if necessary, an antibiotic such as kanamycin or penicillin may be added to the medium.

As the method for expressing the gene encoding the human haptoglobin, in addition to direct expression, a method such as secretory production or fusion protein expression [Molecular Cloning, A Laboratory Manual, Second Edition, Cold Spring Harbor Laboratory Press (1989)] can be used.

The method for producing the human haptoglobin include a method of producing the human haptoglobin in a host cell, a method for causing the human haptoglobin to be secreted out of a host cell, and a method for producing the human haptoglobin on the extracellular membrane of a host cell. Among these, an appropriate method can be selected by changing the host cell to be used or changing the structure of the human haptoglobin to be produced.

In a case where the human haptoglobin is produced in the interior of the host cell or on the extracellular membrane of the host cell, by using the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)], the method described in JP-A-H05-336963 or International Publication No. 94/23021, or the like, it is possible to cause the human haptoglobin to be actively secreted out of the host cell.

In addition, it is possible to increase the amount of the produced human haptoglobin by using a gene amplification system (JP-A-H2-227075) using a dihydrofolic acid reductase gene or the like.

The obtained human haptoglobin can be isolated and purified as below, for example.

In a case where the human haptoglobin is expressed in a dissolved state in a cell, the cell is collected by centrifugation after the end of culture, suspended in an aqueous buffer, and disrupted using an ultrasonic disruptor, a French press, the Manton-Gaulin homogenizer, a dyno mill, or the like, thereby obtaining a cell-free extract.

A purified sample can be obtained from the supernatant obtained by performing centrifugation on the aforementioned cell-free extract by using general protein isolation and purification methods, that is, techniques such as a solvent extraction method, a salting-out method by ammonium sulfate or the like, a desalinization method, a precipitation method by an organic solvent, an anion exchange chromatography method using a resin such as diethylaminoethyl (DEAE)-sepharose or DIAION HPA-75 (Mitsubishi Chemical Corporation), a cation exchange chromatography method using a resin such as S-Sepharose FF (Pharmacia LLC.), a hydrophobic chromatography method using a resin such as butyl sepharose or phenyl sepharose, a gel filtration method using a molecular sieve, an affinity chromatography method, a chromatofocusing method, and an electrophoresis method such as isoelectric focusing electrophoresis which are used singly or in combination.

In a case where the human haptoglobin is expressed in a cell by forming an insoluble substance, the cell is collected and then disrupted in the same manner as described above and subjected to centrifugation, thereby collecting insoluble human haptoglobin as a precipitated fraction. The collected insoluble human haptoglobin is solubilized using a protein denaturant. After the solution is diluted or dialyzed such that the normal 3-dimensional structure of the human haptoglobin is restored, a purified polypeptide sample can be obtained by the same isolation and purification method as that described above.

In a case where human haptoglobin or a derivative thereof such as human haptoglobin modified with sugar is secreted out of the cell, the human haptoglobin or a derivative thereof such as human haptoglobin modified with sugar can be collected from the culture supernatant. A soluble fraction can be obtained by treating the culture by a technique such as centrifugation in the same manner as described above, and a purified sample can be obtained from the soluble fraction by using the same isolation and purification method as that described above.

Furthermore, the human haptoglobin used in the present invention can be manufactured by a chemical synthesis method such as the Fmoc method or the tBoc method. In addition, the human haptoglobin can also be chemically synthesized using peptide synthesizers from Advanced ChemTech, PerkinElmer Inc., Pharmacia LLC., Protein Technology Instruments, Synthecell/Vega Biomolecules Corp., Perceptive Biosystems, Shimadzu Corporation, and the like.

(2) Animal immunization and preparation of antibody-producing cells for fusion

Animals such as 3 to 20-week-old mice, rats, or hamsters are immunized with the antigen obtained in (1), and antibody-producing cells are collected from the spleen, the lymph nodes, and the peripheral blood of the animals. In a case where the immunogenicity is low, and a sufficient increase in the antibody titer is not confirmed in the animals, a haptoglobin knockout mouse can also be used as an animal to be immunized. Furthermore, antibody-producing cells can be collected from human antibody-producing animals.

The animals are immunized, for example, by administering the antigen together with an appropriate adjuvant such as complete Freund's adjuvant or aluminum hydroxide gel and a Bordetella pertussis vaccine into the subcutis, the caudal vein portion, the vein, or the peritoneum of the animals. In a case where the antigen is a partial peptide, a conjugate of the antigen and a carrier protein such as bovine serum albumin (BSA) or Keyhole Limpet Hemocyanin (KLH) is prepared and used as an immunogen.

After the first administration of the antigen, the antigen is administered once to ten times at the interval of 1 to 4 weeks. On the $1^{st}$ to $14^{th}$ day after each administration, blood is collected from the venous plexus of the ocular fundus or from the caudal vein, and the antibody titer in the serum is measured using enzyme immunoassay [Antibodies-A Laboratory Manual, Cold Spring Harbor Laboratory (1988)] and the like.

On the $3^{rd}$ to $7^{th}$ day after the final administration of the antigen, tissue containing antibody-producing cells is extracted from the spleen, the lymph nodes, and the like of the immunized animals, and the antibody-producing cells are collected. In a case where spleen cells are used, the spleen is ground, dissociated, and then subjected to centrifugation, and the erythrocytes are removed, thereby obtaining antibody-producing cells for fusion.

(3) Preparation of myeloma cells

As myeloma cells, established cells obtained from a mouse are used. For example, 8-azaguanine-resistant mouse (BALB/c-derived) myeloma cell line P3-X63Ag8-U1 (P3-U1) [Current Topics in Microbiology and Immunology, 18, 1, (1978)], P3-NS1/1Ag41 (NS-1) [European J. Immunology, 6, 511 (1976)], SP2/0-Ag14 (SP-2) [Nature, 276, 269 (1978)], P3-X63-Ag8653 (653) [J. Immunology, 123, 1548 (1979)], P3-X63-Ag8 (X63) [Nature, 256, 495 (1975)], and the like are used.

The myeloma cells are subcultured in a normal medium [RPMI 1640 medium to which glutamine, 2-mercaptoethanol, gentamycin, FBS, and 8-azaguanine are added] and then subcultured in a normal medium 3 to 4 days before cell fusion, such that a cell count of equal to or higher than $2 \times 10^7$ is secured on the day of fusion.

(4) Cell fusion and preparation of monoclonal antibody-producing hybridoma

The antibody-producing cells for fusion obtained (2) and the myeloma cells obtained in (3) are thoroughly washed with a minimum essential medium (MEM) or PBS (containing 1.83 g of disodium phosphate, 0.21 g of potassium phosphate, 7.65 g of sodium chloride, and 1 L of distilled water, pH of 7.2), mixed together such that the cell count becomes antibody-producing cell for fusion:myeloma cell = 5 to 10:1, and subjected to centrifugation, and then the supernatant is removed.

The precipitated cell groups are thoroughly dissociated, and then a mixed solution of polyethylene glycol 1000 (PEG-1000), the MEM, and dimethyl sulfoxide is added thereto at 37°C with stirring. Furthermore, 1 to 2 mL of the MEM is added thereto several times at an interval of 1 to 2 minutes, and then the MEM is added thereto such that the total amount thereof becomes 50 mL. The solution is subjected to centrifugation, and then the supernatant is removed. The precipitated cell groups are gently dissociated, and then the cells are gently suspended in the HAT medium [normal medium to which hypoxanthine, thymidine, and aminopterin are added]. The suspension is cultured for 7 to 14 days in a 5% $CO_2$ incubator at 37°C.

After the culturing, a portion of the culture supernatant is extracted by a hybridoma selection method which will be described later, and cell groups which are reactive with a human haptoglobin-containing antigen and are unreactive to human haptoglobin-free antigen are selected. Then, cloning is repeated twice by a limiting dilution method [the HT medium (medium obtained by removing aminopterin from the HAT medium) is used for the first cloning, and a normal medium is used for the second cloning], and a cell found to be stable and have a high antibody titer is selected as a monoclonal antibody-producing hybridoma.

(5) Preparation of purified monoclonal antibody

The monoclonal antibody-producing hybridoma obtained in (4) is administered to 8 to 10-week-old mice or hairless mice, which are treated with pristane [0.5 mL of 2,6,10,14-tetramethylpentadecane (pristane) is given to the animals by intraperitoneal administration, and the animals are raised for 2 weeks], by intraperitoneal injection. On the $10^{th}$ to $21^{st}$ day, the hybridoma turns ascites cancer. The ascitic fluid is collected from the mice, the solid content is removed by centrifugation, and then salting-out is carried out using 40% to 50% ammonium sulfate. Purification is performed using a caprylic acid precipitation method, a DEAE-sepharose column, a protein-A column, or a gel filtration column, and an IgG or IgM fractions are collected and used as a purified monoclonal antibody.

The monoclonal antibody-producing hybridoma obtained in (4) is cultured in the RPMI 1640 medium, to which 10% FBS is added, and the like and then subjected to centrifugation, the supernatant is removed, and the hybridoma is suspended in a Hybridoma-SFM, and cultured for 3 to 7 days. The purified monoclonal antibody can also be obtained in a manner in which the obtained cell suspension is subjected to centrifugation, the monoclonal antibody is purified from the obtained supernatant by using a protein A-column or a protein G-column, and IgG fractions are collected. 5% Daigo's GF21 can also be added to the Hybridoma-SFM.

The subclass of the antibody is determined by the enzyme immunoassay by using a subclass typing kit. The quantity of the protein is calculated by the Lowry's method or the absorbance at 280 nm.

(6) Selection of monoclonal antibody

As will be described below, the monoclonal antibody is selected by analyzing the binding activity thereof to the human haptoglobin and the mouse haptoglobin by enzyme-linked immunosorbent assay (ELISA).

The human haptoglobin or the mouse haptoglobin is dispensed into a plate such as a 96-well plate, and the protein is immobilized. Then, as a primary antibody, a test substance such as the serum, the culture supernatant of the hybridoma, or the purified monoclonal antibody is dispensed into the plate and allowed to react. The plate having undergone the reaction is thoroughly washed with PBS containing 0.1% Tween (hereinafter, described as PBST). Thereafter, as a secondary antibody, an anti-immunoglobulin antibody labeled with biotin, an enzyme, a chemiluminescent substance, a radioactive compound, or the like is dispensed into the plate and allowed to react. The plate is thoroughly washed with PBST or the like, and then a reaction is performed according to the labeling substance of the secondary antibody, and a monoclonal antibody specifically reacts with the human haptoglobin or the mouse haptoglobin is selected.

The monoclonal antibody binding to the human haptoglobin and the mouse haptoglobin in competition with the monoclonal antibody of the present invention can be obtained by adding a test antibody to a binding reaction detection system using the aforementioned ELISA and allowing the antibodies to react with each other. That is, by screening out the antibody that inhibits the binding of the monoclonal antibody of the present invention at the time of adding the test antibody, it is possible to obtain the monoclonal antibody which binds to the amino acid sequences of the human haptoglobin and the mouse haptoglobin or to the 3-dimensional structure structures thereof in competition with the monoclonal antibody obtained in the present invention.

Furthermore, the monoclonal antibody, binding to the same epitope as the epitope to which the monoclonal antibody of the present invention binds and which is comprised in the human haptoglobin or the mouse haptoglobin, can be obtained by identifying the epitope of the antibody obtained by the binding reaction detection system using ELISA described above, preparing a partial synthetic peptide of the identified epitope, a synthetic peptide mimicking the 3-dimensional structure of the epitope, or the like, and performing immunization.

2. Preparation of recombinant antibody

Hereinafter, as a preparation example of a recombinant antibody, a method for preparing a human chimeric antibody and a humanized antibody will be described.

(1) Establishment of vector for expressing recombinant antibody

The vector for expressing a recombinant antibody is a recombinant vector for animal cells into which DNA encoding CH and CL of a human antibody is incorporated, and can be established by cloning DNAs encoding CH and CL of a human antibody to the recombinant vector for animal cells.

As the C region of a human antibody, CH and CL of any human antibody can be used. For example, $\gamma$ 1 subclass CH and $\kappa$ class CL of a human antibody and the like can be used. As the DNAs encoding CH and CL of the human antibody, cDNA is used, and chromosomal DNA consisting of exon and intron can also be used.

As the recombinant vector for animal cells, any vector can be used as long as a gene encoding the C region of a human antibody can be incorporated into and expressed in the vector. For example, pAGE107 [Cytotechnol., 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981)], pSGlbd2-4 [Cytotechnol, 4,173 (1990)], pSE1UK1Sed1-3 [Cytotechnol., 13, 79 (1993)], and the like.

As a promoter and an enhancer in the recombinant vector for animal cells, an early promoter of SV40 [J. Biochem., 101, 1307 (1987)], Moloney murine leukemia virus LTR [Biochem. Biophys. Res. Commun., 149, 960 (1987)], a promoter [Cell, 41, 479 (1985)] and an enhancer [Cell, 33, 717 (1983)] of the immunoglobulin H chain, and the like are used.

As the vector for expressing a recombinant antibody, in view of ease of establishing the recombinant vector, ease of introducing the vector into an animal cell, and establishing balance between the amount of the H chain and the amount of the L chain of the antibody expressed in the animal cell, a recombinant vector of a type (tandem type) in which the H chain and the L chain of the antibody are present on the same vector [J. Immunol. Methods, 167, 271 (1994)] is used. However, a type in which the H chain and the L chain of the antibody are present on different vectors can also be used. As the tandem-type recombinant vector, pKANTEX93 (International Publication No. 97/10354), pEE18 [Hybridoma, 17, 559 (1998)], and the like are used.

(2) Obtaining cDNA encoding V region of antibody derived from non-human animal and amino acid sequence analysis

In the manner described below, cDNA encoding VH and VL of a non-human antibody can be obtained, and the amino acid sequence can be analyzed.

mRNA is extracted from a hybridoma cell producing a non-human antibody, and cDNA is synthesized. The synthesized cDNA is cloned to a vector such as a phage or a plasmid, thereby preparing a cDNA library.

From the library, a recombinant phage or a recombinant plasmid having cDNA encoding VH or VL is isolated using, as a probe, DNA encoding the portion of the C region or the portion of the V region of a mouse antibody. The whole base sequence of VH or VL of the target mouse antibody on the recombinant phage or the recombinant plasmid is determined, thereby estimating the whole amino acid sequence of VH or VL from the base sequence.

As the non-human animal for preparing the hybridoma cell producing a non-human antibody, a mouse, a rat, a hamster, a rabbit, or the like is used. However, as long as the hybridoma cell can be prepared, any animal can be used.

For preparing total RNA from the hybridoma cells, a guanidine thiocyanate-cesium trifluoroacetate method [Methods in Enzymol., 154, 3 (1987)] or a kit such as an RNA easy kit (QIAGEN) is used.

For preparing mRNA from total RNA, oligo (dT) fixation cellulose column method [Molecular Cloning, A Laboratory Manual, Second Edition, Cold Spring Harbor Laboratory Press (1989)] or a kit such as an Oligo-dT30 <Super> mRNA Purification Kit (TAKARA BIO INC.) is used. Furthermore, it is also possible to prepare mRNA from the hybridoma cell by using a kit such as a Fast Track mRNA Isolation Kit (Invitrogen) or a QuickPrep mRNA Purification Kit (GE Healthcare Bio-Sciences).

For synthesizing cDNA or preparing the cDNA library, a known method [Molecular Cloning, A Laboratory Manual, Second Edition, Cold Spring Harbor Laboratory Press (1989), Current Protocols in Molecular Biology, Supplement 1, John Wiley & Sons (1987-1997)], a SperScript Plasmid System for cDNA Synthesis and Plasmid Cloning (Invitrogen), or a kit such as a ZAP-cDNA Synthesis Kit (Stratagene Corporation) is used.

At the time of preparing the cDNA library, as a vector into which cDNA, synthesized using mRNA extracted from the hybridoma cell as a template, is incorporated, any vector can be used as long as the cDNA can be incorporated into the vector. For example, ZAP ExPress [Strategies, 5, 58 (1992)], pBluescript II SK (+) [Nucleic Acids Research, 17, 9494 (1989)], λ ZAPII (Stratagene Corporation), λgt10, λgt11 [DNA Cloning: A Practical Approach, I, 49 (1985)], Lambda BlueMid (Clontech Laboratories, Inc.), λEx Cell, pT7T3-18U (Pharmacia), pcD2 [Mol. Cell. Biol., 3, 280 (1983)], or pUC18 [Gene, 33, 103 (1985)], and the like are used.

As the E. coli into which the cDNA library established using a phage or plasmid vector is introduced, any E. coli can be used as long as the cDNA library can be introduced into the bacteria, and the bacteria can express and retain the cDNA library. For example, XL-1Blue MRF [Strategies, 5, 81 (1992)], C600 [Genetics, 39, 440 (1954)], Y1088, Y1090 [Science, 222, 778 (1983)], NM522 [J. Mol. Biol., 166, 1 (1983)], K802 [J. Mol. Biol., 16, 118 (1966)], JM105 [Gene, 38, 275 (1985)], and the like are used.

For selecting a cDNA clone encoding VH or VL of the non-human antibody from the cDNA library, a colony hybridization method using a probe labeled with an isotope or fluorescence, a plaque hybridization method [Molecular Cloning, A Laboratory Manual, Second Edition, Cold Spring Harbor Laboratory Press (1989)], and the like are used.

In addition, it is also possible to prepare the cDNA encoding VH or VL by preparing a primer and performing a polymerase chain reaction method [hereinafter, described as PCR method, [Molecular Cloning, A Laboratory Manual, Second Edition, Cold Spring Harbor Laboratory Press (1989), Current Protocols in Molecular Biology, Supplement 1, John Wiley & Sons (1987-1997)] by using the cDNA or the cDNA library synthesized from mRNA as a template.

The selected cDNA is cleaved using an appropriate restriction enzyme and then cloned to a plasmid such as pBluescript SK (-) (Stratagene Corporation), and the base sequence of the cDNA is determined by a generally used base sequence analysis method and the like. In the base sequence analysis method, for example, a reaction such as a deoxy method [Proc. Natl. Acad. Sci. USA, 74, 5463 (1977)] or the like is performed, and then an automatic base sequence analyzer such as ABI PRISM 3700 (PE Biosystems) or A. L. F. DNA sequencer (Pharmacia LLC.) is used.

The whole amino acid sequence of each of VH and VL is estimated from the determined base sequence and compared with the whole amino acid sequence of each of VH and VL of a known antibody [A. L. F. DNA, US Dept. Health and Human Services (1991)]. In this way, whether the obtained cDNA encodes the whole amino acid sequence of each of VH and VL of the antibody comprising a secretory signal sequence is checked.

By comparing the whole amino acid sequence of each of VH and VL of the antibody comprising a secretory signal sequence with the whole amino acid sequence of each of VH and VL of a known antibody [A. L. F. DNA, US Dept. Health and Human Services (1991)], the length of the secretory signal sequence and the N-terminal amino acid sequence can be estimated, and the subgroups to which the amino acid sequences belong can be ascertained. Furthermore, the amino acid sequence of each of CDRs of VH and VL can also be found by the comparison with the amino acid sequence of each of VH and VL of a known antibody [A. L. F. DNA, US Dept. Health and Human Services (1991)].

In addition, for example, by performing identity search such as BLAST method [J. Mol. Biol., 215, 403 (1990)] on a certain database such as SWISS-PROT or PIR-Protein by using the obtained whole amino acid sequences of VH and VL, it is possible to check whether the whole amino acid sequences of VH and VL are novel ones.

(3) Establishment of recombinant vector for expressing human chimeric antibody

By cloning the cDNAs encoding VH and VL of a non-human antibody to the upper stream of each of the genes encoding CH or CL of a human antibody in the vector for expressing a recombinant antibody obtained in (1), a recombinant vector for expressing a human chimeric antibody can be established.

In order to connect the 3'-terminal side of cDNA encoding VH or VL of the non-human antibody to the 5'-terminal side of CH or CL of the human antibody, cDNAs of VH and VL are prepared which is designed such that the base sequence of the connection portion encodes appropriate amino acids and becomes an appropriate restriction enzyme recognition sequence.

The prepared cDNAs of VH and VL are cloned to the upper stream of each of the genes encoding CH or CL of the human antibody in the vector for expressing a recombinant antibody obtained in (1) such that the cDNAs are expressed in an appropriate form, thereby establishing a recombinant vector for expressing a human chimeric antibody.

Furthermore, each of cDNAs encoding VH or VL of a non-human antibody can be amplified through a PCR method by using synthetic DNA having an appropriate restriction enzyme recognition sequence on both terminals, and cloned to the vector for expressing a recombinant antibody obtained in (1).

(4) Establishment of cDNA encoding V region of humanized antibody

cDNA encoding VH or VL of a humanized antibody can be established as below.

Each of amino acid sequences of FR of VH or VL of a human antibody, to which an amino acid sequence of CDR of VH or VL of a non-human antibody will be grafted, is selected. As the amino acid sequence of FR to be selected, any amino acid sequence can be used as long as it is derived from a human antibody.

For example, the amino acid sequence of FR of a human antibody registered in a database such as Protein Data Bank, the amino acid sequence common to the subgroups of FR of a human antibody [A. L. F. DNA, US Dept. Health and Human Services (1991)], or the like is used. In order to prevent the decrease in the binding activity of the antibody, an amino acid sequence of FR is selected which shares high identity (at least equal to or higher than 60%) with the amino acid sequence of FR of VH or VL of the original antibody as far as possible.

Then, each of the amino acid sequences of CDRs of the original antibody is grafted to the selected amino acid sequence of FR of VH or VL of the human antibody, thereby designing each of the amino acid sequences of VH or VL of a humanized antibody. The designed amino acid sequence is converted into a DNA sequence in consideration of the frequency of use of the codon found in the base sequence of the gene of the antibody [A. L. F. DNA, US Dept. Health and Human Services (1991)], thereby designing each of the DNA sequences encoding the amino acid sequence of VH or VL of the humanized antibody.

Based on the designed DNA sequences, several synthetic DNA strands having a length consisting of around 100 bases are synthesized, and a PCR reaction is performed using these. In this case, it is preferable to design 6 DNA strands for each of the H chain and the L chain based on the reaction efficiency of the PCR reaction and the length of DNA which can be synthesized.

Furthermore, by introducing an appropriate restriction enzyme recognition sequence to the 5'-terminal of the synthetic DNA positioned on both terminals, it is possible to easily clone cDNA, which encodes VH or VL of the humanized antibody, to the vector for expressing a recombinant antibody obtained in (1).

After the PCR reaction, the amplification products are respectively cloned to a plasmid such as pBluescript SK (-) (Stratagene Corporation), and the base sequence is determined by the same method as the method described in (2), thereby obtaining a plasmid having a DNA sequence encoding an amino acid sequence of the full-length H chain or the full-length L chain of a desired humanized antibody.

Alternatively, materials, which are obtained by synthesizing each of the full-length VH and the full-length VL as a single-stranded long-chain DNA based on the designed DNA sequence, can also be used instead of the aforementioned PCR amplification products. In addition, by introducing an appropriate restriction enzyme recognition sequence into both terminals of a synthetic long-chain DNA, it is possible to easily clone the cDNA encoding VH or VL of the humanized antibody to the vector for expressing a recombinant antibody obtained in (1).

(5) Modification of amino acid sequence of V region of humanized antibody

In a case where only CDRs of VH and VL of a non-human antibody are simply grafted to FRs of VH and VL of a human antibody, the antigen binding activity of the humanized antibody is further reduced compared to that of the original non-human antibody [BIO/TECHNOLOGY, 9, 266 (1991)].

In the humanized antibody, by maintaining amino acid residues, which are directly involved in the binding to an antigen, and amino acid residues, which interact with the amino acid residue of CDR, in the amino acid sequences of FR of VH and VL of the human antibody, maintaining the 3-dimensional structure of the antibody, identifying amino acids residues which are indirectly involved with the binding to an antigen, and substituting these amino acid residues with amino acid residues of the original non-human antibody, the reduced antigen binding activity can be increased.

In a case where X-ray crystallographic analysis [J. Mol. Biol., 112, 535 (1977)], computer modeling [Protein Engineering, 7, 1501 (1994)], or the like is used for identifying amino acid residues of FR involved in the antigen binding activity, it is possible to establish and analyze the 3-dimensional structure of the antibody. Furthermore, by preparing several kinds of modified antibodies for each of the antibodies, repeatedly investigating the correlation thereof with the antigen binding activity, and undergoing trial and error, it is possible to obtain a modified humanized antibody having necessary antigen binding activity.

The amino acid residues of FR of VH and VL of the human antibody can be modified by performing the PCR reaction described in (4) by using synthetic DNA for modification. The base sequence of the amplification product obtained after the PCR reaction is determined by the method described in (2), and whether modification has occurred as intended is checked.

(6) Establishment of recombinant vector for expressing humanized antibody

By cloning each of cDNAs encoding VH or VL of the established recombinant antibody to the upper stream of each of the genes encoding CH or CL of the human antibody in the vector for expressing a recombinant antibody obtained in (1), a recombinant vector for expressing a humanized antibody can be established.

For example, an appropriate restriction enzyme recognition sequence is introduced into the 5'-terminal of the synthetic DNA positioned on both terminals among the synthetic DNAs used at the time of establishing VH or VL of the humanized antibody obtained in (4) and (5), thereby cloning the DNAs to the upper stream of each of the genes encoding CH or CL of the human antibody in the vector for expressing a recombinant antibody obtained in (1) such that the DNAs are expressed in an appropriate form.

(7) Transient expression of recombinant antibody

The antigen binding activity of the prepared plural kinds of humanized antibodies can be efficiently evaluated by performing transient expression of the recombinant antibody by using the recombinant vectors for expressing a recombinant antibody obtained in (3) and (6) and recombinant vectors obtained by modifying the above vectors.

As a host cell into which the recombinant vectors will be introduced, any cell can be used as long as it can express the recombinant antibody. For example, a COS-7 cell (ATCC NO: CRL1651) is used [Methods in Nucleic Acids Res., CRC Press, 283 (1991)].

For introducing the recombinant vector into the COS-7 cell, a DEAE-dextran method [Methods in Nucleic Acids Res., CRC Press (1991)], a lipofection method [Proc. Natl. Acad. Sci. USA, 84, 7413 (1987)], or the like is used.

After the introduction of the recombinant vector, the expression amount and the antigen binding activity of the recombinant antibody in the culture supernatant is measured using enzyme-linked immunosorbent assay [Monoclonal Antibodies-Principles and practice, Thiral Antibodies-Principles and Press (1996), Antibodies-A Laboratory Manual, Cold Spring Harbor Laboratory (1988), Monoclonal Antibody Experiment Manual, Kodansha Scientific Ltd. (1987)] or the like.

(8) Obtaining transformant stably expressing recombinant antibody and preparation of recombinant antibody

By introducing the recombinant vectors for expressing a recombinant antibody obtained in (3) and (6) into an appropriate host cell, it is possible to obtain a transformant stably expressing a recombinant antibody.

For introducing the recombinant vector into the host cell, an electroporation method [JP-A-H02-257891, Cytotechnology, 3, 133 (1990)] or the like is used.

As the host cell into which the recombinant vector will be introduced, any cell can be used as long as it is a host cell which can express the recombinant antibody. For example, CHO-K1 (ATCC NO: CCL-61), DUkXB11 (ATCC NO: CCL-9096), Pro-5 (ATCC NO: CCL-1781), CHO-S (Life Technologies, Cat #11619), a rat myeloma cell YB2/3HL. P2. G11. 16Ag. 20 (or called YB2/0), a mouse myeloma cell NS0, a mouse myeloma cell SP2/0-Ag14 (ATCC NO: CRL1581), a mouse P3-X63-Ag8653 cell (ATCC NO: CRL1580), a CHO cell from which a dihydrofolic acid reductase gene (Dihydrofolate Reductase, hereinafter, described as DHFR) is deleted [Proc. Natl. Acad. Sci. USA, 77, 4216 (1980)], Lec13 having attained lectin resistance [Somatic Cell and Molecular Genetics, 12, 55 (1986)], a CHO cell from which α1,6-fucose transferase gene is deleted (International Publication No. 2005/035586, International Publication No. 02/31140), a rat YB2/3HL. P2. G11. 16Ag. 20 cell (ATCC NO: CRL1662), and the like are used.

It is also possible to use host cells obtained by reducing or removing the activity of a protein such as an enzyme involved in the synthesis of glyconucleotide GDP-fucose in a cell, a protein such as an enzyme involved in the modification of a sugar chain in which position 1 of fucose is α-bound to position 6 of N-acetylglucosamine at the reducing terminal of N-glycoside-linked hybrid-type sugar chain, a protein involved in the transport of a glyconucleotide GDP-fucose to the Golgi body in a cell, or the like, such as a CHO cell from which α1,6-fucose transferase gene is deleted (International Publication No, 2005/035586 and International Publication No. 02/31140).

After the introduction of the recombinant vector, the transformant stably expressing the recombinant antibody is selected by being cultured in an animal cell culture medium containing a drug such as G418 sulfate (JP-A-H02-257891).

As the animal cell culture medium, the RPMI 1640 medium (Invitrogen), the GIT medium (NIHON PHARMACEUTICAL CO., LTD.), the EX-CELL301 medium (JRH Biosciences), IMDM (Invitrogen), Hybridoma-SFM (Invitrogen), media obtained by adding various additives such as FBS to the above media, and the like are used.

By culturing the obtained transformant in the medium, the recombinant antibody is expressed and accumulated in the culture supernatant. The expression amount and the antigen binding activity of the recombinant antibody in the culture supernatant can be measured by the ELISA method and the like. Furthermore, the expression amount of the recombinant antibody produced from the transformant can be increased using a DHFR amplification system (JP-A-H02-257891) or the like.

The recombinant antibody is purified from the culture supernatant of the transformant by using the protein A-column [Monoclonal Antibodies-Principles and practice, Third edition, Academic Press (1996), Antibodies-A Laboratory Manual, Cold Spring Harbor Laboratory (1988)]. Furthermore, the method used for protein purification, such as gel filtration, ion exchange chromatography, or ultrafiltration can be used in combination.

The molecular weight of the H chain or the L chain of the purified recombinant antibody or the total molecular weight of the antibody molecule can be measured using a polyacrylamide gel electrophoresis method [Nature, 227, 680 (1970)], the Western blotting method [Monoclonal Antibodies-Principles and practice, Third edition, Academic Press (1996), Antibodies-A Laboratory Manual, Cold Spring Harbor Laboratory (1988)], or the like.

3. Evaluation of activity of purified monoclonal antibody

The activity of the purified monoclonal antibody of the present invention, for example, the binding activity thereof to human haptoglobin can be measured using the binding reaction detection system using ELISA in (6) of 1 described above.

4. Method for controlling effector activity of antibody

As the method for controlling the effector activity of the monoclonal antibody of the present invention, a method of controlling the amount of fucose (also called core fucose) α1,6-bound to N-acetylglucosamine (GlcNAc) at the reducing terminal of a N-glucoside-linked sugar chain linked to asparagine (Asn) at position 297 of the Fc domain of an antibody (International Publication No. 2005/035586, International Publication No. 2002/31140, and International Publication No. 00/61739), a method of controlling the effector activity by modifying the amino acid residues in the Fc domain of an antibody, and the like are known. The effector activity of the monoclonal antibody of the present invention can be controlled by any of these methods.

The effector activity refers to antibody-dependent activity performed through the Fc domain of an antibody. As the effector activity, the ADCC activity, the CDC activity, the ADCP activity by a phagocyte such as a macrophage or a dendritic cell, and the like are known.

Regarding the method for measuring the effector activity, for example, for the ADCC activity, an inflammatory cell as a target, a peripheral blood mononuclear cell (PBMC) as an effector, and an antibody specific to the inflammatory cell are mixed together and incubated for about 4 hours, and then free lactate dehydrogenase (LDH) as an index of cell damage can be measured as the effector activity. Alternatively, the human PBMC is mixed, for example, with an antibody recognizing an antigen such as CD20 specific to a blood cell and incubated, and then the free LDH or the reduction in the cell count by flow cytometry can be measured as the effector activity.

By controlling the content of fucose of the N-linked hybrid-type sugar chain of Fc of the antibody, the effector activity of the antibody can be increased or reduced. As the method for reducing the content of fucose bound to the N-linked hybrid-type sugar chain linked to Fc of the antibody, the antibody is expressed using a CHO cell from which α1,6-fucose transferase gene is deleted, and in this way, it is possible to obtain an antibody to which fucose is not bound. The binding activity of the antibody to which fucose is not bound (hereinafter, also called defucosylated antibody) to CD16a is higher than that of the antibody to which fucose is bound (hereinafter, also called fucosylated antibody). Furthermore, it is known that accordingly, the defucosylated antibody exhibits high ADCC activity.

As the method for increasing the content of fucose-bound to the N-linked hybrid-type sugar chain bound to Fc of the antibody, the antibody is expressed using a host cell into which an α1,6-fucose transferase gene is introduced, and in this way, an antibody to which fucose is bound can be obtained. The binding activity of the antibody, to which fucose is bound, to CD16a is lower than that of the antibody to which fucose is not bound. Furthermore, accordingly, the fucosylated antibody exhibits low ADCC activity.

In addition, by modifying the amino acid residues in the Fc domain of the antibody, the ADCC activity or the CDC activity can be increased or reduced. For example, by using the amino acid sequence of the Fc domain described in US Patent Application Publication No. 2007/0148165, the CDC activity of the antibody can be increased.

Furthermore, by modifying amino acids as described in US Patent No. 6,737,056, US Patent No. 7,297,775, or US Patent No. 7,317,091, the ADCC activity or the CDC activity can be increased or reduced. By combining the aforementioned methods and using them for one antibody, it is possible to obtain an antibody whose effector activity is controlled.

5. Method for treating diseases by using monoclonal antibody of the present invention

The antibody of the present invention can be used for treating autoimmune diseases involved in cell damage caused by an autoantibody.

The autoimmune diseases are not limited, and examples thereof include autoimmune diseases involved in cell damage caused by an autoantibody. Examples of the autoimmune diseases include autoimmune diseases for which an IVIG preparation is used as a treatment agent, such as immune thrombocytopenic purpura, Kawasaki syndrome, ANCA-associated vasculitis, Guillain-Barre syndrome, chronic demyelinating polyradiculoneuropathy, myasthenia gravis, multifocal motor neuropathy, rheumatoid arthritis, systemic lupus erythematosus, autoimmune hemolytic anemia, scleroderma, autoimmune urticaria, pemphigus, Goodpasture's syndrome, psoriatic arthritis, Sjogren's syndrome, and polymyositis/dermatomyositis, and autoimmune diseases in which the production of autoantibodies is involved in the pathological condition, such as IgG4-related diseases.

Examples of the route of administration of the antibody of the present invention include oral administration or parenteral administration such as intraoral administration, intra-airway administration, intrarectal administration, subcutaneous administration, intradermal administration, intravenous administration, or intraperitoneal administration. The antibody of the present invention is administered, for example, in the form of an aerosol, a capsule, a tablet, powder, granules, a syrup, an emulsion, a suppository, an injection, an ointment, or a tape.

Examples of the preparation appropriate for the oral administration include an emulsion, a syrup, a capsule, a tablet, powder, granules, and the like.

The liquid preparation such as the emulsion or the syrup is manufactured using, as additives, water, saccharides such as sucrose, sorbitol, or fructose, glycols such as polyethylene glycol or propylene glycol, oils such as sesame oil, olive oil, or soybean oil, a preservative such as a p-hydroxybenzoic acid ester, flavors such as a strawberry flavor or peppermint, and the like.

The capsule, the tablet, the powder, the granules, and the like are manufactured using, as additives, an excipient such as lactose, glucose, sucrose, or mannitol, a disintegrating agent such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, and the like.

Examples of the preparation appropriate for the parenteral administration include the injection, the suppository, the aerosol, and the like.

The injection is manufactured using a salt solution, a glucose solution, a carrier formed of a mixture of the salt solution and the glucose solution, and the like.

The suppository is manufactured using a carrier such as cacao butter, hydrogenated fat or a carboxylic acid.

The aerosol is manufactured using a carrier, which enables the monoclonal antibody of the present invention or the fragments of the antibody to be dispersed as fine particles without irritating the oral cavity and the mucous membrane of the airway of the user and facilitates the absorption, and the like. As the carrier, for example, lactose or glycerin is used. Furthermore, an aerosol or dry powder can also be manufactured.

The components exemplified above as additives for the preparation appropriate for oral administration can also be added to the aforementioned parenteral agents.

Hereinafter, the present invention will be described based on examples, but the present invention is not limited thereto.

## Examples

### [Example 1] Preparation of soluble CD16a

(1) Preparation of cDNA of human peripheral mononuclear cell

Venous blood (30 mL) was collected from a healthy person, gently mixed with 0.5 mL of sodium heparin (Shimizu Seiyaku), and then mixed with 30 mL of physiological saline (Otsuka Pharmaceutical Co, Ltd.). The mixed solution (10 mL) was gently layered on 4 mL of Lymphoprep (NYCOMED PHARMA AS) and subjected to centrifugation at 2,000 rpm for 30 minutes at room temperature.

The separated mononuclear cell fractions were collected through each of centrifuge tubes, mixed together, suspended in 30 mL of RPMI 1640 (GIBCO) containing 10% FBS (GIBCO) [hereinafter, described as RPMI 1640-FBS (10)], and subjected to centrifugation at 1,200 rpm for 15 minutes at room temperature.

After the centrifugation, the supernatant was removed, and the cells were suspended in 20 mL of RPMI 1640-FBS (10). This washing operation was repeated twice, and then a peripheral blood mononuclear cell suspension at $2 \times 10^6$ cells/mL was prepared using RPMI 1640-FBS (10).

The prepared peripheral blood mononuclear cell suspension (5 mL) was subjected to centrifugation at 800 rpm for 5 minutes at room temperature. After the centrifugation, the supernatant was removed, the cells were suspended in 5 mL of phosphate buffered saline (PBS), and subjected to centrifugation at 800 rpm for 5 minutes at room temperature.

After the centrifugation, the supernatant was removed, and from the obtained pellets, total RNA was prepared using a QIAamp RNA Blood Mini Kit (QIAGEN) according to the attached document.

From the obtained total RNA (2 μg), cDNA was prepared using SUPERSCRIPT (trademark) Preamplification System for First Strand cDNA Synthesis (Life Technologies) according to the attached document.

(2) Obtaining cDNA encoding human CD16a

cDNA was obtained from human CD16a (hereinafter, described as CD16a) as below.

First, from the base sequence of cDNA of CD16a [J. Exp. Med., 170, 481 (1989)], a specific forward primer (represented by SEQ ID NO: 1) comprising a translation start codon and a specific reverse primer (represented by SEQ ID NO: 2) comprising a translation stop codon were designed.

Then, by using cDNA derived from the human peripheral blood mononuclear cell prepared in (1) of Example 1 as a template and two kinds of primers (SEQ ID NOs: 1 and 2) described above, PCR was performed. The PCR was performed based on the instruction attached to DNA polymerase ExTaq (TAKARA BIO INC.).

After the PCR, the reaction solution was purified using a QIAquick PCR Purification Kit (QIAGEN) according to the attached document. The purified DNA was digested using restriction enzymes EcoRI (TAKARA BIO INC.) and BamHI (TAKARA BIO INC.) and then subjected to 0.8% agarose gel electrophoresis, thereby collecting a specific amplified DNA fragment (about 800 bp).

Meanwhile, 2.5 μg of a plasmid pBlueScriptII SK (-) (Stratagene Corporation) was digested using restriction enzymes EcoRI (TAKARA BIO INC.) and BamHI (TAKARA BIO INC.) and then subjected to 0.8% agarose gel electrophoresis, thereby collecting a DNA fragment of about 2.9 kbp.

The DNA fragment of about 800 bp and the fragment of about 2.9 kbp were ligated by using a DNA Ligation Kit Ver. 2.0 (TAKARA BIO INC.) and transformed into an E. coli DH5α strain (Toyobo Co., Ltd), thereby obtaining a plasmid pBsCD16a (V) comprising DNA encoding cDNA (SEQ ID NO: 3) in which the amino acid at position 176 in the amino acid sequence of the human CD16a is valine [hereinafter, described as CD16a (V)].

(3) Obtaining cDNA encoding soluble CD16a

cDNA encoding soluble CD16a (V) [hereinafter, described as soluble CD16a (V)], which has the extracellular domain (amino acids of positions 1 to 193 of SEQ ID NO: 4) of CD 16a and a histidine tag (consist of six histidine residues) on the C-terminal, was established as below.

First, from the base sequence (SEQ ID NO: 3) of cDNA of CD16a (V), a primer FcgR3-1 (SEQ ID NO: 5) specific to the extracellular domain was designed. Then, by using 5 ng of pBs CD16a (V) and a DNA polymerase ExTaq (TAKARA BIO INC.), PCR was performed in the same manner as in (2) of Example 1.

After the PCR, the reaction solution was purified using a QIAquick PCR Purification Kit (QIAGEN) according to the attached document and dissolved in sterile water. The purified DNA was digested using restriction enzymes PstI (TAKARA BIO INC.) and BamHI (TAKARA BIO INC.), and then subjected to 0.8% agarose gel electrophoresis, thereby collecting a specific amplified DNA fragment (about 110 bp).

Meanwhile, 2.5 μg of pBs CD16a (V) was digested using restriction enzymes PstI (TAKARA BIO INC.) and BamHI (TAKARA BIO INC.) and then subjected to 0.8% agarose gel electrophoresis, thereby collecting a fragment of about 3.5 kbp. The DNA fragment of about 110 bp and the fragment of about 3.5 kbp were ligated by using a DNA Ligation Kit Ver. 2.0 (TAKARA BIO INC.) and transformed into an E. coli DH5α strain (Toyobo Co., Ltd), thereby obtaining a plasmid pBssCD16a (V)-His encoding histidine tag-conjugated soluble CD16a (SEQ ID NO: 6).

(4) Establishment of recombinant vector of soluble CD 16a

The plasmid pBssCD16a (V)-His (3.0 μg) obtained in (3) of Example 1 was digested using restriction enzymes EcoRI (TAKARA BIO INC.) and BamHI (TAKARA BIO INC.) and then subjected to 0.8% agarose gel electrophoresis, thereby collecting a fragment of about 620 bp.

Meanwhile, 2.0 μg of the plasmid pKANTEX 93 described in International Publication No. 97/10354 was digested using restriction enzymes EcoRI (TAKARA BIO INC.) and BamHI (TAKARA BIO INC.) and then subjected to 0.8% agarose gel electrophoresis, thereby collecting a fragment of about 10.7 kbp.

The DNA fragment of about 620 bp and the fragment of about 10.7 kbp were ligated by using a DNA Ligation Kit Ver. 2.0 (TAKARA BIO INC.) and transformed into an E. coli DH5α strain (Toyobo Co., Ltd), thereby obtaining a recombinant vector pKANTEX sCD16a (V)-His encoding the histidine tag-conjugated soluble CD16a.

(5) Preparation of cell stably producing soluble CD16a

The recombinant vector pKANTEX sCD16a (V)-His established in (4) of Example 1 was introduced into a rat myeloma YB2/0 cell [ATCC CRL-1662, J. Cell. Biol., 93, 576 (1982)], and a cell stably producing the soluble CD16a was prepared as below.

The plasmid was digested using a restriction enzyme AatII, and 10 μg of the linearized pKANTEX sCD16a (V)-His was introduced into $4 \times 10^6$ YB2/0 cells by the electroporation method [Cytotechnology, 3, 133 (1990)]. Then, the cells were suspended in 40 mL of a Hybridoma-SFM medium (Life Technologies) containing 10% FBS [hereinafter, described as Hybridoma-SFM-FBS (10)], and dispensed into a 96-well culture plate (Sumitomo Bakelite Co. Ltd.) at 200 μL/well.

The cells were cultured for 24 hours in a 5% $CO_2$ incubator at 37°C, G418 was then added thereto such that the concentration thereof became 1.0 mg/mL, and the cells were cultured for 1 to 2 weeks. The concentration of MTX was gradually increased from 50 nmol/L. In this way, finally, transformant cells were obtained which can grow in the Hybridoma-SFM-FBS (10) medium containing 1.0 mg/mL G418 and 200 nmol/L MTX and produce a large amount of soluble CD16a.

(6) Purification of soluble CD16a

The transformant cells producing the soluble CD16a (V) obtained in (5) of Example 1 were suspended in Hybridoma-SFM-GF (5) [Hybridoma-SFM medium (Life Technologies) containing 5% Daigo's GF21 (Wako Pure Chemical Industries, Ltd.)] containing G418 at 1.0 mg/mL and MTX at 200 nmol/L such that the cell concentration became at $3 \times 10^5$ cells/mL, and 50 mL of the suspension was dispensed into a 182 $cm^2$ flask (Greiner Bio-One GmbH).

The cells were cultured for 4 days in a 5% $CO_2$ incubator at 37°C, and the culture supernatant was collected. From the culture supernatant, the soluble CD16a (V) was purified using a Ni-NTA agarose (QIAGEN) column according to the attached document.

[Example 2] Separation and analysis of component exhibiting high CD16a binding activity from IVIG

(1) Separation of component exhibiting high CD16a binding activity

His-CD16a (0.25 mg) was immobilized in a HiTrap NHS-activated HP column (GE Healthcare) according to the attached document. At this time, unreacted activated ester groups were blocked using 0.5 mol/L Tris-HCl (pH 8.0) as a blocking buffer, and a column in which only blocking was performed and CD16a was not immobilized was used as a control column.

IVIG (100 μL) at 50 mg/ml was added to 2.4 mL of two times diluted serum (final concentration 1 mg/ml), and the components were mixed together by being rotated for 1 hour at room temperature. As competitive antibodies, 2.5 mL of 0.5 mg/mL Xencor-type anti-Gal antibodies were added thereto (final concentration 0.25 mg/mL), and the resultant was used as a load sample.

The load sample (2 mL) was applied to the column containing immobilized CD16a or the control column. An operation of reapplying a flow-through fraction was repeated twice. The columns were washed 7 times with 4 mL (28 ml in total) of 0.1% Tween-containing phosphate buffered saline (PBS) (hereinafter described as PBST), and eluted 3 times using 4 mL of PBS containing 0.5 mol/L NaCl.

(2) Analysis of eluted fraction

(i) Separation by SDS-PAGE

A fraction (1.7 ml) in the firstly eluted fraction was concentrated until the amount became 41 μL, then diluted with a reductive sample buffer containing mercaptoethanol, and heated for 5 minutes at 90°C. After the heating, half of the solution was subjected to electrophoresis by using 12% acrylamide gel (concentrated gel 20 mA 1 hour, separation gel 40 mA 1 hour) and then stained using SYPRO Ruby protein gel stain (Invitrogen).
As a result, a difference was found between this fraction and the control, and bands of sufficient amount of protein necessary for analysis were checked.

(ii) In-gel digestion

The target band was cut out and cut in 1 mm × 1 mm, a reducing buffer [10 mM DTT (Nacalai Tesque, Inc.), 25 mmol/L $NH_4HCO_3$] was added thereto, and reduced for 1 hour at 56°C. The supernatant was removed, an alkylation buffer [5 mmol/L IAA (Nacalai Tesque, Inc.), 25 mmol/L $NH_4HCO_3$] was added thereto, and a reaction was performed for 1 hour at room temperature in a dark place.
The gel pieces were washed with 25 mmol/L $NH_4HCO_3$ and then further washed twice with 25 mmol/L $NH_4HCO_3$ containing 50% acetonitrile (Wako Pure Chemical Industries, Ltd.), and thoroughly dried in a centrifugal concentrator. A trypsin solution [20 μL, a solution obtained by diluting trypsin (Promega Corporation) with 25 mmol/L $NH_4HCO_3$ such that the concentration thereof became 18 ng/20 μL] was added thereto, the solution was left to stand for 30 minutes at 4°C, and then 80 μL of 25 mmol/L $NH_4HCO_3$ was added thereto and the solution was vortexed, and digested with trypsin for 16 hours at 37°C.
The supernatant of the reaction solution was collected into a tube, which contained 5% formic acid containing 50% acetonitrile in an amount 1/100 of the amount of the supernatant, and peptides were extracted from the gel pieces by using water and 5% formic acid containing 50% acetonitrile. The extract was collected, acetonitrile contained in the extract was then removed using a centrifugal concentrator, and the resultant was used as an analysis sample.

(iii) LC-MS/MS

The analysis sample was analyzed by LC-MS/MS (HPLC; Magic 2002, Michrome BioResources Inc., column; MonoCap C18 Nano-flow, 0.2 × 50 mm, GL Sciences Inc., MS; LTQ Orbitrap XL, Thermo Fisher Scientific Inc).
First, for analyzing a band having a molecular weight of 51 kDa (hereinafter, described as 51k-H) corresponding to the H chain of the antibody, the signal intensity of an MS peak of a Xencor-type peptide comprising 332Ile → Glu mutation (m/z = 838. 504, z = 1, ALPAPEEK) or a non-Xencor-type peptide without mutation (m/z = 854. 462, z = 1, ALPAPIEK) was calculated, thereby calculating the proportion of the Xencor-type antibodies comprised in the eluted fraction.
For analyzing the MS data, analysis software Xcalibur Qual Browser (Thermo Fisher Scientific) was used. As a result, the signal intensity of the Xencor-type peptide was $4.07 \times 10^6$ and the signal intensity of the non-Xencor-type peptide was $1.06 \times 10^6$. As a result of comparing the signal intensities, it was considered that the proportion of the Xencor-type antibodies is about 80% and the proportion of the non-Xencor-type antibodies is about 20%.
It was considered that the non-Xencor-type antibodies accounting for about 20% of the eluted fraction are components, such an aggregate contained in IVIG or an immune complex of serum proteins and autoreactive components in IVIG, which bind to CD16a instead of the Xencor-type antibodies and exhibit CD16a binding activity equal to or higher than that of the Xencor-type antibodies.
Then, an attempt was made to identify the serum proteins constituting the immune complex. Seven bands, which showed a difference from control in the separation performed by SDS-PAGE and includes proteins in an amount sufficient for analysis, were analyzed by LC-MS/MS.
The analysis results were analyzed using a search engine MASCOT (Matrix Science) for mass spectrometry analysis, thereby identifying the proteins. As a result, the bands of a high score were considered to be derived from a fragment-polymer of the H chain. From the band having a molecular weight of 43 kDa, the human Hp was identified.

[Example 3] Immunizing human antibody-producing animal

In order to obtain monoclonal antibodies against the human Hp, two human antibody-producing animals were immunized with the human Hp mixed with an adjuvant (Athens Research & Technology, Inc, mixed type) 5 times in total at an interval of 3 to 4 weeks. In order to measure the antibody titer, the binding activity of the antibody to the human Hp and the mouse Hp was analyzed by ELISA approximately on the 10$^{th}$ day after immunization. As a result, both of the animals exhibited binding activity.

[Example 4] Preparation of hybridoma

After the 3rd to 5th immunization, the lymphatic tissue was surgically extracted and used for cell fusion. First, the extracted lymphatic tissue was suspended in the DMEM medium (Invitrogen), and the tissue was ground and washed with the DMEM. The obtained cells were mixed with a mouse myeloma cell line Sp2/0 and washed with the DMEM medium.
The supernatant was removed by centrifugation, and then 1 mL of PEG 1500 (Roche Diagnostics) preheated to 37°C was added thereto by 100 μL every 6 seconds. After the addition, the suspension was mixed for 2 minutes by being warmed by hands.
Thereafter, 4 ml of DMEM medium preheated to 37°C was added thereto for 4 minutes, and then 10 mL of the medium was further added thereto for 1 minute. The suspension was warmed for 5 minutes in a warm bath at 37°C and then subjected to centrifugation at 1,200 rpm for 5 minutes at room temperature. After the centrifugation, the cells were suspended in DMEM medium and cultured by being seeded in a 96-plate well (BD Biosciences). From the next day, the cells were cultured for about 1 week in a drug selective medium.

[Example 5] Hybridoma screening

The binding activity of the hybridoma prepared in Example 4 to the human Hp and the mouse Hp in the culture supernatant was analyzed by enzyme-linked immunosorbent assay (ELISA). First, the human Hp (Athens Research & Technology, Inc, mixed type) or the mouse Hp (Life Diagnotics Inc) dissolved in PBS so as to have a concentration of 10 μg/mL was dispensed in a 96-well plate (MAXISORP NUNC-IMMUNO PLATE, Thermo Fisher Scientific) at 50 μL/well, and the plate was left to stand overnight at 4°C such that the cells were adsorbed onto the plate.
The immobilized solution was removed, PBS containing 1% bovine serum albumin (BSA) (hereinafter, described as BSA-PBS) was then dispensed into the plate at 100 μL/well, and the plate was left to stand 1 hour at room temperature for blocking and washed three times with PBST. Thereafter, the culture supernatant of the hybridoma was dispensed into the plate at 50 μL/well, and the plate was left to stand for 1 hour at room temperature.
The plate was washed three times with PBST, and Goat anti Human IgG (H&L)-HRP (American Qualex Scientific Products.) diluted with 1% BSA-PBS was dispensed into the plate at 50 μL/well, and the plate was left to stand for 1 hour at room temperature.
The plate was washed three times with PBST, and ABTS was dispensed into the plate at 50 μL/well so as to develop color. At a point in time when color was appropriately developed, 1% sodium dodecyl sulfate (SDS) was dispensed into the plate at 50 μL/well, and by using a plate reader, the absorbance at a sample wavelength of 415 nm and a reference wavelength of 490 nm was measured.

[Example 6] Cloning of genes of heavy chain variable region and light chain variable region of antibody

For the hybridomas in wells in which the antibody was found to react with both the human Hp and the mouse Hp, total RNA was prepared using an RNeasy Plus Micro Kit (QIAGEN) according to the attached document. From the obtained total RNA, cDNA was prepared using a SMARTer RACE cDNA amplification kit (Clontech Laboratories, Inc.) according to the attached document.
By using the obtained cDNA as a template, the human IgG heavy chain gene was amplified by PCR by using primers hhiu1 (SEQ ID NO: 7) and hhiu3 (SEQ ID NO: 8), and the human light chain (κ chain) gene was amplified by PCR by using primers hk2 (SEQ ID NO: 9) and hk5 (SEQ ID NO: 10). The amplified genes were subcloned, and the base sequences thereof were analyzed.
As a result, for the clones #4, #6, #27, #105, and #96-6, the base sequences and the amino acid sequences of VH and VL from which the signal sequence was removed were identified. The base sequences of VH and VL of the clone #4 are represented by SEQ ID NOs: 11 and 12, respectively, and the amino acid sequences of VH and VL of the clone #4 are represented by SEQ ID NOs: 21 and 22, respectively. The base sequences of VH and VL of the clone #6 are represented by SEQ ID NOs: 13 and 14, respectively, and the amino acid sequences of VH and VL of the clone #6 are represented by SEQ ID NOs: 23 and 24, respectively. The base sequences of VH and VL of the clone #27 are represented by SEQ ID NOs: 15 and 16, respectively, and the amino acid sequences of VH and VL of the clone #27 are represented by SEQ ID NOs: 25 and 26, respectively. The base sequences of VH and VL of the clone #105 are represented by SEQ ID NOs: 17 and 18, respectively, and the amino acid sequences of VH and VL of the clone #105 are represented by SEQ ID NOs: 27 and 28, respectively. The base sequences of VH and VL of the clone #96-6 are represented by SEQ ID NOs: 19 and 20, respectively, and the amino acid sequences of VH and VL of the clone #96-6 are represented by SEQ ID NOs: 29 and 30, respectively.
Furthermore, the amino acid sequences of CDR1, CDR2, and CDR3 of VH of the clone #27 are represented by SEQ ID NOs: 31, 32, and 33, respectively. The amino acid sequences of CDR1, CDR2, and CDR3 of VL of the clone #27 are represented by SEQ ID NOs: 34, 35, and 36, respectively. The amino acid sequences of CDR1, CDR2, and CDR3 of VH of the clone #105 are represented by SEQ ID NOs: 37, 38, and 39, respectively. The amino acid sequences of CDR1, CDR2, and CDR3 of VL of the clone #105 are represented by SEQ ID NOs: 40, 41, and 42, respectively.

[Example 7] Establishment of recombinant vector for expressing human IgG 1-type anti-Hp antibody

The genes of the heavy chain variable region and the light chain variable region of the anti-Hp antibody determined in Example 6 were connected to the genes of the heavy chain constant region and the κ chain constant region of human IgG1, respectively, thereby preparing a recombinant vector for expressing a human IgG1-type anti-Hp antibody.
As recombinant vectors, a human IgG1 heavy chain recombinant vector pFUSEss-CHIg-hG1 (InvivoGen) and a human IgG1 κ chain recombinant vector pFUSE2ss-CLIg-hk (InvivoGen) were used.
From the transcription product obtained in Example 5, the gene of the heavy chain variable region was inserted into a portion between the sites of the restriction enzymes EcoRI and BsiWI, and the gene of the light chain variable region was inserted into a portion between the sites of the restriction enzymes EcoRI and NheI, by using a CloneEZ (registered trademark) PCR Cloning Kit (GenScript). The vectors were transformed into the E. coli DH5α competent cell (Toyobo Co., Ltd) and cultured, the plasmids were extracted, and the sequence was checked. The recombinant vector for expressing a human IgG1-type anti-Hp antibody was prepared in this way.
For culturing the transformed E. coli, a medium LB Broth (Difco), to which Zeocin (Invitrogen) was added to yield a final concentration of 50 μg/mL, was used for the heavy chain recombinant vector, and Fast-Media Blas TB (InvivoGen) was used for the κ chain recombinant vector.

[Example 8] Transient expression of anti-Hp antibody

In order to prepare a transient expression line of the human IgG1-type anti-Hp antibody, the recombinant vector prepared in Example 7 was introduced into host cells. As the host cells, Freestyle 293F cells (Invitrogen) were used.
Plasmid introduction was performed using 293 Fectin Transfection Reagent (Invitrogen) according to the attached document. As a human IgG1-type anti-Hp antibody recombinant vector, a recombinant vector was used which was obtained by mixing together the heavy chain recombinant vector and the κ chain recombinant vector prepared in Example 7 at a ratio of 2:3. The collected culture supernatant was subjected to centrifugation, and the obtained culture supernatant was filtered through a 0.22 μm filter, thereby preparing a culture supernatant containing human IgG1-type anti-Hp antibodies.

[Example 9] Purification of anti-Hp antibody

The transient expression line of the human IgG1-type anti-Hp antibody obtained in Example 8 was suspended in a Free style 293 expression medium (Invitrogen) and cultured for 6 days in a conical flask, and then the culture supernatant was collected. From the prepared culture supernatant, human IgG1-type anti-Hp antibodies were purified using MabSelect SuRe (GE Healthcare).
First, the culture supernatant was passed through a column, the column was washed with a washing buffer (0.15 mol/L NaCl and 0.2 mol/L sodium borate/pH 7.5), and then elution was performed using an elution buffer (0.1 mol/L sodium citrate/pH 3.5) having a pH of 3.5.
The eluted fractions were promptly neutralized using a neutralization buffer (1 mol/L Tris-HCl/pH 8.5). The absorbance ($A_{280}$) of each of the fractions at 280 nm was measured, and consecutive fractions having a high absorbance were collected as antibody fractions.
By using Econo-Pac 10DG columns (Bio-Rad Laboratories, Inc.), the buffer was exchanged with PBS, and the antibody fractions were passed through a 0.22 μm filter, thereby obtaining purified proteins. The concentration thereof was calculated by setting the absorbance coefficient at 280 nm to be 1.4.

[Example 10] CD16a binding activity (ELISA)

By using the anti-Hp antibodies (#4, #6, #27, #105, and #96-6) prepared in Example 9, the CD16a binding activity of the immune complex of the anti-Hp antibodies and the human Hp was measured by ELISA. Anti-Tetra-His antibody (QIAGEN) dissolved in PBS such that the concentration thereof became 5 μg/mL were dispensed into a 96-well plate (MAXISORP NUNC-IMMUNO PLATE, Thermo Fisher Scientific) at 50 μL/well, and the plate was left to stand overnight at 4°C such that the antibodies were adsorbed onto the plate.
The immobilized solution was removed, 1% BSA-PBS was then dispensed into the plate at 100 μL/well, and the plate was left to stand for 1 hour at room temperature for blocking and washed 5 times with PBST. Then, the soluble CD16a prepared in Example 1 that was diluted with 1% BSA-PBS such that the concentration thereof became 5 μg/mL was dispensed into the plate at 50 μL/well, a reaction was performed for 2 hours at room temperature, and the plate was washed 5 times with PBST.

A mixed solution (hereinafter, also described as IC) of the anti-Hp antibodies and the human Hp was diluted with 1% BSA-PBS and left to stand for 1 hour at room temperature. Regarding the concentration, the anti-Hp antibodies and the human Hp were mixed together such that the molar ratio thereof became 1:2 on the premise that the molecular weight of the anti-Hp antibodies is 150 kDa and the molecular weight of the human Hp is 200 kDa. The mixed solution appropriately diluted with 1% BSA-PBS was dispensed into the plate at 50 μL/well, and a reaction was performed for 1 hour at room temperature.

The plate was washed 5 times with PBST, Goat anti Human IgG (H&L)-HRP (American Qualex Scientific Products.) diluted with 1% BSA-PBS was dispensed into the plate at 50 μL/well, and the plate was left to stand for 1 hour at room temperature.

The plate was washed 5 times with PBST, and ABTS was dispensed into the plate at 50 μL/well so as to develop color. At a point in time when color was appropriately developed, 1% SDS was dispensed into the plate at 50 μL/well, and by using a plate reader, the absorbance at a sample wavelength of 415 nm and a reference wavelength of 490 nm was measured.

As a result, while IVIG had an $EC_{50}$ value of 1.06 μg/mL, the #4 antibody had an $EC_{50}$ value of 1.99 μg/mL, the #6 antibody had an $EC_{50}$ value of 0.805 μg/mL, the #27 antibody had an $EC_{50}$ value of 0.763 μg/mL, the #105 antibody had an $EC_{50}$ value of 0.405 μg/mL, and the #96-6 antibody had an $EC_{50}$ value of 0.473 μg/mL. Therefore, it was revealed that the binding activity of all of these anti-Hp antibodies to CD16a is higher than the binding activity of IVIG to CD16a (Fig. 1A to Fig. IF).

Furthermore, regarding the $EC_{50}$ value obtained in a case where each of these antibodies was mixed with the human Hp, surprisingly, while #4 had an $EC_{50}$ value of 0.298 μg/mL, #6 had an $EC_{50}$ value of 0.0664 μg/mL, #27 had an $EC_{50}$ value of 0.0490 μg/mL, #105 had an $EC_{50}$ value of 0.0540 μg/mL, and #96-6 had an $EC_{50}$ value of 0.0459 μg/mL. This results showed that in a case where the antibodies are mixed with the human Hp, the antibodies form an immune complex together with the human Hp, and hence the binding activity to CD16a is improved (Fig. 1A to Fig. IE).

[Example 11] Preparation of anti-Hp antibodies by stable expression

Anti-Hp antibodies (#4, #6, #27, #105, and #96-6) were prepared by the method described below.

(1) Establishment of recombinant vector for stably expressing anti-Hp antibodies

As a recombinant vector containing the gene of the constant region of human IgG1κ, pDELTA was used. The gene of the heavy chain variable region was inserted into a portion between the sites of the restriction enzymes NotI and ApaI, and the gene of the light chain variable region was inserted into a portion between the sites of the restriction enzymes EcoRI and BsiWI, by using an In-Fusion HD Cloning Kit (Clontech Laboratories, Inc.). In this way, a recombinant vector for stably expressing anti-Hp antibodies was prepared.

The vector was transformed into the E. coli DH5α competent cell and cultured, the plasmids were extracted, and the sequence was checked. In this way, a recombinant vector was prepared. PCR was performed using the recombinant vector for expressing human IgG1-type anti-Hp antibodies prepared in Example 7 as a template and using primers represented by SEQ ID NOs: 43 to 62 as PCR primers.

(2) Preparation of fucosylated antibodies

In order to prepare a stable expression line of fucosylated anti-Hp antibodies, the recombinant vector prepared in (1) of Example 11 was introduced into host cells. As the host cells, CHO-K1 cells were used. The introduction of the gene of the recombinant vector into the cells by the electroporation method, the culture, and the selection of drugs were performed by general methods. While the cells were being cultured for 1 week, the drug-containing medium was replaced once, and a line in which the proportion of living cells was restored to about 98% was used as a stable expression bulk line.

The stable expression line was suspended in a medium such as EX-CELL 325 PF CHO Serum-Free Medium for CHO Cells (Sigma-Aldrich Co. LLC.) containing 8 mmol/L Gln and 50 μg/mL gentamycin (Nacalai Tesque, Inc.) at $3 \times 10^5$ cells/mL and cultured for 14 days, and then the culture supernatant was collected. From the culture supernatant, anti-Hp antibodies were purified by the method described in Example 9.

(3) Preparation of defucosylated antibodies

In order to prepare a stable expression line of defucosylated anti-Hp antibodies, the recombinant vector prepared in (1) of Example 11 was introduced into host cells. As the host cells, FUT8 knockout CHO cells (International Publication No. 2005/035586 and International Publication No. 02/31140) were used, and then the operation to be carried out thereafter was performed based on the method described in (2) of Example 11.

(4) Preparation of DNP antibody as negative control antibody

Dinitrophenylhydrazine (DNP) antibody as negative control antibody was prepared based on a known method (Motoki K et. al., Clin. Cancer Res. 11, 3126-3135, 2005).

[Example 12] Preparation of Fc amino acid-modified anti-Hp antibodies

Synthetic DNA (GENEWIZ) of the gene sequence (SEQ ID NO: 63) of the Fc domain of human IgG1 in which amino acids were modified (S239D and I332E) (hereinafter, abbreviated to hCg1 Xen 239 332 in some cases) was inserted into a portion between the sites of restriction enzymes NheI and BamHI in the recombinant vector prepared in (1) of Example 11, thereby preparing a recombinant vector. The recombinant vector can also be prepared using ApaI instead of NheI.

The operation to be carried out thereafter was performed based on the method described in (3) of Example 11, thereby preparing defucosylated Fc amino acid-modified anti-Hp antibodies. By the same method, defucosylated Fc amino acid-modified anti-Hp antibodies were prepared in which amino acids at three sites (S239D, S298A, and I332E) of the Fc domain were modified.

[Example 13] Antigen binding activity of anti-Hp antibodies

By using the anti-Hp antibodies (#4, #6, #27, #105, and #96-6) prepared in Example 9, the binding activity of the anti-Hp antibodies to the human Hp was analyzed by ELISA. First, the human Hp (Japan Blood Products Organization) dissolved in PBS such that the concentration thereof became 5 μg/mL was dispensed into a 96-well plate (MAXISORP NUNC-IMMUNO PLATE, Thermo Fisher Scientific) at 50 μL/well, and the plate was left to stand overnight at 4°C such that the human Hp was adsorbed onto the plate.

The immobilized solution was removed, 1% BSA-PBS was then dispensed into the plate at 100 μL/well, the plate was left to stand for 1 hour at room temperature for blocking, and the plate was washed 3 times with PBST. Then, the anti-Hp antibodies diluted with 1% BSA-PBS so as to have an appropriate concentration were dispensed into the plate at 50 μL/well, and the plate was left to stand for 1 hour at room temperature. The operation to be carried out thereafter was performed based on the method described in Example 5, except for the measurement of absorbance (sample wavelength 405 nm) performed using a plate reader.

As a result, all of 4 antibodies of #6, #27, #105, and #96-6 exhibited high binding activity to the human Hp (Fig. 2). #4 also exhibited binding activity equivalent to that of these 4 antibodies. Then, Western blotting was performed to analyze to which one the anti-Hp antibodies (#4, #6, #27, #105, and #96-6) prepared in Example 9 exhibit binding activity between the α chain and the β chain of the human Hp. First, the human Hp prepared using a reductive sample buffer containing mercaptoethanol was separated by SDS-PAGE electrophoresis and then transferred to a PVDF membrane by blotting. The separated human Hp was reacted with each of the anti-Hp antibodies, and then the antibodies having bound to the separated human Hp were detected using Goat anti Human IgG (H&L)-HRP (American Qualex Scientific Products.).

As a result, all of 5 antibodies of #4, #6, #27, #105, and #96-6 exhibited specific binding activity to the β chain. From the above result, it was understood that all of 5 antibodies of #4, #6, #27, #105, and #96-6 are antibodies which specifically recognize the β chain and bind to the human Hp.

[Example 14] Analysis of molecular weight of immune complex of anti-Hp antibody and human Hp

In order to check whether the anti-Hp antibodies have bound to the human Hp and formed a immune complex, analysis was performed by gel filtration chromatography (Size Exclusion Chromatography, SEC) (column; BioSep SEC-s4000, 7.8 × 300 mm, Phenomenex Inc.) using HPLC (Prominence, Shimadzu Corporation).

The column was equilibrated using a mobile phase [50 mmol/L phosphate buffer (pH 7.0, 500 mmol/L NaCl)], the anti-Hp antibodies and the human Hp (Japan Blood Products Organization) were diluted with PBS and mixed together such that the concentrations of the anti-Hp antibodies and the human Hp became 0.5 mg/mL and 1.3 mg/mL, respectively, and the mixed solution was left to stand for 16 hours at 37°C.

The components contained in 50 μL of the mixed solution were separated at a flow rate of 0.5 mL/min. Then, for each peak detected at a wavelength of 280 nm, a molecular weight was calculated using a maximum value of scattering intensity detected by a multi angle light scattering (MALS) detector (miniDAWN TREOS, Wyatt Technology Corporation). The results are shown in Table 1.

[Table 1]

| Sample name | Peak molecular weight of immune complex |
|---|---|
| #4 | $341 \times 10^3$ |
| #6 | $771 \times 10^3$ |
| | $1344 \times 10^3$ |
| #27 | $523 \times 10^3$ |
| #105 | $703 \times 10^3$ |
| | $1180 \times 10^3$ |
| #96-6 | $737 \times 10^3$ |
| | $6776 \times 10^3$ |

As a result, in a case where the mixed solution was analyzed, a peak of higher molecular weight at a faster elution time was detected than in a case where the anti-Hp antibodies or the human Hp were separately analyzed, and the peak showed that immune complexes formed of the anti-Hp antibodies and the human Hp are formed.

As described above, Hp can have a plurality of structures of different molecular weights depending on the number of $\alpha/\beta$-subunits comprised in Hp. Through the molecular weight analysis by MALS, the molecular weight of the human Hp was found to be $206 \times 10^3$ by calculation. Accordingly, the main component of the human Hp used herein was assumed to comprise 4 $\alpha/\beta$-subunits [J Sep Sci., 32, 1224 (2009)].

Furthermore, as shown in Table 1, the molecular weight of the immune complex formed of each of #6, #27, #105, and #96-6 was larger than the molecular weight of the immune complex formed of #4.

In addition, the number of antibody molecules and the human Hp molecules comprised in the immune complex were estimated by regarding the molecular weight of the antibodies as $150 \times 10^3$. As a result, the immune complex formed of #4 was assumed to comprise one antibody molecule and one human Hp molecule. In contrast, the immune complex formed of #27 was assumed to comprise two antibody molecules and one human Hp molecule, and the immune complex formed of each of #6, #105, and #96-6 was assumed to comprise two antibody molecules and two or more human Hp molecules.

[Example 15] CD32a binding activity (ELISA)

(1) Preparation of soluble CD32a

Based on the method described in Example 1, histidine tag-conjugated soluble CD32a was prepared. The amino acid sequence thereof is represented by SEQ ID NO: 64.

(2) CD32a binding activity of immune complex of anti-Hp antibody and human Hp

By using the soluble CD32a prepared in (1) of Example 15, CD32a binding activity of an immune complex of the anti-Hp antibody and the human Hp was analyzed in the same manner as in Example 10.

As a result, among the antibodies, which formed the immune complex together with the human Hp by being mixed with the human Hp in Example 10 and were found to be exhibit improved binding activity to CD16a, 4 antibodies (#6, #27, #105, and #96-6) were found to exhibit improved binding activity to CD32a by being mixed with the human Hp in the analysis of the binding activity to CD32a. In contrast, the binding activity of #4 was not improved (Fig. 3).

[Example 16] Inhibitory activity to binding of CD16a to anti-DNP antibody

(1) Establishment of recombinant vector of CD16a

The plasmid pBs CD16a (V) (5.0 $\mu$g), which was prepared in (2) of Example 1 and comprised cDNA encoding full-length CD16a (V) (SEQ ID NO: 4), was digested using restriction enzymes NotI (TAKARA BIO INC.) and BamHI (TAKARA BIO INC.) and then subjected to 1.5% agarose gel electrophoresis, thereby collecting a DNA fragment of about 800 bp.

Meanwhile, 10 μg of the plasmid pKANTEX 93 described in International Publication No. 97/10354 was digested using restriction enzymes NotI I (TAKARA BIO INC.) and BamHII (TAKARA BIO INC.) and then subjected to 1.5% agarose gel electrophoresis, thereby collecting a DNA fragment of about 11.7 kbp. The DNA fragment of about 800 bp and the DNA fragment of about 11.7 kbp were ligated by using a DNA Ligation Kit Ver. 2.0 (TAKARA BIO INC.) and transformed into an E. coli DH5α strain (Toyobo Co., Ltd), thereby obtaining a recombinant vector pKANTEX CD16a (V) encoding full-length CD16a (V).

(2) Preparation of CD16a expression cell

The recombinant vector pKANTEX CD16a (V) encoding full-length CD16a (V) established in (1) of Example 16 was introduced into a DHFR gene-deleted CHO cell DG44 line (CHO/DG44 cells), and cells stably producing CD16a were prepared as below.

The gene of pKANTEX CD16a (V) was introduced into the CHO/DG44 cells by an electroporation method. The concentration of MTX was gradually increased by the same method as that described in (5) in Example 1. In this way, finally, transformant cells were obtained which can grow in IMDM (GIBCO) containing 0.5 mg/mL G418, 500 nmol/L MTX, and 10% FBS (GIBCO) and highly express CD16a.

The obtained transformant cells were exfoliated using 0.02% EDTA solution (Nacalai Tesque, Inc.), washed with phosphate buffered saline (PBS), and then suspended in a buffer 1 for FCM (PBS containing 2% FBS, 0.05% $NaN_3$, and 1 mmol/L EDTA).

Then, the cells were seeded in a 96-well plate with a U-bottom shape (Falcon) at $2 \times 10^5$ cells/well, stained with PE-labeled anti-human CD16 antibody (BD Biosciences), washed, and then suspended in the buffer 1 for FCM. The fluorescence intensity thereof was analyzed using a flow cytometer (BD Biosciences, FACS CantoII) so as to check whether CD16a was highly expressed.

(3) Inhibitory activity to binding of CD16a to anti-DNP antibody

By using the anti-Hp antibodies (fucosylated type and defucosylated type) and the immune complex formed of the defucosylated anti-Hp antibodies and the human Hp prepared in Example 11, and IVIG, the inhibitory activity to the binding of CD16a to the anti-DNP antibody was detected.

The CD16a expression cells prepared in (2) of Example 16 were seeded in a 96-well plate at $1.2 \times 10^5$ cells/well. Then, the anti-Hp antibodies (a fucosylated type and a defucosylated type) prepared in Example 11, the immune complex formed of the defucosylated anti-Hp antibodies and the human Hp and IVIG were diluted with the buffer 1 for FCM, and then the plate was left to stand for 1 hour at room temperature, thereby obtaining a test subject.

The immune complex formed of the defucosylated anti-Hp antibodies and the human Hp was prepared such that the molar ratio between the anti-Hp antibodies and the human Hp became 1:2 on the premise that the molecular weight of the anti-Hp antibodies is 150 kDa and the molecular weight of the human Hp is 200 kDa. The test subject was added to the aforementioned plate, in which the cells were seeded, at 10 μL/well and allowed to react for 5 minutes at 4°C.

In addition, the anti-DNP antibody prepared in (4) of Example 11 were mixed with 2-4-dinitrophenol (DNP)-BSA Protein Biotin Conjugate (Alpha Diagnostics International Inc.) such that the concentration of the antibody became 0.05 mg/mL, and the solution was left to stand for 1 hour at room temperature, thereby obtaining an anti-DNP antibody solution.

As competitive antibody, the anti-DNP antibody solution was added at 10 μL/well to the aforementioned plate, to which the test subject was added and allowed to react for 5 minutes at 4°C, and allowed to react for 3 hours at 4°C. The plate was washed with the buffer 1 for FCM, and then the anti-DNP antibody having bound to CD16a was detected using streptavidin and an Alexa Flour 647 conjugate (Molecular probes) so as to investigate the inhibitory activity to the binding of CD16a.

As a result, the $IC_{50}$ values of the #27 fucosylated antibody and the #27 defucosylated antibody were 238 μg/mL and 53.8 μg/mL, respectively, which showed that the inhibitory activity is further improved in a case where the antibodies are made into defucosylated antibodies.

Furthermore, surprisingly, in a case where an immune complex is formed by mixing the human Hp with the #27 defucosylated antibody or the #105 defucosylated antibody, the $IC_{50}$ values of the immune complexes became 11.7 μg/mL and 7.92 μg/mL, respectively, which showed that higher binding inhibitory activity is obtained by the formation of the immune complex. The $IC_{50}$ value of IVIG was 540 μg/mL.

As is evident from the above results, the inhibitory activity of #27 or #105 to the binding of CD16a to the anti-DNP antibody is prominently improved in a case where each of #27 and #105 is modified into a defucosylated antibody and forms an immune complex together with the human Hp (Fig. 4).

[Example 17] Inhibitory activity to ADCC reaction using PBMC

The inhibitory activity of the anti-Hp antibodies and the anti-Hp antibodies forming an immune complex to the ADCC reaction using PBMC was investigated. The ADCC activity was measured based on the method described in International Publication No. 2007/011041.

Her2-positive human breast cancer cells SK-BR-3 were used as target cells, and frozen PBMC (Lonza) derived from a healthy human donor was used as effector cells. The target cells were opsonized using commercial 100 ng/mL anti-Her2 antibody Herceptin. The experiment was performed by setting the ratio between the effector cells and the target cells to be 1:20.

First, the anti-Hp antibodies (a fucosylated type and a defucosylated type) prepared in Example 11, the immune complex formed of the defucosylated anti-Hp antibodies and the human Hp, and IVIG were diluted with RPMI 1640 containing 5% FBS and then left to stand for 1 hour at room temperature, thereby obtaining a test subject. The immune complex of the defucosylated anti-Hp antibodies and the human Hp was prepared such that the molar ratio between the anti-Hp antibodies and the human Hp became 1:2 on the premise that the molecular weight of the anti-Hp antibodies is 150 kDa and the molecular weight of the human Hp is 200 kDa.

Then, Herceptin, the test subject, the effector cells, and the target cells were added in this order to a 96-well plate with a U-bottom shape (Falcon) and allowed to react for 4 hours at 37°C (5% $CO_2$). After the reaction, the plate was subjected to centrifugation, and the activity of lactate dehydrogenase (LDH) in the supernatant was measured using LDH-Cytotoxic Test (Wako Pure Chemical Industries, Ltd.) according to the attached instruction. ADCC (%) was calculated by the following formula.

$$\text{ADCC (\%)} = 100 \times (S - Ab)/(Max - T)$$

S = absorbance of well in which sample reacts - absorbance of well containing medium
Ab = absorbance of antibody-free well - absorbance of well containing medium
T = absorbance of target well - absorbance of well containing medium
Max = absorbance of well in which reaction rate is 100% - absorbance of control well in which reaction rate is 100%

As a result, in a case where the #27 defucosylated anti-Hp antibody or the #105 defucosylated anti-Hp antibody was mixed with the human Hp so as to form an immune complex, respectively, the immune complex exhibited action of inhibiting the ADCC activity from a lower concentration compared to that of IVIG or the #27 defucosylated anti-Hp antibody. From this result, it was revealed that the ADCC activity is prominently reduced by the formation of the immune complex (Fig. 5). By the same experiments, it was confirmed that #6 and #96-6 anti-Hp antibodies also exhibit high inhibitory activity.

[Example 18] Inhibitory activity to ADCC reaction using peripheral blood from healthy human donor

(1) Preparation of defucosylated rituximab

A defucosylated anti-CD20 antibody rituximab (US Patent No. 5,736,137) was prepared based on a known method (Masuda K et. al., Mol. Immunol. 44, 3122-3131, 2007).

(2) Inhibitory activity to ADCC reaction using peripheral blood from healthy human donor

The inhibitory activity of anti-Hp antibodies to the ADCC reaction using the peripheral blood from a healthy human donor was investigated. The ADCC reaction using the peripheral blood from a healthy human donor was measured based on a known method (Masuda K et. al., Mol. Immunol. 44, 3122-3131, 2007).

First, the anti-Hp antibodies and the anti-DNP antibody prepared in Examples 11 and 12, various antibodies obtained by modifying these antibodies, and IVIG were diluted with RPMI 1640 such that the concentration thereof became 15 times the final concentration, thereby obtaining a test subject.

Then, 30 μL of the test subject and 390 μL of the peripheral blood from a healthy human donor were dispensed into the wells of a 48-well plate (Greiner Bio-One GmbH) and cultured for 30 minutes at 37°C (5% $CO_2$). Then, the defucosylated rituximab prepared in (1) was diluted with RPMI 1640 such that the final concentration thereof became 1 μg/mL and dispensed into the wells in an amount of 30 μL.

Instead of the defucosylated rituximab, RPMI 1640 was dispensed into the well in which the ADCC reaction did not occur. Instead of the test subject, RPMI 1640 was dispensed into the well in which the ADCC reaction occurred by the defucosylated rituximab in the absence of the test subject. The 48-well plate was cultured for 20 minutes at 37°C

(5% $CO_2$), and then the number of B cells was counted.

Specifically, 150 μL of the reaction solution was collected from each of the wells, CountBright Absolute Counting Beads, for flow cytometry (Molecular Probes) were added thereto at 30 μL/sample, and then a hemolysis operation was performed using a solution for hemolysis [solution obtained by diluting 10× RBC Lysis buffer (eBioscience) with sterile water at 10 times] according to the attached document.

After centrifugation, the supernatant was discarded, and the cells obtained as a residue were moved to a 96-well plate with a U-bottom shape (Falcon) and suspended in 75 μL of a buffer 2 for FCM (1% BSA-PBS containing 0.05% $NaN_3$ and 1 mmol/L EDTA).

An FcR Blocking Reagent, human (Milteniy Biotec) was dispensed into the plate at 5 μL/well, and the plate was left to stand for 5 minutes at 4°C. Then, APC Mouse Anti-Human CD19 (BD Biosciences) and PE Mouse Anti-Human CD2 (BD Biosciences) diluted with the buffer 2 for FCM were added thereto and allowed to react for 30 minutes or longer at 4°C.

Dead cells were stained using a LIVE/DEAD Fixable Violet Dead Cell Stain Kit, for 405 nm excitation (Molecular Probes) according to the attached instruction. The cells were washed again and then suspended in the buffer 2 for FCM, and the fluorescence intensity thereof was analyzed using a flow cytometry (BD Biosciences, FACS CantoII). B cells were detected as LIVE-/DEAD-negative cells, CD19-positive cells, and a CD2-negative fraction in lymphocyte fractions in the FSC-SSC plot.

The ADCC activity was evaluated by either a method of analyzing the number of B cells per 2,400 CountBright Absolute Counting Beads or a method of analyzing the proportion of the LIVE-/DEAD-negative cells and the CD19-positive cells in the lymphocyte fraction.

As a result, it was revealed that the #27 defucosylated antibody and the #105 defucosylated antibody exhibit higher inhibitory activity to the ADCC reaction compared to the defucosylated anti-DNP antibody in which the Fc domains have the same structure. The inhibitory activity was prominently higher than that of IVIG. Furthermore, the inhibitory activity of the defucosylated anti-Hp antibodies, in which the Fc amino acids were modified (S239D and I332E), was much higher (Fig. 6A).

It is considered that unlike the anti-Hp antibody of the present invention, the anti-DNP antibody is present in the peripheral blood in the form of a monomer without a ligand. Accordingly, the above results show that the anti-Hp antibody of the present invention can exhibit high inhibitory activity by forming an immune complex together with the human Hp in the blood.

From the above results, it was revealed that the inhibitory activity to the ADCC reaction can be markedly improved by combining the Fc amino acid modification with the formation of an immune complex and the Potelligent technology (Fig. 6A).

The #27 defucosylated antibody in which the Fc amino acids were modified (S239D, S298A, and I332E) exhibited high inhibitory activity as in the experiment of Fig. 6A. From this result, it was revealed that the inhibitory activity against the ADCC reaction is also markedly improved by the modification of the Fc amino acids (S239D, S298A, and I332E).

In contrast, the #4 defucosylated antibody in which the Fc amino acids were modified (S239D, S298A, and I332E) exhibited low inhibitory activity. From this result, it was revealed that the potency of the inhibitory activity varies with the clone of the anti-Hp antibodies. Considering the result obtained in Example 14 in combination with this result, it was considered that in order to obtain high inhibitory activity, it is important for the immune complex of the human Hp and the anti-Hp antibodies to contain two or more antibodies (Fig. 6B).

[Example 19] Effect on binding of the human Hp to anti-Hp antibody

(1) Preparation of enzyme-labeled antibody

The anti-Hp antibodies (#6, #105, and #96-6) prepared in (2) of Example 11 and anti-Hp rabbit polyclonal antibody (Rockland Immunochemicals, Inc.) (hereinafter, described as anti-Hp polyclonal antibody) were labeled using a Peroxidase Labeling Kit-$NH_2$ (Dojindo Molecular Technologies, Inc.) based on the attached instruction. The solvent containing each of the anti-Hp antibodies in an amount of 200 μg was substituted with a washing buffer by using a filtration tube, $NH_2$-reactive peroxidase was then added thereto, and the solution was allowed to react for 2 hours at 37°C. Thereafter, the solvent of the labeled antibodies was substituted again with the washing buffer.

(2) Effect on binding of the human Hp to obtained anti-Hp antibody

The effect of #27 and #105 exerted on the binding of the labeled anti-Hp antibodies (#6, #105, and #96-6) prepared in (1) of Example 19 to the human Hp was evaluated. The human Hp dissolved in PBS such that the concentration thereof became 5 μg/mL was dispensed in a 96-well plate (MAXISORP NUNC-IMMUNO PLATE, Thermo Fisher Scientific) at 50 μL/well, and the plate was left to stand overnight at 4°C such that the antibodies were adsorbed onto the plate.

The immobilized solution was removed, 1% BSA-PBS was then dispensed into the plate at 100 μL/well, and the plate was left to stand for 1 hour at room temperature for blocking and washed 3 times with PBST. Then, the anti-Hp antibodies (#27, #105, #6, or #96-6) diluted with 1% BSA-PBS such that the concentration thereof became 20 μg/mL, or, the anti-DNP antibody (prepared in (4) of Example 11) or the anti-Hp polyclonal antibody as control antibodies were dispensed into the plate at 50 μL/well, and the plate was left to stand for 10 minutes at room temperature.

Thereafter, the labeled anti-Hp antibodies (#6, #105, or #96-6) [prepared in (1) of Example 19] diluted with 1% BSA-PBS were dispensed into the plate at 50 μL/well, and the plate was left to stand for 1 hour at room temperature. The plate was washed 3 times of PBST, and ABTS was dispensed into the plate at 50 μL/well for developing color. At a point in time when color was appropriately developed, 1% SDS was dispensed into the plate at 50 μL/well, and by using a plate reader, the absorbance at a sample wavelength of 415 nm and a reference wavelength of 490 nm was measured.

The results are shown in Table 2. In Table 2, + shows that the absorbance is equal to or higher than 0.5, and - shows that the absorbance is less than 0.5. The addition of the anti-DNP antibody did not inhibit the binding of the human Hp to the labeled anti-Hp antibodies (#6, #105, or #96-6). In contrast, the addition of unlabeled anti-Hp antibodies and the addition of the anti-Hp polyclonal antibody inhibited the binding of the human Hp to the anti-Hp antibodies. Therefore, it was confirmed in advance that whether there is competition can be evaluated by the present evaluation system.

[Table 2]

| | #27 | #105 | #6 | #96-6 | Anti-DNP antibody | Anti-Hp polyclonal antibody |
|---|---|---|---|---|---|---|
| Labeled #6 | + | + | - | + | + | - |
| Labeled #105 | + | - | + | + | + | - |
| Labeled #96-6 | + | + | + | - | + | - |

As shown in Table 2, the addition of #27 did not inhibit the binding of the human Hp to the labeled anti-Hp antibodies (#6, #105, or #96-6). This result shows that the epitope of the human Hp that #27 recognizes is different from epitope that #6, #105, and #96-6 recognize. Furthermore, the addition of #105 did not inhibit the binding of the human Hp to #6 or #96-6. This result shows that the epitope that #105 recognizes is different not only from the epitope that #27 recognizes but also from the epitope that #6 and #96-6 recognize.

(3) Effect on binding of human Hp to commercial anti-Hp antibody

(i) Binding properties between human Hp and commercial anti-Hp antibody

By using 3 clones of commercial anti-Hp mouse antibodies, the binding properties to the human Hp was evaluated. First, the human Hp was immobilized in a 96-well plate based on the method described in (2) of Example 19, and blocking was performed. After the blocking, commercial anti-Hp antibody 2B11 (LifeSpan BioSciences, Inc.) (hereinafter, described as commercial 2B11 antibody) diluted with 1% BSA-PBS such that the concentration thereof became 1 μg/mL, 2F4 (Santa Cruz Biotechnology) (hereinafter, described as commercial 2F4 antibody) or F8 (Santa Cruz Biotechnology) (hereinafter, described as commercial F8 antibodies), and mouse IgG1-type isotype control antibody or labeled anti-Hp polyclonal antibody [prepared in (1) of Example 19] as control antibodies were dispensed into the plate at 50 μL/well, and the plate was left to stand for 1 hour at room temperature.

The plate was washed 3 times with PBST, Rabbit anti-Mouse IgG-HRP (Dako) diluted with 1% BSA-PBS was then dispensed into the plate at 50 μL/well, and the plate was left to stand for 1 hour at room temperature. The color development and the absorbance measurement to be carried out thereafter were performed based on the method described in Example 5. As a result, the commercial 2B11 antibody and the commercial 2F4 antibody exhibited binding properties to the human Hp, but the commercial F8 antibody substantially did not exhibit binding properties to the human Hp. Based on this result, other items were evaluated using the commercial 2B11 antibody and the commercial 2F4 antibody.

(ii) Binding properties of bovine Hp or mouse Hp to commercial anti-Hp antibody

The binding properties of the commercial 2B11 antibody and the commercial 2F4 antibody to bovine Hp (Life Diagnostics, Inc.) and mouse Hp were compared with the binding properties of #27 and #105 to bovine Hp and mouse Hp. First, based on the method described in (2) of Example 19, the human Hp, bovine Hp, or mouse Hp was immobilized in a 96-well plate, and blocking was performed.

After the blocking, the anti-Hp antibodies (#27 or #105) diluted with 1% BSA-PBS such that the concentration thereof became 1 μg/mL and the commercial anti-Hp antibodies (the commercial 2B11 antibody or the commercial 2F4 antibody) or control antibodies (anti-DNP antibody, mouse IgG1-type isotype control antibody, or labeled anti-Hp polyclonal antibody) were dispensed into the plate at 50 μL/well, and the plate was left to stand for 1 hour at room temperature.

The plate was washed 3 times with PBST, and Goat anti Human IgG (H&L)-HRP or Rabbit anti-Mouse IgG-HRP diluted with 1% BSA-PBS was dispensed into the plate at 50 μL/well, and the plate was left to stand for 1 hour at room temperature. The plate was washed 3 times with PBST, and TMB (Dako) was dispensed into the plate at 50 μL/well for developing color. At the point in time when color was appropriately developed, 0.5 mol/L $H_2SO_4$ (Wako Pure Chemical Industries, Ltd.) was dispensed into the plate at 50 μL/well, and the absorbance at 450 nm was measured.

The results are shown in Table 3. For the absorbance of the well in which the anti-Hp antibodies (#27 or #105) were reacted, a value obtained by subtracting the absorbance of the well, in which the anti-DNP antibody were reacted, from the aforementioned absorbance was determined. Furthermore, for the absorbance of the well in which the commercial anti-Hp antibodies (the commercial 2B11 antibody or the commercial 2F4 antibody) were reacted, a value obtained by subtracting the absorbance of the well, in which the mouse IgG1-type isotype control antibodies were reacted, from the aforementioned absorbance was determined. In the table, + shows that the determined value is equal to or greater than 1, and - shows that the determined value is less than 1. From the binding of the anti-Hp polyclonal antibody to the human Hp, bovine Hp, and mouse Hp, it was confirmed in advance that the antigens are immobilized.

[Table 3]

| | Human Hp | Bovine Hp | Mouse Hp |
|---|---|---|---|
| #27 | + | - | + |
| #105 | + | - | + |
| Commercial 2B11 antibody | + | + | + |
| Commercial 2F4 antibody | + | - | - |
| Anti-Hp polyclonal antibody | + | + | + |

As shown in Table 3, while #27, #105, and the commercial 2F4 antibody did not bind to the bovine Hp, the commercial 2B11 antibody also bound to the bovine Hp. This result shows that while the commercial 2B11 antibody bind to the homologous amino acid residues shared by a human being and a bovine, #27, #105, and the commercial 2F4 antibody bind to the nonhomologous amino acid residues not being shared by a human being and a bovine. Therefore, it was revealed that #27 and #105 recognize an epitope different from the epitope that the commercial 2B11 antibody recognize.

In addition, while #27 and #105 bound to the mouse Hp, the commercial 2F4 antibody did not bind to the mouse Hp. From this result, it was revealed that just like the commercial 2F4 antibody, #27 and #105 recognize the non-identical amino acid residues shared by a human being and a bovine. Furthermore, while #27 and #105 were found to bind to the identical amino acid residues shared by a human being and a mouse, the commercial 2F4 antibody were found to bind to the nonhomologous amino acid residues not being shared by a human being and a mouse. Therefore, it was revealed that #27 and #105 recognize an epitope different from the epitope that the commercial 2F4 antibody recognize.

(iii) Competitive ELISA

For the purpose of evaluating whether #27 and #105 recognize an epitope different from the epitope that the commercial 2F4 antibody recognize by another method, the effect of #27 and #105 exerted on the binding of the commercial 2F4 antibody to the human Hp was evaluated. Based on the method described in (1) of Example 19, the human Hp was immobilized in a 96-well plate, and blocking was performed. Thereafter, the anti-Hp antibodies (#27 or #105) diluted with 1% BSA-PBS such that the concentration thereof became 20 μg/mL or the anti-DNP antibody or anti-Hp polyclonal antibody as control antibody were dispensed into the plate at 50 μL/well, and the plate was left to stand for 10 minutes at room temperature.

Subsequently, the commercial 2F4 antibody diluted 500 times with 1% BSA-PBS were dispensed into the plate at 50 μL/well, and the plate was left to stand for 1 hour at room temperature. The plate was washed 3 times with PBST, Rabbit anti-mouse IgG HRP (Dako) diluted with 1% BSA-PBS was then dispensed into the plate at 50 μL/well, and the plate was left to stand for 1 hour at room temperature. The color development and the absorbance measurement to be carried out thereafter were performed based on the method described in Example 5.

The results are shown in Table 4. In Table 4, + shows that the absorbance is equal to or higher than 0.5, and - shows that the absorbance is less than 0.5. While the addition of the anti-DNP antibody did not inhibit the binding of the commercial 2F4 antibody to the human Hp, the addition of the anti-Hp polyclonal antibodies inhibited the binding of the commercial 2F4 antibody to the human Hp. From this result, it was confirmed in advance that whether there is competition can be evaluated by the present evaluation system.

[Table 4]

| | #27 | #105 | Anti-DNP antibody | Anti-Hp polyclonal antibody |
|---|---|---|---|---|
| Commercial 2F4 antibody | + | + | + | - |

As shown in Table 4, the binding of the commercial 2F4 antibody to the human Hp is not inhibited by the addition of #27 or #105, and #27 and #105 recognized an epitope different from the epitope that the commercial 2F4 antibody recognize as in (ii) of (3) of Example 19.

[Example 20] Epitope analysis for obtained anti-Hp antibody

(1) Preparation of human Hp/bovine Hp chimeric protein

As described above in Example 13, it was indicated that #27 and #105 recognize the β chain of the human Hp. For the purpose of clarifying which amino acids in the β chain of the human Hp are recognized by #27 and #105 and comparing the epitope of the antibodies with the epitope of the commercial 2F4 antibody, epitope analysis was performed.

As described above in (ii) of (3) of Example 19, #27 and #105 bound not to the bovine Hp but to the mouse Hp. Based on this result, a plurality of chimeric Hpβ chains were designed by substituting some of the amino acid residues in the human Hpβ chain (SEQ ID NO: 70) with bovine amino acid residues. Specifically, the amino acid sequence (SEQ ID NO: 65) of the human Hp, the amino acid sequence (SEQ ID NO: 73) of the bovine Hp, and the amino acid sequence (SEQ ID NO: 74) of the mouse Hp were compared with each other, and from the human Hpβ chain (SEQ ID NO: 70), the amino acid residues that do not exhibit homology between a human being and a bovine but exhibit homology between a human being and a mouse were extracted. Furthermore, the extracted residues were substituted with the corresponding amino acid residues of the bovine Hp.

A recombinant vector for expressing the chimeric Hpβ chain was designed such that the human Hpα chain (SEQ ID NO: 71), the chimeric Hpβ chain, and histidine tag were encoded in this order from the 5'-terminal. In this way, the chimeric Hpβ chain is expressed in a manner in which histidine tag is linked to the C-terminal side and the human Hpα chain is linked by a disulfide bond (hereinafter, described as chimeras A to F).

Table 5 shows the positions and types of the substituted amino acid residues in the prepared chimeras A to F. The amino acid residues of the prepared chimeras A to F are represented by SEQ ID NOs: 75 to 80, and the base sequences of synthetic DNAs (FASMAC or GENEWIZ) comprising base sequences encoding the amino acid sequences of the chimeras A to F are represented by SEQ ID NOs: 81 to 86.

Recombinant vectors for expressing the chimeras A to F were prepared by inserting the synthetic DNAs (SEQ ID NOs: 81 to 86) into a portion between the sites of restriction enzymes EcoRI and SalI in a pCI vector by using an In-Fusion HD cloning Kit. Furthermore, for the Hpβ chain (SEQ ID NO: 70) in which amino acid substitution was not performed, a recombinant vector for expression was prepared in the same manner as that used for the chimeric proteins.

The amino acid sequence of the prepared recombinant human Hp is represented by SEQ ID NO: 87, and the sequence of the synthetic DNA is represented by the SEQ ID NO: 88. By using an ExpiFectamin 293 Transfection Kit (GIBCO), the chimeras A to F and the recombinant human Hp were prepared by transient expression according to the instruction.

As host cells, Expi293F (Life Technologies) was used. As a culture medium, an Expi293 Expression Medium (GIBCO) was used. From the collected culture supernatant, the chimeras A to F and the recombinant human Hp were purified using a cOmplete His-Tag Purification Resin (Hoffman-La Roche Ltd) according to the attached instruction.

[Table 5]

| Name of chimera protein | Substitution |
|---|---|
| Chimera A | S155V/T156D/V157A |
| Chimera B | Q34R/Q239R |
| Chimera C | T25I/D133E |

(continued)

| Name of chimera protein | Substitution |
|---|---|
| Chimera D | R100K/D133E |
| Chimera E | Q34R/K92R |
| Chimera F | F44Y/E49S |

(2) Binding properties to human Hp/bovine Hp chimeric protein

The binding properties of #27 and #105 to the chimeras A to F prepared in (1) of Example 20 were evaluated. The chimeras A to F, the recombinant human Hp and bovine Hp were immobilized in a 96-well plate based on the method described in Example 5, and blocking was performed. After the blocking, the anti-Hp antibodies (#27 or #105) diluted with 1% BSA-PBS, the commercial 2F4 antibody, or control antibodies(the anti-DNP antibody, the mouse IgG1-type isotype control antibody, or the labeled anti-Hp polyclonal antibody) were dispensed into the plate at 50 μL/well, and the plate was left to stand for 1 hour at room temperature.

The plate was washed 3 times with PBST, Goat anti Human IgG (H&L)-HRP or Rabbit anti-Mouse IgG-HRP diluted with 1% BSA-PBS was dispensed into the plate at 50 μL/well, and the plate was left to stand for 1 hour at room temperature. The color development and the absorbance measurement to be carried out thereafter were performed based on the method described in Example 5. By regarding the absorbance of the well, in which each of the anti-Hp antibodies was reacted with the recombinant human Hp, as 1, a ratio of the absorbance, in which the sample was reacted, to the aforementioned absorbance was calculated.

The results are shown in Table 6. In Table 6, + shows that the absorbance ratio is equal to or higher than 0.5, and - shows that the absorbance ratio is less than 0.5. It was confirmed in advance that the anti-DNP antibody and the mouse IgG1-type isotype control antibody do not bind to any of the immobilized antigens. Furthermore, the anti-Hp polyclonal antibody exhibited binding properties to all the immobilized antigens, and from this result, it was confirmed that the antigens are immobilized.

[Table 6]

| | #27 | #105 | Commercial 2F4 antibody |
|---|---|---|---|
| Recombinant human Hp | + | + | + |
| Chimera A | + | - | + |
| Chimera B | + | + | + |
| Chimera C | + | + | + |
| Chimera D | + | + | + |
| Chimera E | + | + | + |
| Chimera F | - | + | + |

As shown in Table 6, the binding properties of #27 to the chimera F was specifically reduced, and the binding properties of #105 to the chimera A was also specifically reduced. From these results, it was revealed that #27 contains at least F44 and E49 in the epitope thereof, and #105 contains at least S155, T156, and V157 in the epitope thereof. In contrast, the commercial 2F4 antibody exhibited high binding properties to all the chimeras A to F. From this result, it was revealed that the amino acid residues as the epitopes of #27 and #105 are not contained in the epitope of the commercial 2F4 antibody.

(3) Preparation of human Hp/mouse Hp chimeric protein

For the purpose of clarifying the epitope of the prepared commercial 2F4 antibody and comparing this epitope with the epitope of #27 and #105, the epitope of the commercial 2F4 antibody was analyzed. As described in (ii) of (3) of Example 19, the commercial 2F4 antibody did not bind to the bovine Hp and the mouse Hp.

Based on this result, a plurality of chimeric Hpβ chains were designed by substituting some of the amino acid residues in the human Hpβ chain (SEQ ID NO: 70) with amino acid residues of a mouse. Specifically, the amino acid sequence (SEQ ID NO: 65) of the human Hp, the amino acid sequence (SEQ ID NO: 73) of the bovine Hp, and the amino acid sequence (SEQ ID NO: 74) of the mouse Hp were compared with each other, and from the human Hpβ chain (SEQ ID NO: 70), amino acid residues that do not exhibit homology between a human being and a bovine but exhibit homology between a human being and a mouse were extracted. Furthermore, the extracted residues were substituted with the corresponding amino acid residues of the mouse Hp.

The chimeric Hpβ chain was expressed in a manner in which histidine tag is linked to the C-terminal side and the human Hpα chain (SEQ ID NO: 71) is linked by a disulfide bond as in the method described in (1) of Example 20 (hereinafter, described as chimeras G to K).

Table 7 shows the positions and types of the substituted amino acid residues in the prepared chimeras G to K. The amino acid sequences of the prepared chimeras G to K are represented by SEQ ID NOs: 89 to 93, and the base sequences of the synthetic DNAs are represented by SEQ ID NOs: 94 to 98.

[Table 7]

| Name of chimeric protein | Substitution |
|---|---|
| Chimera G | L2I/H5S/M182L |
| Chimera H | H21R/T52S/Y81H/Q83V |
| Chimera I | K67N/K130R |
| Chimera J | E113A/Q147K/M182L |
| Chimera K | Q147K/I149V/R150V/P163L |

(4) Binding properties to human Hp/mouse Hp chimeric protein

Based on the method described in (2) of Example 20, the binding properties of the commercial 2F4 antibody to the chimeras G to K prepared in (3) of Example 20 were evaluated. The results are shown in Table 8. By regarding the absorbance of the well, in which each of the anti-Hp antibodies was reacted with the recombinant human Hp, as 1, a ratio of the absorbance, in which the sample was reacted, to the aforementioned absorbance was calculated.

In Table 8, + shows that the absorbance ratio is equal to or higher than 0.5, and - shows that the absorbance ratio is less than 0.5. It was confirmed in advance that the anti-DNP antibody and the mouse IgG1-type isotype control antibody did not bind to any of the immobilized antigens. Furthermore, the anti-Hp polyclonal antibody exhibited binding properties to all the immobilized antigens, and from this result, it was confirmed that the antigens are immobilized.

[Table 8]

| | #27 | #105 | Commercial 2F4 antibody |
|---|---|---|---|
| Recombinant human Hp | + | + | + |
| Chimera G | + | + | + |
| Chimera H | + | + | + |
| Chimera I | + | + | + |
| Chimera J | + | + | + |
| Chimera K | + | + | - |

As shown in Table 8, the binding properties of the commercial 2F4 antibody to the chimera K were specifically reduced. From this result, it was revealed that the commercial 2F4 antibody contain Q147, 1149, R150, and P163 in the epitope thereof. In contrast, #27 and #105 antibodies exhibited high binding properties to all the chimeras G to K. From this result, it was revealed that the amino acid residues as the epitope of the commercial 2F4 antibody are not contained in the epitopes of #27 and #105.

The epitopes [identified in (2) of Example 20] of #27 and #105 and the epitope of the commercial 2F4 antibody are shown in Table 9.

[Table 9]

| | Amino acid residues comprised in epitope |
|---|---|
| #27 | F44/E49 |

(continued)

| | Amino acid residues comprised in epitope |
|---|---|
| #105 | S155/T156/V157 |
| Commercial 2F4 antibody | Q147/I149/R150/P163 |

From the above results, it was revealed that the epitope of the commercial 2F4 antibody is different from the epitopes of #27 and #105.

[Example 21] Preparation of defucosylated Fc amino acid-modified anti-Hp antibody having high affinity with CD16a/CD32a

Synthetic DNA (GENEWIZ) of the gene sequence of the Fc domain of human IgG1 in which amino acids were modified (G236A, S239D, and I332E) (SEQ ID NO: 72, hereinafter, abbreviated to hCg1 Xen 236 239 332 in some cases) was inserted into a portion between the sites of restriction enzymes ApaI and BamHI in the recombinant vector prepared in (1) of Example 11, thereby preparing a recombinant vector. The operation to be carried out thereafter was performed based on the method described in (3) of Example 11, thereby preparing defucosylated Fc amino acid-modified anti-Hp antibodies having high affinity with CD16a/CD32a.

[Example 22] Effect on mouse immune thrombocytopenia (ITP) model

(1) Preparation of anti-mouse platelet antibody

As a recombinant vector, a pCI vector into which the gene of the mouse IgG2bK constant region was inserted was used. The genes of the heavy chain variable region and the light chain variable region of an anti-mouse platelet antibody clone 6A6 [Mizutani H et al., Blood. 82(3), 837-844, 1993] cloned from a NZW × BXSB F1 mouse showing the symptoms of systemic autoimmune disease such as thrombocytopenia were inserted into the vector by using an In-Fusion HD cloning Kit.

The vector was transformed into the E. coli DH5α competent cells and cultured, the plasmid was extracted, and the sequence thereof was checked, thereby preparing a recombinant vector for expressing mouse IgG2b-type anti-mouse platelet 6A6 antibody. By using an ExpiFectamin 293 Transfection Kit (GIBCO), antibodies were prepared by transient expression according to the instruction. The collection of the culture supernatant and the antibody purification were performed based on the methods described in Example 8 and Example 9 by using Ab-Capcher (Protenova K. K.) as a resin for antibody purification.

(2) Effect on mouse immune thrombocytopenia (ITP) model

The inhibitory effect of the defucosylated Fc amino acid-modified #105 (G236A, S239D, and I332E) exerted on the thrombocytopenia caused by the anti-platelet antibody in the mouse ITP model was evaluated. The pathological conditions of the mouse ITP model were induced based on a known method [Mizutani H et al., Blood. 82(3), 837-844, 1993].

Male Crl:CDl (Icr) mice (Charles River Laboratories Japan, Inc.) were purchased and used when they were 6 weeks old (body weight of about 30 g). The mice were divided into 8 groups (n = 5 for each group), the defucosylated Fc amino acid-modified #105 (G236A, S239D, and I332E) prepared in Example 21 or IVIG were dissolved in PBS, and the obtained solutions were given to the mice by intraperitoneal administration at doses of 0.08, 0.4, 2, and 10 mg/head (the defucosylated Fc amino acid-modified #105) and 10 and 40 mg/head (IVIG).

PBS was administered to a control group. Thirty minutes after the administration of the test subject, the anti-mouse platelet 6A6 antibody prepared in (1) of Example 22 were given to the mice by intravenous administration at a dose of 10 μg/head so as to induce thrombocytopenia. To the control group used for ascertaining the number of platelets in a case where the disease is not induced, PBS was administered instead of the anti-mouse platelet 6A6 antibody. Twenty four hours after the administration of the anti-mouse platelet 6A6 antibody, blood was collected from the inferior vena cava under isoflurane anesthesia and put into a blood collection tube containing EDTA, and the number of platelets was measured using an ADVIA 120 Hematology System (Siemens Healthineers).

As a result, by the administration of the defucosylated Fc amino acid-modified #105 (G236A, S239D, and I332E), the thrombocytopenia caused by the anti-mouse platelet 6A6 antibody was dose-dependently inhibited. Furthermore, the effect of defucosylatetd Fc amino acid-modified #105 (G236A, S239D, and I332E) was equivalent to or stronger than that of IVIG at a dose lower than that of IVIG (Fig. 7).

Although the present invention has been specifically described with reference to certain aspects, those skilled in the related art know that the present invention can be modified and revised in various ways without departing from the gist and scope of the present invention. The present application is based on Japanese Patent Application No. 2016-066829 filed on March 29, 2016, the entire content of which is incorporated into the present specification by reference. Furthermore, all the references cited herein are incorporated into the present specification.

Sequence listing free text

SEQ ID NO: 1: base sequence of PCR forward primer for cloning human CD16a
SEQ ID NO: 2: base sequence of PCR reverse primer for cloning human CD16a
SEQ ID NO: 3: base sequence of human CD16a (V)
SEQ ID NO: 4: amino acid sequence of human CD16a
SEQ ID NO: 5: base sequence of primer FcgR3-1 for preparing soluble CD16a recombinant vector
SEQ ID NO: 6: amino acid sequence of histidine tag-conjugated soluble CD16a
SEQ ID NO: 7: base sequence of primer hhiu1
SEQ ID NO: 8: base sequence of primer hhiu3
SEQ ID NO: 9: base sequence of primer hk2
SEQ ID NO: 10: base sequence of primer hk5
SEQ ID NO: 11: base sequence of #4-VH
SEQ ID NO: 12: base sequence of #4-VL
SEQ ID NO: 13: base sequence of #6-VH
SEQ ID NO: 14: base sequence of #6-VL
SEQ ID NO: 15: base sequence of #27-VH
SEQ ID NO: 16: base sequence of #27-VL
SEQ ID NO: 17: base sequence of #105-VH
SEQ ID NO: 18: base sequence of #105-VL
SEQ ID NO: 19: base sequence of #96-6-VH
SEQ ID NO: 20: base sequence of #96-6-VL
SEQ ID NO: 21: amino acid sequence of #4-VH
SEQ ID NO: 22: amino acid sequence of #4-VL
SEQ ID NO: 23: amino acid sequence of #6-VH
SEQ ID NO: 24: amino acid sequence of #6-VL
SEQ ID NO: 25: amino acid sequence of #27-VH
SEQ ID NO: 26: amino acid sequence of #27-VL
SEQ ID NO: 27: amino acid sequence of #105-VH
SEQ ID NO: 28: amino acid sequence of #105-VL
SEQ ID NO: 29: amino acid sequence of #96-6-VH
SEQ ID NO: 30: amino acid sequence of #96-6-VL
SEQ ID NO: 31: amino acid sequence of #27-VH CDR1
SEQ ID NO: 32: amino acid sequence of #27-VH CDR2
SEQ ID NO: 33: amino acid sequence of #27-VH CDR3
SEQ ID NO: 34: amino acid sequence of #27-VL CDR1
SEQ ID NO: 35: amino acid sequence of #27-VL CDR2
SEQ ID NO: 36: amino acid sequence of #27-VL CDR3
SEQ ID NO: 37: amino acid sequence of #105-VH CDR1
SEQ ID NO: 38: amino acid sequence of #105-VH CDR2
SEQ ID NO: 39: amino acid sequence of #105-VH CDR3
SEQ ID NO: 40: amino acid sequence of #105-VL CDR1
SEQ ID NO: 41: amino acid sequence of #105-VL CDR2
SEQ ID NO: 42: amino acid sequence of #105-VL CDR3
SEQ ID NO: 43: base sequence of PCR forward primer for amplifying #4-VH gene
SEQ ID NO: 44: base sequence of PCR reverse primer for amplifying #4-VH gene
SEQ ID NO: 45: base sequence of PCR forward primer for amplifying #4-VL gene
SEQ ID NO: 46: base sequence of PCR reverse primer for amplifying #4-VL gene
SEQ ID NO: 47: base sequence of PCR forward primer for amplifying #6-VH gene

SEQ ID NO: 48: base sequence of PCR reverse primer for amplifying #6-VH gene
SEQ ID NO: 49: base sequence of PCR forward primer for amplifying #6-VL gene
SEQ ID NO: 50: base sequence of PCR reverse primer for amplifying #6-VL gene
SEQ ID NO: 51: base sequence of PCR forward primer for amplifying #27-VH gene
SEQ ID NO: 52: base sequence of PCR reverse primer for amplifying #27-VH gene
SEQ ID NO: 53: base sequence of PCR forward primer for amplifying #27-VL gene
SEQ ID NO: 54: base sequence of PCR reverse primer for amplifying #27-VL gene
SEQ ID NO: 55: base sequence of PCR forward primer for amplifying #105-VH gene
SEQ ID NO: 56: base sequence of PCR reverse primer for amplifying #105-VH gene
SEQ ID NO: 57: base sequence of PCR forward primer for amplifying #105-VL gene
SEQ ID NO: 58: base sequence of PCR reverse primer for amplifying #105-VL gene
SEQ ID NO: 59: base sequence of PCR forward primer for amplifying #96-6-VH gene
SEQ ID NO: 60: base sequence of PCR reverse primer for amplifying #96-6-VH gene
SEQ ID NO: 61: base sequence of PCR forward primer for amplifying #96-6-VL gene
SEQ ID NO: 62: base sequence of PCR reverse primer for amplifying #96-6-VL gene
SEQ ID NO: 63: base sequence of synthetic DNA (hCg1 Xen 239 332)
SEQ ID NO: 64: amino acid sequence of soluble CD32a
SEQ ID NO: 65: amino acid sequence of human haptoglobin, polymorphism 1 (NP 005134. 1)
SEQ ID NO: 66: amino acid sequence of human haptoglobin, polymorphism 2 (NP_001119574. 1)
SEQ ID NO: 67: gene sequence of human haptoglobin, polymorphism 1 (NM_005143. 3)
SEQ ID NO: 68: gene sequence of human haptoglobin, polymorphism 2 (NM_001126102. 1)
SEQ ID NO: 69: amino acid sequence of α chain of polymorphism 1 (NP_005134. 1) of signal sequence-free human haptoglobin
SEQ ID NO: 70: amino acid sequence of β chain of human haptoglobin
SEQ ID NO: 71: amino acid sequence of α chain of polymorphism 2 (NP_001119574. 1) of signal sequence-free human haptoglobin
SEQ ID NO: 72: base sequence of synthetic DNA (hCg1 Xen 236 239 332)
SEQ ID NO: 73: amino acid sequence of bovine haptoglobin
SEQ ID NO: 74: amino acid sequence of mouse haptoglobin
SEQ ID NO: 75: amino acid sequence of chimera A
SEQ ID NO: 76: amino acid sequence of chimera B
SEQ ID NO: 77: amino acid sequence of chimera C
SEQ ID NO: 78: amino acid sequence of chimera D
SEQ ID NO: 79: amino acid sequence of chimera E
SEQ ID NO: 80: amino acid sequence of chimera F
SEQ ID NO: 81: base sequence of chimera A
SEQ ID NO: 82: base sequence of chimera B
SEQ ID NO: 83: base sequence of chimera C
SEQ ID NO: 84: base sequence of chimera D
SEQ ID NO: 85: base sequence of chimera E
SEQ ID NO: 86: base sequence of chimera F
SEQ ID NO: 87: amino acid sequence of recombinant human Hp
SEQ ID NO: 88: base sequence of recombinant human Hp
SEQ ID NO: 89: amino acid sequence of chimera G
SEQ ID NO: 90: amino acid sequence of chimera H
SEQ ID NO: 91: amino acid sequence of chimera I
SEQ ID NO: 92: amino acid sequence of chimera J
SEQ ID NO: 93: amino acid sequence of chimera K
SEQ ID NO: 94: base sequence of chimera G
SEQ ID NO: 95: base sequence of chimera H
SEQ ID NO: 96: base sequence of chimera I
SEQ ID NO: 97: base sequence of chimera J
SEQ ID NO: 98: base sequence of chimera K

## Claims

1. A monoclonal antibody which specifically recognizes a β chain of human haptoglobin consisting of an amino acid sequence represented by SEQ ID NO: 70 and forms a polyvalent immune complex by binding to the human haptoglobin.

2. A monoclonal antibody which specifically recognizes a β chain of the human haptoglobin represented by SEQ ID NO: 70 in competition with one antibody selected from the following (a) to (d) and binds to the same epitope as an epitope included in the human haptoglobin to which the selected antibody binds:

(a) antibody comprising a heavy chain (hereinafter, described as H chain) of an antibody in which complementary determining regions (hereinafter, described as CDRs) 1 to 3 comprise amino acid sequences represented by SEQ ID NOs: 31, 32, and 33, respectively, and a light chain (hereinafter, described as L chain) of an antibody in which CDRs 1 to 3 comprise amino acid sequences represented by SEQ ID NOs: 34, 35, and 36, respectively;
(b) antibody comprising an H chain of an antibody in which CDRs 1 to 3 comprise amino acid sequences represented by SEQ ID NOs: 37, 38, and 39, respectively, and an L chain of an antibody in which CDRs 1 to 3 comprise amino acid sequences represented by SEQ ID NOs: 40, 41, and 42, respectively;
(c) antibody comprising an H chain of an antibody comprising an amino acid sequence represented by SEQ ID NO: 25 and an L chain of an antibody comprising an amino acid sequence represented by SEQ ID NO: 26; and
(d) antibody comprising an H chain of an antibody comprising an amino acid sequence represented by SEQ ID NO: 27 and an L chain of an antibody comprising an amino acid sequence represented by SEQ ID NO: 28.

3. A monoclonal antibody selected from the following (a) to (d),

(a) antibody comprising an H chain of an antibody in which CDRs 1 to 3 comprise amino acid sequences represented by SEQ ID NOs: 31, 32, and 33, respectively, and an L chain of an antibody in which CDRs 1 to 3 comprise amino acid sequences represented by SEQ ID NOs: 34, 35, and 36, respectively;
(b) antibody comprising an H chain of an antibody in which CDRs 1 to 3 comprise amino acid sequences represented by SEQ ID NOs: 37, 38, and 39, respectively, and an L chain of an antibody in which CDRs 1 to 3 comprise amino acid sequences represented by SEQ ID NOs: 40, 41, and 42, respectively;
(c) antibody comprising an H chain of an antibody comprising an amino acid sequence represented by SEQ ID NO: 25 and an L chain of an antibody comprising an amino acid sequence represented by SEQ ID NO: 26; and
(d) antibody comprising an H chain of an antibody comprising an amino acid sequence represented by SEQ ID NO: 27 and an L chain of an antibody comprising an amino acid sequence represented by SEQ ID NO: 28.

**4.** The monoclonal antibody according to any one of claims 1 to 3 which binds to at least Phe at position 44 and Glu at position 49 in the amino acid sequence of the β chain of the human haptoglobin represented by SEQ ID NO: 70.

**5.** The monoclonal antibody according to any one of claims 1 to 3 which binds to at least Ser at position 155, Thr at position 156, and Val at position 157 in the amino acid sequence of the β chain of the human haptoglobin represented by SEQ ID NO: 70.

**6.** The monoclonal antibody according to any one of claims 1 to 5 which is a recombinant antibody.

**7.** The monoclonal antibody according to claim 6 that is a recombinant antibody selected from a human chimeric antibody, a humanized antibody, and a human antibody.

**8.** The monoclonal antibody according to any one of claims 1 to 7,
wherein the polyvalent immune complex formed of the monoclonal antibody binds to at least one human Fcγ receptor selected from the group consisting of a human Fcγ receptor III, a human Fcγ receptor II, and a human Fcγ receptor I.

**9.** The monoclonal antibody according to any one of claims 1 to 8,
wherein the polyvalent immune complex formed of the monoclonal antibody comprises at least two antibody molecules and one human haptoglobin molecule.

**10.** The monoclonal antibody according to any one of claims 1 to 9,
wherein fucose is not bound to N-acetylglucosamine at a reducing terminal of an N-glycoside-linked sugar chain linked to a Fc domain of the antibody.

**11.** DNA encoding the monoclonal antibody according to any one of claims 1 to 10.

**12.** A recombinant vector comprising the DNA according to claim 11.

**13.** A transformant obtained by introducing the recombinant vector according to claim 12 into a host cell.

**14.** A method for producing the monoclonal antibody according to any one of claims 1 to 10, comprising:
culturing the transformant according to claim 13 in a medium such that the monoclonal antibody according to any one of claims 1 to 10 is generated and accumulated in a culture; and collecting the antibody from the culture.

**15.** A method for treating autoimmune diseases, comprising:
administering the monoclonal antibody according to any one of claims 1 to 10.

**16.** A therapeutic agent for treatment of autoimmune diseases, comprising:

the monoclonal antibody according to any one of claims 1 to 10; and
a pharmacologically accepted carrier.

**17.** A therapeutic agent for treatment of autoimmune diseases, comprising:
a monoclonal antibody binding to human haptoglobin.

**18.** The therapeutic agent for treatment of autoimmune diseases according to claim 17, wherein the monoclonal antibody is a recombinant antibody.

**19.** The therapeutic agent for treatment of autoimmune diseases according to claim 18, wherein the monoclonal antibody is a recombinant antibody selected from a human chimeric antibody, a humanized antibody, and a human antibody.

**20.** The therapeutic agent for treatment of autoimmune diseases according to any one of claims 17 to 19, wherein the monoclonal antibody is an antibody forming a polyvalent immune complex by binding to human haptoglobin.

**21.** The therapeutic agent for treatment of autoimmune diseases according to any one of claims 17 to 20, wherein the polyvalent immune complex formed of the monoclonal antibody binds to at least one human Fcγ receptor selected from the group consisting of a human Fcγ receptor III, a human Fcγ receptor II, and a human Fcγ receptor I.

**22.** The therapeutic agent for treatment of autoimmune diseases according to claim 20 or 21, wherein the polyvalent immune complex formed of the monoclonal antibody comprises at least two antibody molecules and one human haptoglobin molecule.

[Fig. 1]

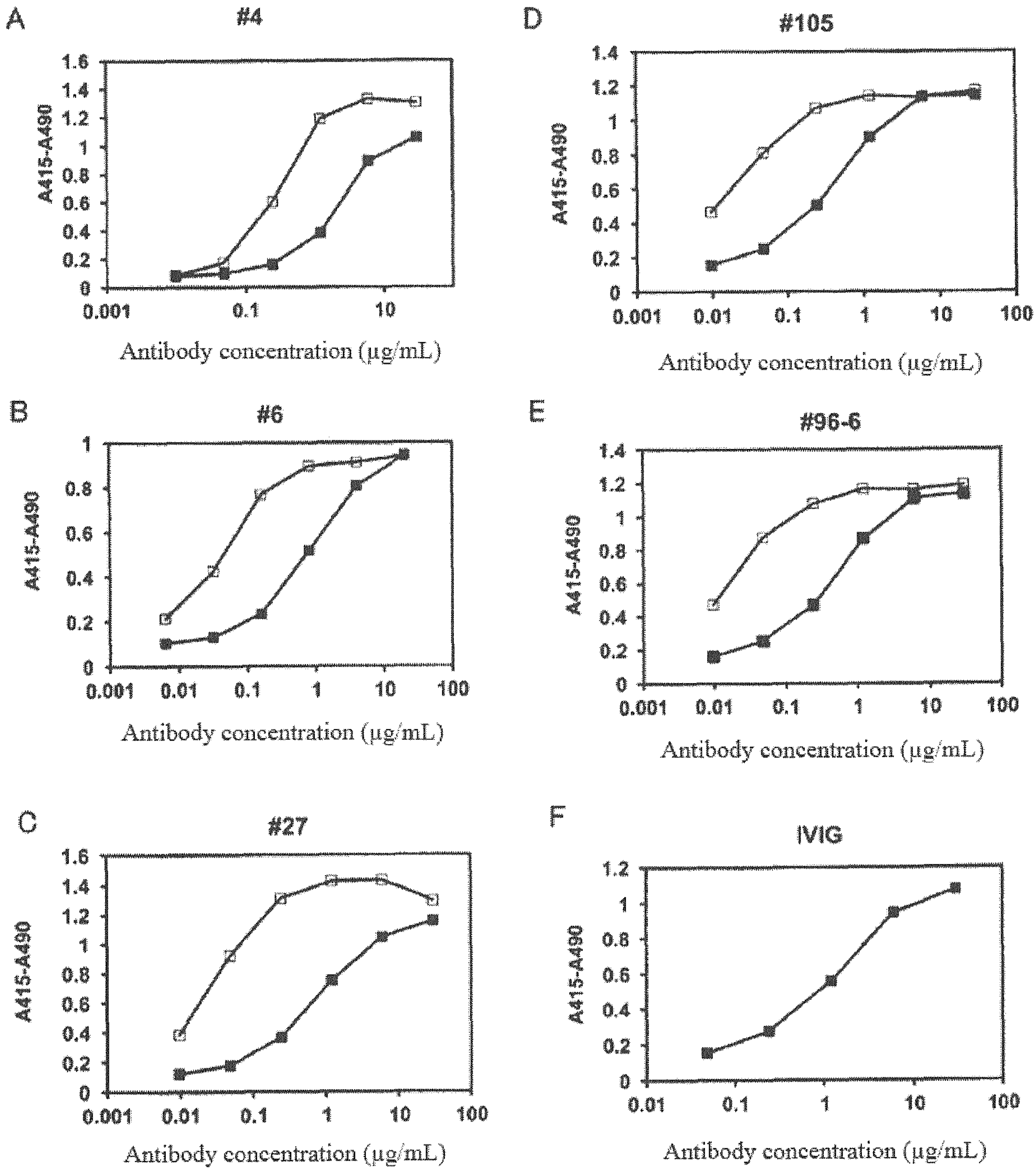
A
#4
A415-A490
Antibody concentration (µg/mL)
D
#105
A415-A490
Antibody concentration (µg/mL)
B
#6
A415-A490
Antibody concentration (µg/mL)
E
#96-6
A415-A490
Antibody concentration (µg/mL)
C
#27
A415-A490
Antibody concentration (µg/mL)
F
IVIG
A415-A490
Antibody concentration (µg/mL)

[Fig. 2]

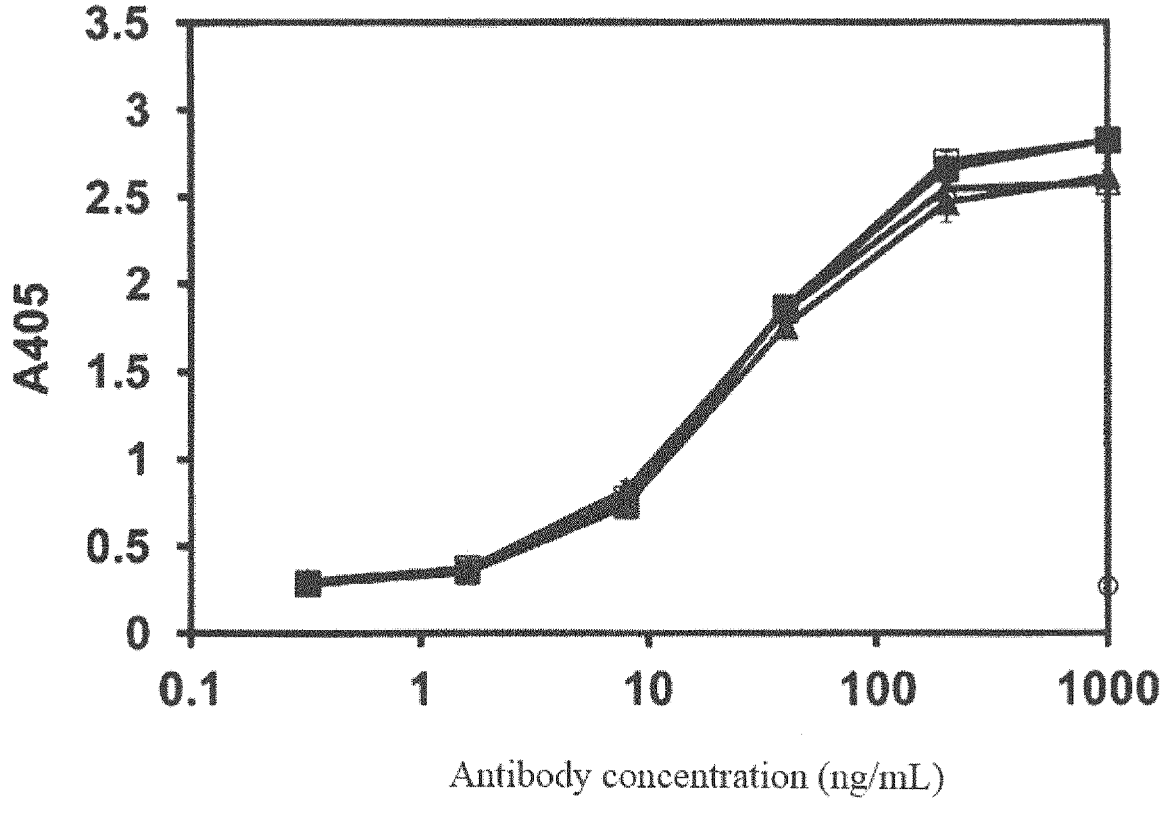
3.5
3
2.5
2
1.5
1
0.5
0
A405
0.1
1
10
100
1000
Antibody concentration (ng/mL)

[Fig. 3]

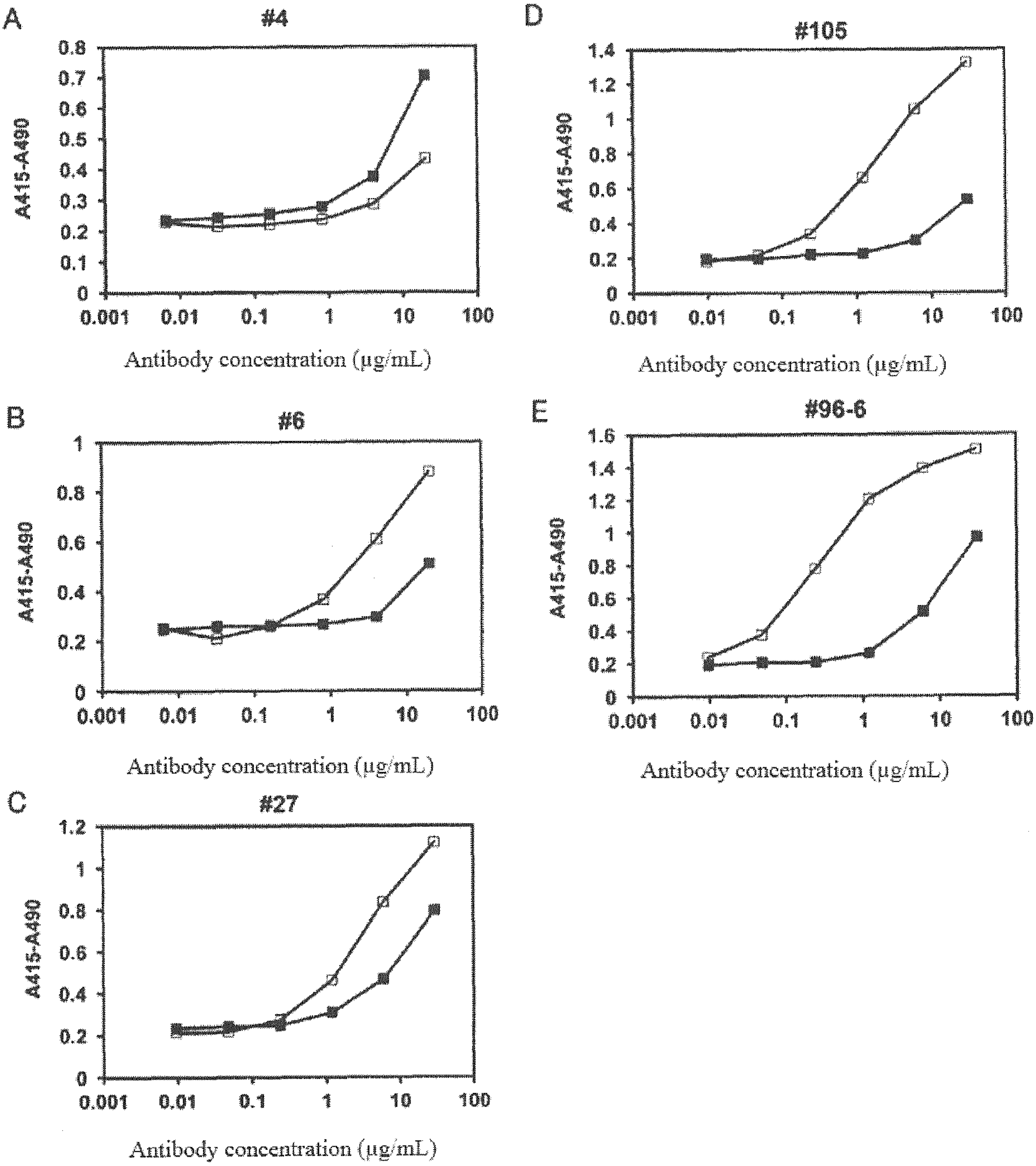
A
#4
A415-A490
0.8
0.7
0.6
0.5
0.4
0.3
0.2
0.1
0
0.001 0.01 0.1 1 10 100
Antibody concentration (μg/mL)
D
#105
A415-A490
1.4
1.2
1
0.8
0.6
0.4
0.2
0
0.001 0.01 0.1 1 10 100
Antibody concentration (μg/mL)
B
#6
A415-A490
1
0.8
0.6
0.4
0.2
0
0.001 0.01 0.1 1 10 100
Antibody concentration (μg/mL)
E
#96-6
A415-A490
1.6
1.4
1.2
1
0.8
0.6
0.4
0.2
0
0.001 0.01 0.1 1 10 100
Antibody concentration (μg/mL)
C
#27
A415-A490
1.2
1
0.8
0.6
0.4
0.2
0
0.001 0.01 0.1 1 10 100
Antibody concentration (μg/mL)

[Fig. 4]

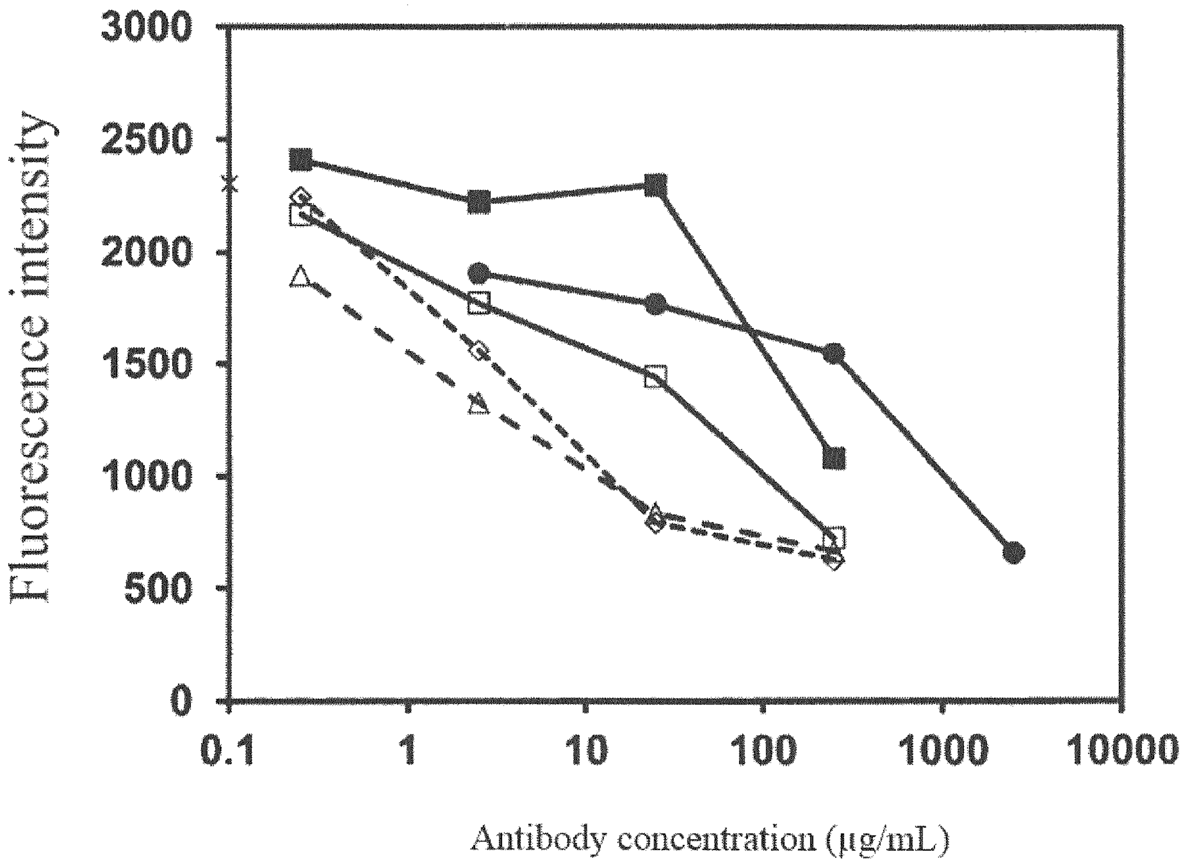
3000
2500
2000
1500
1000
500
0
Fluorescence intensity
0.1
1
10
100
1000
10000
Antibody concentration (μg/mL)

[Fig. 5]

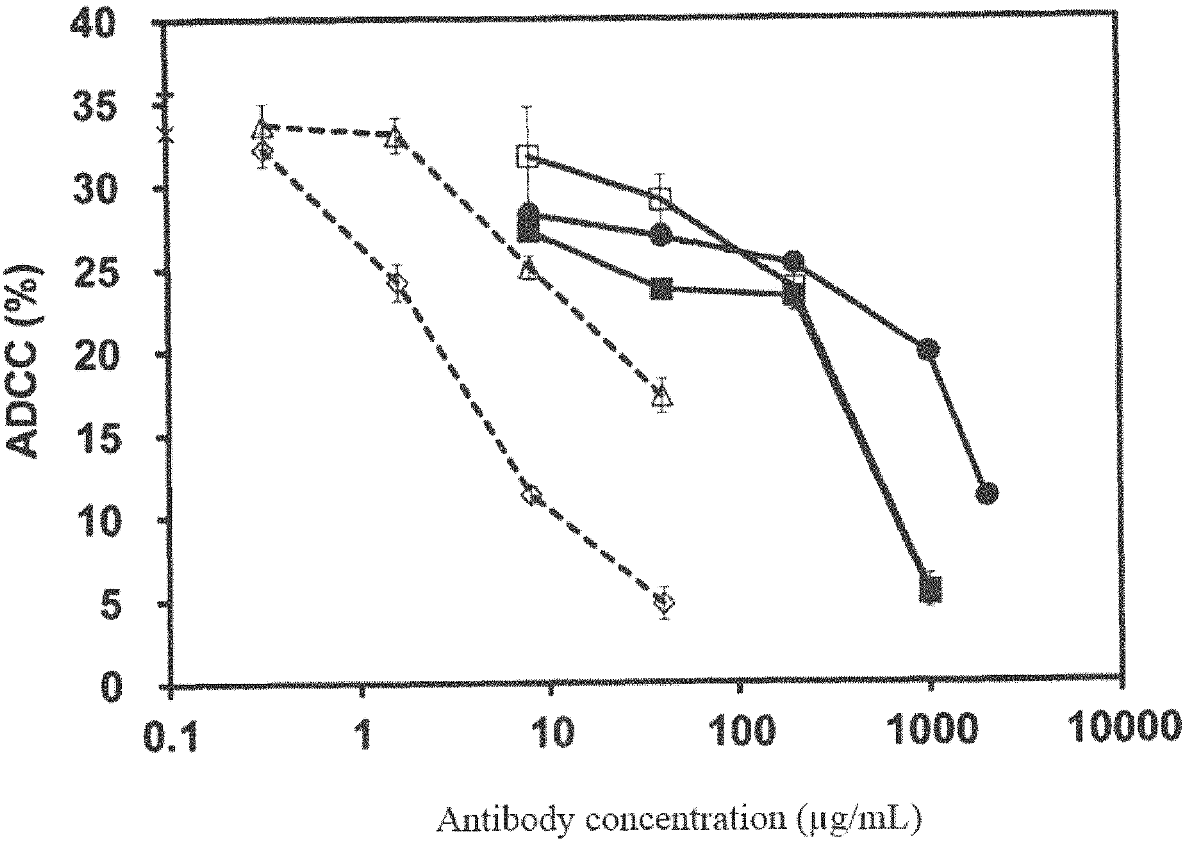
ADCC (%)
40
35
30
25
20
15
10
5
0
0.1
1
10
100
1000
10000
Antibody concentration (µg/mL)

[Fig. 6]

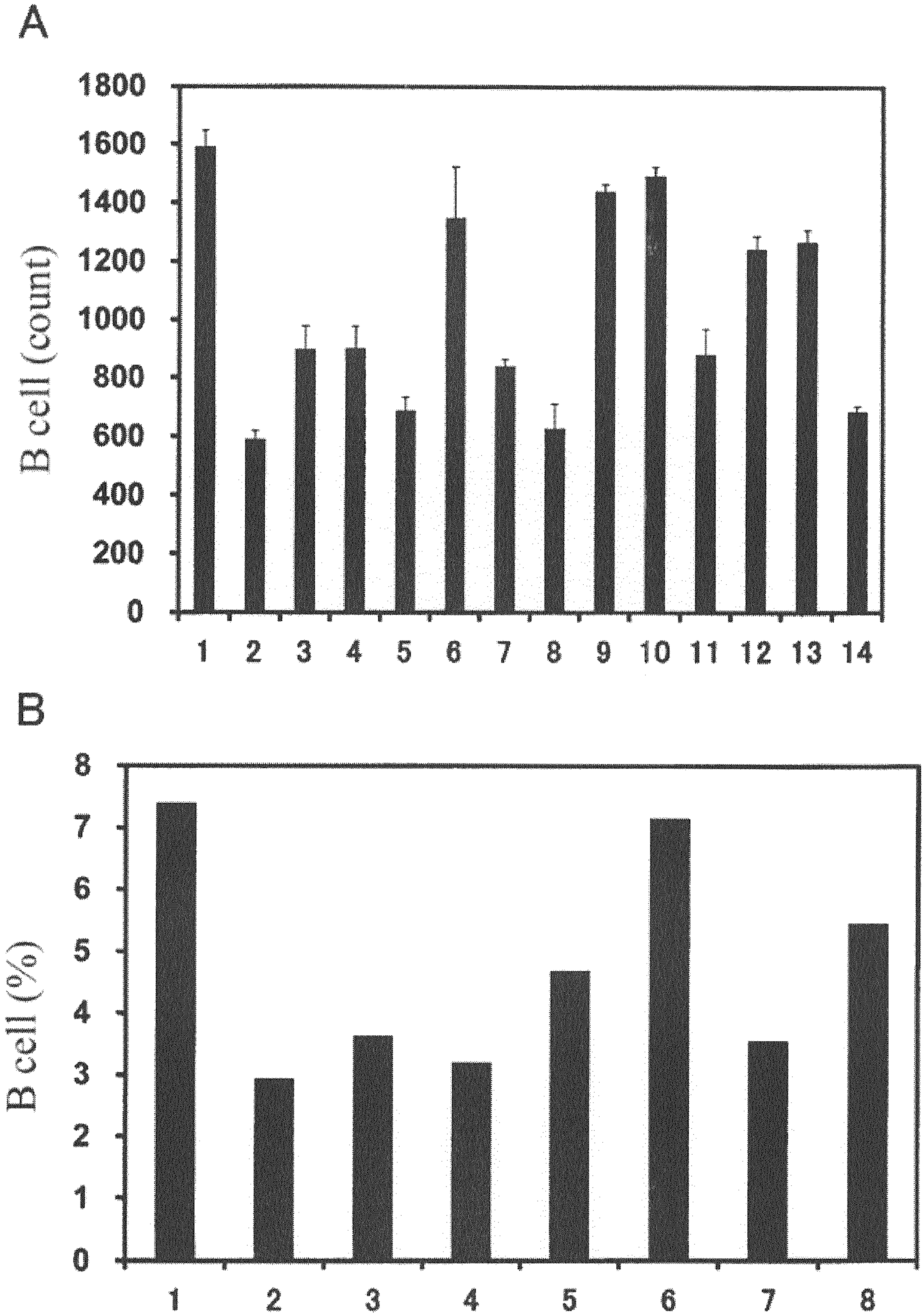
A
B cell (count)
1800
1600
1400
1200
1000
800
600
400
200
0
1 2 3 4 5 6 7 8 9 10 11 12 13 14
B
B cell (%)
8
7
6
5
4
3
2
1
0
1 2 3 4 5 6 7 8

[Fig. 7]

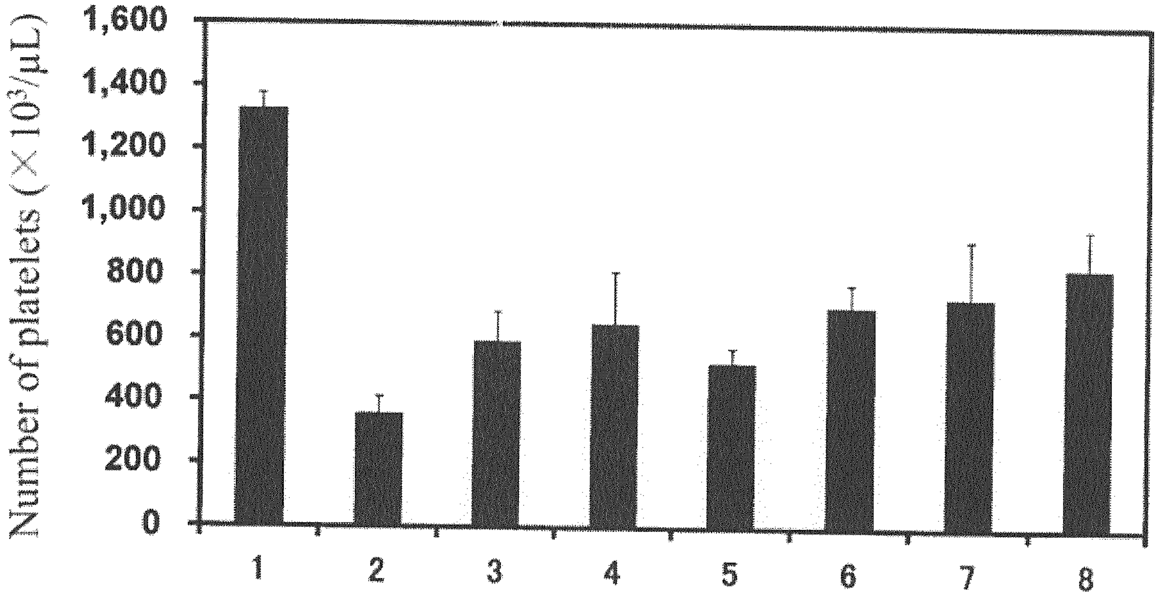
Number of platelets (×10³/μL)
1,600
1,400
1,200
1,000
800
600
400
200
0
1
2
3
4
5
6
7
8

# INTERNATIONAL SEARCH REPORT

International application No.
PCT/JP2017/012736

A. CLASSIFICATION OF SUBJECT MATTER
*C12N15/09*(2006.01)i, *A61K39/395*(2006.01)i, *A61K48/00*(2006.01)i, *A61P37/06* (2006.01)i, *C07K16/18*(2006.01)i, *C12P21/08*(2006.01)i

According to International Patent Classification (IPC) or to both national classification and IPC

B. FIELDS SEARCHED

Minimum documentation searched (classification system followed by classification symbols)
C12N15/09, A61K39/395, A61K48/00, A61P37/06, C07K16/18, C12P21/08

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/BIOSIS/WPIDS(STN), UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br>Y | KATNIK, I. et al., Monoclonal Antibodies Against Human Haptoglobin, Hybridoma, 1989, Vol.8, No.5, pp.551-560, particularly, Abstract, ISSN: 1554-0014 | 1,6-14<br>1,6-14 |
| X<br>Y | LAI, I. H. et al., Cloning and expression of human haptoglobin subunits in Escherichia coli: Delineation of a major antioxidant domain, Protein Expression & Purification, 2007, Vol.52, pp.356-362, particularly, p.358, left column, l.29-35, ISSN: 1046-5928 | 1,6-14<br>1,6-14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 June 2017 (16.06.17) | 27 June 2017 (27.06.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

INTERNATIONAL SEARCH REPORT

International application No. PCT/JP2017/012736

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | TSENG, C. F. et al., Purification of human haptoglobin 1-1, 2-1, and 2-2 using monoclonal antibody affinity chromatography, Protein Expression & Purification, 2004, Vol.33, pp.265-273, particularly, p.226, right column, l.14-30, ISSN: 1046-5928 | 1,6-14<br>1,6-14 |
| X<br>Y | MAO, S. J. T. et al., Molecular properties of human Haptoglobin as probed by monoclonal antibodies, FASEB Journal, 2004, Vol.18, No.4-5, Abst.774.30, Whole document, ISSN: 0892-6638 | 1,6-14<br>1,6-14 |
| X<br>Y | JP 2007-504463 A (Royal Women's Hospital), 01 March 2007 (01.03.2007), claims 1, 10; paragraph [0075] & WO 2005/024054 A1 claims 1, 10; page 25, lines 10 to 24 & US 2007/0053896 A1 & EP 1668149 A1 & CN 1871362 A | 1,6-14<br>1,6-14 |
| X<br>Y | Masaru RYOJI, "Proteomic search for biochemical markers of the mental stress in mice, and its use for testing efficacies of foodstuffs to alleviate the stress buildup", Shokuseikatsu Kagaku Bunka Oyobi Kankyo ni Kansuru Kenkyu Josei Kenkyu Kiyo, 2007, vol.2007th, pages 49 to 54, particularly, abstract, page 51, lines 4 to 16 | 1,6-14<br>1,6-14 |
| A | SAITO, S. et al., Haptoglobin-β chain defined by monoclonal antibody RM2 as a novel serum marker for prostate cancer, Int. J. Cancer, 2008, Vol.123, pp.633-640, particularly, Abstract, ISSN: 1097-0215 | 1-22 |
| A | JP 2010-96663 A (Japan Health Sciences Foundation), 30 April 2010 (30.04.2010), claims 1 to 2; example 1; table 7 (Family: none) | 1-22 |
| A | JP 2015-532301 A (CSL Behring LLC), 09 November 2015 (09.11.2015), abstract; claims 1, 16, 20 to 21 & WO 2014/055552 A1 abstract; claims 1, 16, 20 to 21 & US 2014/0094411 A1 & EP 2904005 A1 & CN 104968676 A & KR 10-2015-0063547 A | 1-22 |

Form PCT/ISA/210 (continuation of second sheet) (January 2015)

**INTERNATIONAL SEARCH REPORT**

International application No.
PCT/JP2017/012736

C (Continuation). DOCUMENTS CONSIDERED TO BE RELEVANT

| Category* | Citation of document, with indication, where appropriate, of the relevant passages | Relevant to claim No. |
|---|---|---|
| A | JP 2009-500458 A (Macrogenics, Inc.), 08 January 2009 (08.01.2009), abstract & WO 2007/009065 A2 abstract & US 2007/0036786 A1 & EP 1907002 A2 & CN 101627054 A | 1-22 |
| A | NAKAMURA, S. et al., Hemolysis due to High-Dose Intravenous Gammaglobulin Treatment for Patients with Idiopathic Thrombocytopenic Purpura, Acta haemat., 1986, Vol.76, pp.115-118, particularly, Abstract | 1-22 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040101909 A
- WO 2005035586 A
- WO 0231140 A
- US 7317091 B
- JP 2013165716 A
- JP 4961501 B
- JP S58110600 A
- JP S60221091 A
- US 4686191 A
- US 4939094 A
- US 5160735 A
- JP H0322979 A
- JP H02227075 A
- WO 9710354 A
- US 6001358 A
- WO 2010143698 A
- JP S63000299 A
- JP H05336963 A
- WO 9423021 A
- JP H2227075 A
- JP H02257891 A
- WO 200231140 A
- WO 0061739 A
- US 20070148165 A
- US 6737056 B
- US 7297775 B
- WO 2007011041 A
- US 5736137 A
- JP 2016066829 A

### Non-patent literature cited in the description

- *Trends in Immunology,* 2008, vol. 29 (12), 608-615
- *Science,* 2006, vol. 313 (5787), 670-673
- The Latest Separate Volume for Medicine. **YASUO IKEDA.** Thrombocytopenia·Thrombocytosis. Saishin Igaku
- *ASH Annual Meeting,* 2006
- *J Clin Invest.,* 2005, vol. 115 (1), 25-27
- *Int Immunol.,* 2004, vol. 16 (7), 929-936
- *Curr Pharm Des.,* 2006, vol. 12 (2), 173-179
- *Br J Haematol.,* 2004, vol. 127 (1), 90-96
- *Blood Frontier,* 2007, vol. 17 (1), 17-19
- *Redox Rep.,* 2001, vol. 6, 379
- *J. Biochem. Mol. Biol.,* 2007, vol. 40, 1028
- Molecular Cloning. Laboratory Manual. Cold Spring Harbor Laboratory Press, 1989
- Current Protocols in Molecular Biology. John Wiley & Sons, 1987
- *Nucleic Acids Research,* 1982, vol. 10, 6487
- *Proc. Natl. Acad. Sci. USA,* 1982, vol. 79, 6409
- *Gene,* 1985, vol. 34, 315
- *Nucleic Acids Research,* 1985, vol. 13, 4431
- *Proc. Natl. Acad. Sci. USA,* 1985, vol. 82, 488
- Molecular Cloning. A Laboratory Manual. Cold Spring Harbor Laboratory Press, 1989
- DNA Cloning 1: Core Techniques, A Practical Approach. Oxford University, 1995
- *J. Mol. Biol.,* 1990, vol. 215, 403
- *Nucleic Acids Res.,* 1997, vol. 25, 3389
- *Genome Res.,* 1997, vol. 7, 649, http://www.ncbi.nlm.nih.gov/Education/BLASTinfo/information3.html
- *Cancer Immunol. Immunother.,* 1993, vol. 36, 373
- Monoclonal Antibodies-Principles and Practice. Academic Press, 1996
- Antibodies-A Laboratory Manual. Cold Spring Harbor Laboratory, 1988
- Monoclonal Antibody Experiment Manual. Kodansha Scientific Ltd, 1987
- *J. Exp. Med.,* 1989, vol. 170, 481
- *J. Exp. Med.,* 1989, vol. 170, 1369
- *J. Biol Chem.,* 1991, vol. 266, 13449
- *Trends in Immunology,* 2008, vol. 29, 608
- **CLARK M.** *Chemical Immunology,* 1997, vol. 65, 88
- **GORTER et al.** *Immunol Today,* 1999, vol. 20, 576
- Molecular Cloning. Current Protocols in Molecular Biology
- *Proc. Natl. Acad. Sci., USA,* 1982, vol. 79, 6409
- *Proc. Natl. Acad Sci USA,* 1985, vol. 82, 488
- *Agricultural Biological Chemistry,* 1984, vol. 48, 669
- *Agric Biol. Chem.,* 1989, vol. 53, 277
- *Proc. Natl. Acad. Sci. USA,* 1985, vol. 82, 4306
- *J. Bacteriol.,* 1990, vol. 172, 2392

- *Proc. Natl. Acad. Sci. USA,* 1972, vol. 69, 2110
- *Gene,* 1982, vol. 17, 107
- *Molecular & General Genetics,* 1979, vol. 168, 111
- *Cytotechnology,* 1990, vol. 3, 133
- *Nature,* 1987, vol. 329, 840
- *J. Biochemistry,* 1987, vol. 101, 1307
- *Journal of Experimental Medicine,* 1958, vol. 108, 945
- *Proc. Natl. Acad. Sci. USA,* 1968, vol. 60, 1275
- *Genetics,* 1968, vol. 55, 513
- *Chromosoma,* 1973, vol. 41, 129
- *Methods in Cell Science,* 1996, vol. 18, 115
- *Radiation Research,* 1997, vol. 148, 260
- *Proc. Natl. Acad. Sci. USA,* 1980, vol. 77, 4216
- *Cell,* 1975, vol. 6, 121
- *Molecular Cellgenetics,* 883-900
- *Proc. Natl. Acad. Sci. USA,* 1987, vol. 84, 7413
- *The Journal of the American Medical Association,* 1967, vol. 199, 519
- *Science,* 1952, vol. 122, 501
- *Virology,* 1959, vol. 8, 396
- *Proc. Soc. Exp. Biol. Med.,* 1950, vol. 73, 1
- Molecular Cloning, A Laboratory Manual. Cold Spring Harbor Laboratory Press, 1989
- **PAULSON et al.** *J. Biol. Chem.,* 1989, vol. 264, 17619
- **LOWE et al.** *Proc. Natl. Acad. Sci. USA,* 1989, vol. 86, 8227
- *Genes Develop.,* 1990, vol. 4, 1288
- *Current Topics in Microbiology and Immunology,* 1978, vol. 18, 1
- *European J. Immunology,* 1976, vol. 6, 511
- *Nature,* 1978, vol. 276, 269
- *J. Immunology,* 1979, vol. 123, 1548
- *Nature,* 1975, vol. 256, 495
- *Cytotechnol.,* 1990, vol. 3, 133
- *J. Biochem.,* 1987, vol. 101, 1307
- *Gene,* 1984, vol. 27, 223
- *Proc. Natl. Acad. Sci. USA,* 1981, vol. 78, 1527
- *Cytotechnol,* 1990, vol. 4, 173
- *Cytotechnol.,* 1993, vol. 13, 79
- *Biochem. Biophys. Res. Commun.,* 1987, vol. 149, 960
- *Cell,* 1985, vol. 41, 479
- *Cell,* 1983, vol. 33, 717
- *J. Immunol. Methods,* 1994, vol. 167, 271
- *Hybridoma,* 1998, vol. 17, 559
- *Methods in Enzymol.,* 1987, vol. 154, 3
- *Strategies,* 1992, vol. 5, 58
- *Nucleic Acids Research,* 1989, vol. 17, 9494
- *DNA Cloning: A Practical Approach,* 1985, vol. I, 49
- *Mol. Cell. Biol.,* 1983, vol. 3, 280
- *Gene,* 1985, vol. 33, 103
- *Strategies,* 1992, vol. 5, 81
- *Genetics,* 1954, vol. 39, 440
- *Science,* 1983, vol. 222, 778
- *J. Mol. Biol.,* 1983, vol. 166, 1
- *J. Mol. Biol.,* 1966, vol. 16, 118
- *Gene,* 1985, vol. 38, 275
- *Proc. Natl. Acad. Sci. USA,* 1977, vol. 74, 5463
- *BIO/TECHNOLOGY,* 1991, vol. 9, 266
- *J. Mol. Biol.,* 1977, vol. 112, 535
- *Protein Engineering,* 1994, vol. 7, 1501
- Methods in Nucleic Acids Res. CRC Press, 1991, 283
- Methods in Nucleic Acids Res. CRC Press, 1991
- Monoclonal Antibodies-Principles and practice. Thiral Antibodies-Principles and Press, 1996
- *Somatic Cell and Molecular Genetics,* 1986, vol. 12, 55
- Monoclonal Antibodies-Principles and practice. Academic Press, 1996
- *Nature,* 1970, vol. 227, 680
- *J. Cell. Biol.,* 1982, vol. 93, 576
- **MOTOKI K.** *Clin. Cancer Res.,* 2005, vol. 11, 3126-3135
- *J Sep Sci.,* 2009, vol. 32, 1224
- **MASUDA K.** *Mol. Immunol.,* 2007, vol. 44, 3122-3131
- **MIZUTANI H et al.** *Blood,* 1993, vol. 82 (3), 837-844